(19) Europäisches Patentamt
European Patent Office
Office européen des brevets

(11) Publication number: **0 216 860 B1**

(12) **EUROPEAN PATENT SPECIFICATION**

(45) Date of publication of patent specification: **28.10.92**

(51) Int. Cl.5: **C12Q 1/68**, C12P 19/34, C12N 15/00

(21) Application number: **86902189.9**

(22) Date of filing: **14.03.86**

(86) International application number: **PCT/US86/00544**

(87) International publication number: **WO 86/05518 (25.09.86 86/21)**

(54) **STEREOREGULAR POLYNUCLEOTIDE-BINDING POLYMERS.**

(30) Priority: **15.03.85 US 712396**

(43) Date of publication of application: **08.04.87 Bulletin 87/15**

(45) Publication of the grant of the patent: **28.10.92 Bulletin 92/44**

(84) Designated Contracting States: **AT BE CH DE FR GB IT LI LU NL SE**

(56) References cited:
**US-A- 4 123 610**
**US-A- 4 507 433**

**Biochemistry, vol. 18, n 23, issued November 13, 1979, P.S. Miller et al, "Nonionic Nucleic Acid Analogues. Synthesis and characterization of dideoxyribonucleoside methylphosphonates", see page 5140**

(73) Proprietor: **SUMMERTON, James**
**1935 N.W. Larch**
**Cornvallis Oregon 97330(US)**

Proprietor: **WELLER, Dwight**
**3323 N.W. Elmwood**
**Cornvallis Oregon 97330(US)**

Proprietor: **STIRCHAK, Eugene**
**3126 Kevington Road**
**Eugene Oregon 97405(US)**

(72) Inventor: **SUMMERTON, James**
**1935 N.W. Larch**
**Cornvallis Oregon 97330(US)**
Inventor: **WELLER, Dwight**
**3323 N.W. Elmwood**
**Cornvallis Oregon 97330(US)**
Inventor: **STIRCHAK, Eugene**
**3126 Kevington Road**
**Eugene Oregon 97405(US)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

Biochemistry, vol. 24, n 22, issued August 1985, A. Murakami et al "Characterization of sequence-specific oligo-deoxyribonucleoside methyl-phosphonates and their interaction with rabbit globin mRNA", see pages 4045-4046

Chemical abstracts, vol. 91, n 19, issued 1979 (Columbos, Oregon, USA), J. Summerton "Intracellular inactivation of specific nucleotide sequences : a general approach to the treatment of viral diseases and virally mediated cancers", see pages 73, column 2 and 74, column 1, the abstract n 151527g, J. Theor Biol. 1979, 78(1), 77-99 (Eng)

Nucleic acids research, vol, 12, n 8, issued April 25, 1984, M. Renz et al "A colorimetric method for DNA hybridization", see page 3435

Biochemistry, vol. 20, n 7, issued March 31, 1981, P.S. Miller et al, "Biochemical and biological effects of nonionic nucleic acid methylphosphonates", see pages 1878-1879

Journal of biological chemistry, vol. 255, n 20, issued October 25, 1980, P.S. Miller et al "Oligothymidylate analogues having stereoregular, alternating methylphosphonate/phosphodiester backbones", see page 9659

(74) Representative: **Goldin, Douglas Michael et al**
**J.A. KEMP & CO. 14, South Square Gray's Inn London WC1R 5EU(GB)**

## Description

1. Field of the Invention

The present invention relates to base-specific polynucleotide-binding polymers

2. Background of the Invention

Compounds capable of recognizing and binding specifically to a target base sequence in a polynucleotide have many potentially valuable applications. For example, in therapeutic uses, the compounds could act to bind to and inactivate viral genomes, or other single-stranded polynucleotide species characteristic of an infecting microorganism. In diagnostic applications, the compounds could be used to "hybridize" with analyte single-stranded polynucleotides, to detect the presence of viral or bacterial-specific nucleic acid species.

The earliest attempts to devise an agent which is capable of binding specifically to a base-specific target region of a polynucleotide were based on the concept of (Belikov, Zarotova, Grinev) derivatizing a target-specific polynucleotide with an alkylating agent, (Summerton and Bartlett (1978), J. Molec. Biol, 122: 145) binding the derivatixed copound to the target single-strand RNA or DNA. Alkylation of the target strand would then bind the compound irreversible to the target. There are two major difficulties with this approach. First, the negatively charged backbone of the derivatized polynucleotide seriously restricts its transport into living cells. Second, the phosphodiester linkages in the agents are susceptible to rapid cleavage by cellular nucleases -- particularly when the gene inactivating agents are taken into cells via endocytosis.

More recently, recombinant DNA methods have allowed for the design of plasmids which carry a anti-sense gene whose RNA transcript can recognize and bind to a particular target single-strand polynucleotide. Introduction of such a plasmid into cells results in continuing production of anti-sense mRNA which appears to pair with its complementary target mRNA and thereby block its translation (Izant and Weintraub (1984), Cell, 36:1007; Izant and Weintraub (1985), Science, 229:345). A particular merit of this approach is that successful introduction of even a single such plasmid could potentially effect continued inactivation of the targeted gene activity and/or permanent resistance to the pathogen containing the targeted genetic sequence. The approach is severely limited, however, by the problems of transforming a large number of tissue or organ cells with the plasmid construct.

Attempts to design a polynucleotide binding agent which is both readily taken up by cells and stable against intracellular degradation have focused on polynucleotide analogs which have uncharged or substantiallY uncharged backbones. One of the earliest reports of such uncharged comppounds involved a number of polynucleotide analogs in which the normal sugar-phosphate backbone of nucleic acids was replaced by a polyvinyl backbone. These nucleic acid analogs were shown to have normal Watson/Crick pairing specificities with complementary polynucleotides -- but with substantially reduced Tm values (Pitha and Pitha (1970), Biochem. Biophys, Acta, 204:39).

Subsequently, uncharged and substantially uncharged heteropolymeric polynucleotide analogs more nearly isostructural with nucleic acids have been prepared for the purpose of in vivo inactivation of targeted single-stranded genetic sequences. These substances include both aryl nitrogen mustard derivatized (Karpova et al (1980), FEBS Letters, 122:21) and underivatized (Richard Tullis, Personal. Communication, Molecular Biosystems, Inc., San Diego) oligonucleotides having their phosphates in the uncharged triester state. A novel class of polynucleotide analogs which has recently been reported contain predominantly uncharged, atactic methylephosphonate backbone linkages, or homochiral methylphosphonate linkages alternating with charged, achiral phosphodiester linkages (Miller, Yano, Yano, Caroll, Jayaraman, and Ts'o (1979), Biochem. , 18:5134; Miller, Dreon, Pulford, and Parland (1980), J. Biol. Chem., 255:9659; Murakami, Blake, and Miller (1985), Biochem., 24:4041; Blake, Murakami, Spitz Glave, Reddy, Ts'o, and Miller (1985), 24:6132; Miller, Agris, Aurelian, Blake, Murakami, Reddy, Spitz and Ts'o (1985), Biochimie, 67:769).

The above-described uncharged or substantially uncharged oligonucleotide analogs have been reported to enter living cells and pair with complementary genetic sequences therein. In particulars it has been found that optimal uptake by the cells is achieved if the compounds are designed with one or a few ionic groups per molecule, to enhance solubility in aqueous medium. IF the charge density on the backbone is increased significantly, however, problems of poor transport across the cell membrane are encountered. Also, greater stability to nucleases is achieved if phosphodiester linkages are avoided.

The above uncharged and substantially uncharged polynucleotide analogs all have chiral backbone linkages, i.e., linkages in which two possible stereoisomeric forms are allowed. As a consequence, the backbone of the polymer produced by linking subunits has a random sequence of left- and right- handed

forms. This type of backbone structure, which is also referred to as an atactic backbone, is characterized by a broad range of analog/target binding constants. The mean binding constant can be significantly reduced with respect to the binding constant between normal complementary polynucleotides.

For the phosphotriester and methylphosphonate-linked nucleic acid analogs this binding constant reduction and broadening presumably derives from one linkage isomer favoring Watson/Crick pairing of proximal bases and the other linkage isomer inhibiting Watson/Crick pairing of proximal bases. This linkage chirality effect on base-pairing has been well established by studies involving two separated isomeric forms of a methylphosphonate-linked dideoxyribonucleoside. Each of these dimers was mixed with its complementary polyribonucleotide. One isomer was found to bind with a Tm of 19.8 °C while the other isomer had a corresponding Tm value of only 15.4 °C (Miller, Dreon, Pulford, and Parland (1980), J. Biol, Chem, 255:9659). These studies were further extended to examine Tm binding values in homoisomeric decamers formed by linking the isomeric methylphosphonate dimers with normal phosphodiester linkages. The decamer formed from dimers having the preferred isomeric methylphosphonate linkages paired with its complementary genetic sequence with a Tm of 33 °C. By contrast, the corresponding decamer containing the other isomeric form of methylphosphonate linkages failed to pair with its complementary genetic sequence even at 0 °C (Miller, Dreon, Pulford, and Parland (1980), J. Biol, Chem, 255:9659).

On theoretical grounds there is reason to believe that for a sequence-specific polynucleotide-binding agent to be of therapeutic and/or diagnostic value, it should pair with its complementary target sequence with a substantially uniform binding constant. The rationale here is that if a given preparation contains multiple molecular species, each with its own separate target binding constant, then those species having lower binding constants will contribute little to the target binding activity while some of those having significantly higher binding constants also form moderately stable mispaired complexes with nontarget sequences. In a therapeutic context any significant binding to nontarget sequences has the potential of generating toxic side effects, while in a diagnostic context such binding is expected to lead to high backgrounds and/or false positives.

In principal, using currently available technologies it should be possible to prepare the foregoing charged or partially charged and chirally linked nucleic acid analogs having substantially uniform target binding constants. One method for accomplishing this would involve binding the the polymer solution, which contains polymer species with a wider range of Tm values for a given target sequence, to an affinity column having the target sequence in an immobilized form. The analog is then eluted with with a gradient of an eluant, such as formamide, which will release increasingly more tightly bound analog at higher eluant concentrations, and/or by eluting while slowly increasing the temperature. A narrow cut of the eluant should contain primarily those molecules having a substantially uniform target binding constant. Achieving greater binding homogeneity with this method obviously means reducing the final yield of usable polymer substqntially. Alternatively, it is possible that uncharged oligonucleotide analogs having tactic linkages (i.e., all molecules having the same sequence of chiral centers) could be prepared by means of a geometric assembly procedure in which one of the two stereoisomers formed at each subunit addition step is retained, and the other discarded. The limitation of this procedure, of course, is that at least half of the material is discarded at each subunit addition step, so that the yield can be at most $1/2^n$ of the starting material, where n is the number of rounds of couplings required in the synthesis.

Thus, although it may be possible to prepare uncharged polynucleotide analogs having chiral intersubunit linkages which pair with their complementary target sequences with a substantially uniform binding constant, nevertheless, the synthesis and purification of such chirally linked polymers would be time-consuming and yields would necessarily be poor.

## 3. Summary of the Invention

It is therefore one general object of the invention to provide a base-specific polynucleotide-binding composition which substantially overcomes problems and limitations associated with prior art polynucleotide-binding agents.

Related objects of the invention include:

(a) Providing such a composition whose polymer molecule components all have substantially the same binding affinity with respect to a target sequence in a single-stranded polynucleotide;

(b) Providing such a composition which can be designed for targeting against virtually any nucleotide target sequence within a length range of between about 5-30 base pairs, and which can be prepared in relatively high yield;

(c) Providing such a binding composition having a desired binding affinity for a selected target sequence; and

(d) Providing such a binding compound which is suitable for a variety of diagnostic and therapeutic applications which take advatage of the of the stereoregular, uncharged or substantially uncharged nature of the backbone linkages in the compound.

The invention relates to a polymeric composition designed to bind, with a selected binding affinity, to a single-stranded polynucleotide containing a target sequence of bases. The composition includes non-homopolymeric, substantially stereoregular polymer molecules of the form:

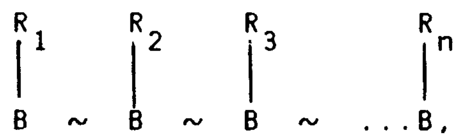

where:

(a) $R_1$-$R_n$ are recognition moieties selected from purine, purine-like, pyrimidine, and pyrimidine like heterocycles effective to bind by Watson/Crick pairing to corresponding, in-sequence bases in the target sequence;

(b) n is such that the total number of Watson/Crick hydrogen bonds formed between a polymer molecule and target sequence is at least about 15;

(c) B ~ B are backbone moieties joined by chemically stable, substantially uncharged, predominantly achiral linkages;

(d) the unit length of backbone moieties is 5-7 atoms if the backbone moieties have a cyclic structure, and 4-6 atoms if the backbone moieties have an acyclic structure; and

(e) the backbone moieties support the recognition moieties at positions which allow Watson/Crick base pairing between the recognition moieties and the corresponding, in-sequence bases of the target sequences.

Thus the present invention provides a polymer composition effective to bind, with a selected binding affinity, to a single-stranded polynucleotide containing a target sequence of bases, comprising non-homopolymeric, substantially stereoregular polymer molecules of the form:

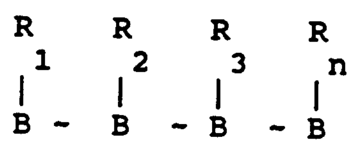

where:

(a) $R_1$ - $R_n$ are recognition moieties selected from purine, purine-like, pyrimidine, and pyrimidine-like heterocycles effective to bind by Watson/Crick pairing to corresponding, in-sequence bases in the target sequence;

(b) n is such that the total number of recognition moieties is at least 5;

(c) B ≈ B are backbone moieties joined predominantly by chemically stable, substantially uncharged, predominantly achiral linkages selected from the group consisting of:

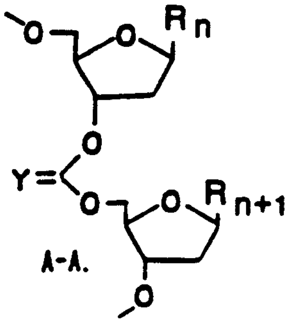

A-A.

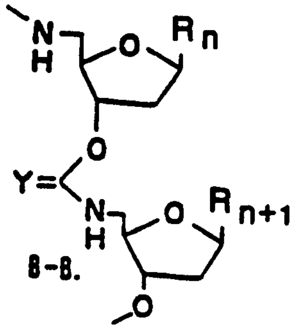

B-B.

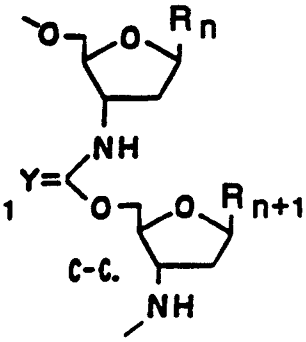

C-C.

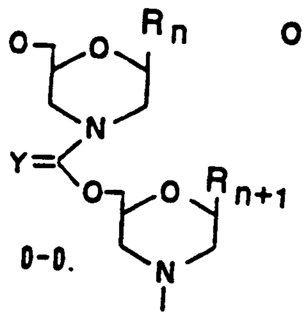

D-D.

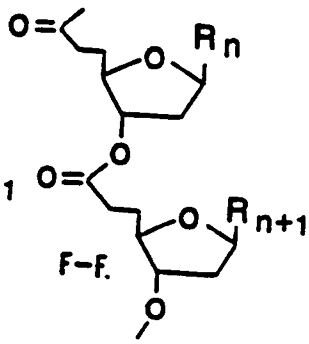

F-F.

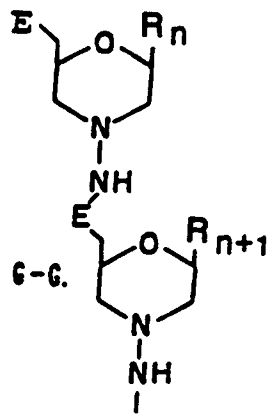

G-G.

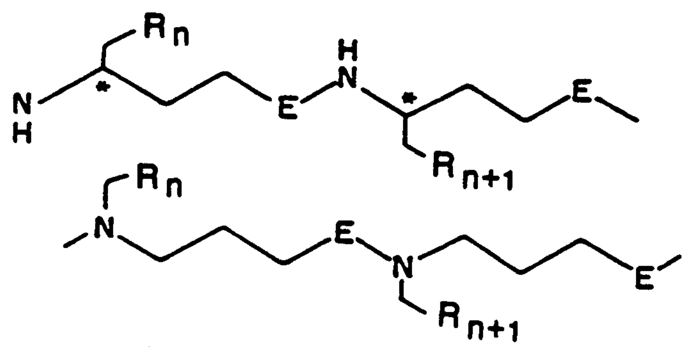

I-I, L-L

J-J, M-M

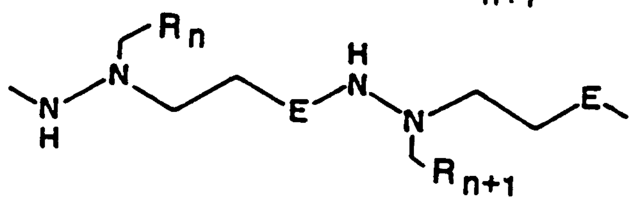

K-K, N-N

where Y = O or S, and

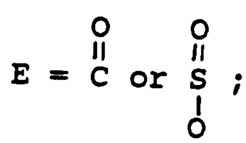

and

(d) the backbone moieties support the recognition moieties at positions which allow Watson/Crick base pairing between the recognition moieties and the corresponding, in-sequence bases of the target sequences.

The invention further provides a method of preparing a substantially uncharged polymeric composition effective to bind specifically and with a substantially uniform binding affinity to a single-stranded polynucleotide, said method comprising:

(1) selecting a specific sequence of bases in the polynucleotide, said sequence constituting the target sequence;

(2) providing a group of subunits of the form B-R, where:

(a) R is a recognition moiety selected from purine, purine-like, pyrimidine, and pyrimidine-like heterocycles effective to bind by Watson/Crick pairing involving two or three hydrogen bonds to corresponding bases contained in the selected target-specific sequence;

(b) B is a backbone moiety which can be coupled to another backbone moiety B by a chemically stable, uncharged, achiral linkage selected from the group consisting of:

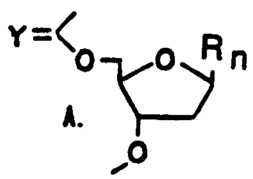
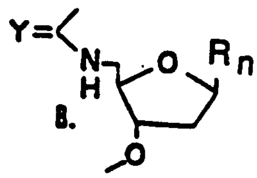
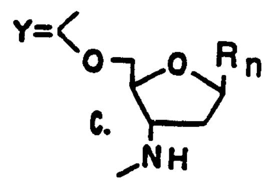
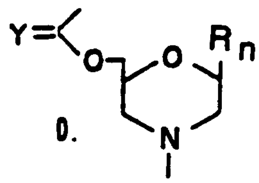
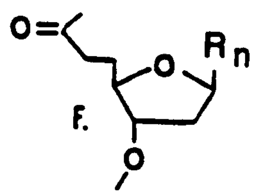
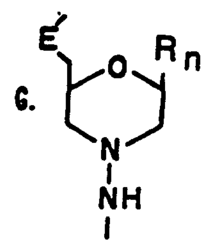

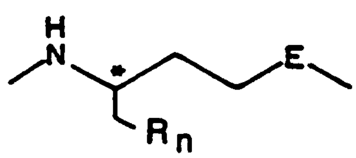

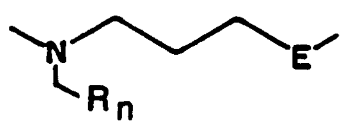

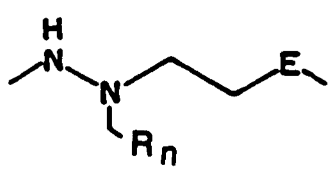

where Y = O,or S, and

E = 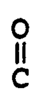 or 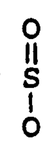 ;

and

(c) the backbone moieties, when coupled, form a backbone which supports the recognition moieties at positions which allow Watson/Crick base pairing between the recognition moieties and the corresponding, in-sequence bases of the target sequences; and

(3) coupling at least 5 subunits in a defined sequence corresponding to the sequence of bases in the target-specific sequence.

The invention also provides a method for inhibiting expression of a single-stranded polynucleotide contained in a system in which a selected activity is expressed, said method comprising:

(1) selecting a target sequence in the polynucleotide which is required for such expression,

(2) providing a group of subunits of the form: B ~ R, where:

(a) R is a recognition moiety selected from purine, purine-like, pyrimidine, and pyrimidine-like heterocycles effective to bind by Watson/Crick pairing involving two or three hydrogen bonds, to corresponding bases contained in the selected target sequence;

(b) B is a backbone moiety which can be coupled to another backbone moiety a by a chemically stable, uncharged, achiral linkage selected from the group consisting of:

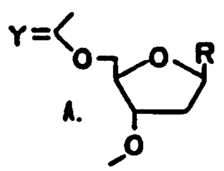
A.

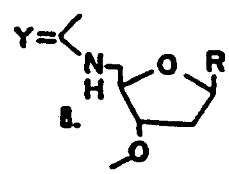
B.

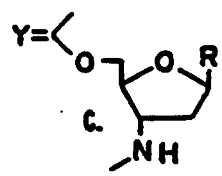
C.

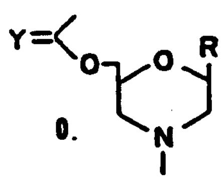
D.

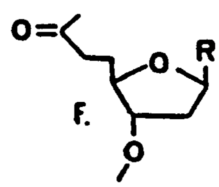
F.

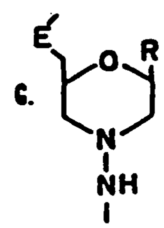
G.

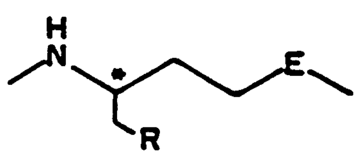

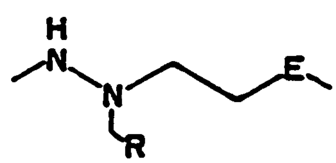

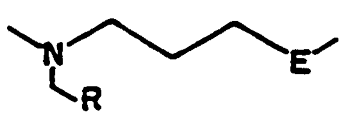

where Y = O or S, and

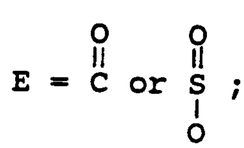

and

(c) the backbone moieties, when coupled, form a backbone which supports the recognition moieties at

positions which allow Watson/Crick base pairing between the recognition moieties and the corresponding, in-sequence bases of the target sequences;

(3) coupling at least 5 subunits in a defined sequence to form a predominantly uncharged, stereoisomer polymer designed to bind specifically to the target sequence,

(4) adding the polymer to the system and observing the extent of inhibition of the polynucleotide expression,

depending on the degree of inhibition of polynucleotide expression observed, adjusting the binding affinity of the polymer for the target sequence by one or both of the steps of (a) varying the length of the target sequence and the corresponding polymer length, or (b) varying the ratio of recognition moieties which form three and two hydrogen bonds with corresponding in-sequence polynucleotide bases; and

by said adjusting producing a polymer whose binding affinity is substantially the lowest affinity which is required to effect such inhibition of polynucleotide expression.

The invention also provides a morpholino subunit of the form:

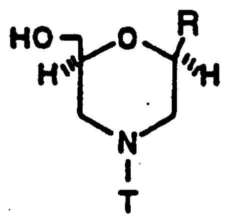

where R is a base-pairing moiety capable of binding by Watson/Crick pairing to complementary bases in a polynucleotide,

and where T is H or an N-protective group.

In a preferred embodiment for use in therapeutic applications, the polymeric molecules, have a small number of charges, to increase compound solubility in aqueous medium. The charges may be attached at end regions of the molecules or at achiral charged backbone linkages.

The polymer molecules are constructed, according to the method of synthesis of the invention, by first selecting a sequence of bases in the target polynucleotide. The sequence is typically between about 5-32 bases, and preferably between about 8-20 bases, corresponding to the number of subunits in the final binding polymer. The binding affinity of the binding polymer for the target polynucleotide can be increased by increasing the number of target-sequence bases (and correspondingly, the number of subunits in the binding polymer). Polymer subunits of the form $B-R_i$ and/or $B-R_j$ are then coupled in a defined sequence corresponding to the sequence of bases in the target-specific sequence. Here $R_i$ and $R_j$ are recognition moities, as defined above, which are capable of forming Watson/Crick base pairing with corresponding polynucleotide bases through either two or three hydrogen bonds, respectively. The binding affinity or the Tm of the binding polymer for the target polynucleotide can be selectively varied according to the ratio of $R_i$ to $R_j$ recognition moieties used in forming the polymer, with a greater percentage of $R_i$ recognition moieties producing a lower binding affinity for the target polynucleotide.

The polymers are useful in a diagnostic system for detecting an analyte polynucleotide having a defined target base sequence. The system includes a support reagent composed of the polymer molecules of the invention linked to a solid support. In one embodiment of the invention, the polymer binding molecules are predominantly uncharged. In another embodiment, the backbone moieties are joined by achiral charged linkages, such as phosphodiester linkages, alternating with one or more uncharged achiral linkages.

In practicing the assay method, a polynucleotide analyte is added to the diagnostic reagent under conditions in which the analyte is in a single-strand form and which allow sequence-specific pairing between the analyte and the reagent polymers. After the analyte/polymer annealing reaction, the reagent is washed to remove unbound test material. The reagent and bound analyte are then reacted with a oligocationic reporter which is designed to bind by charge attraction to the charged, phosphodiester linkages of the bound analyte, but not to the uncharged or substantially uncharged polymer molecules. One feature of the system is that the ratio of bound reporter to bound analyte polynucleotides (the number of reporter molecules bound per analyte molecule) can be $10^2$-$10^4$, giving a highly sensitive assay.

In therapeutic applications, the polymer composition is designed to inhibit expression of a selected target single-stranded polynucleotide, such as an RNA virus. Here the polymer is preferably constructed to bind spsecifically to the target polynucleotide, with the minimum binding needed to produce the desired

inhibition of target expression, to minimize non-specific binding effects.

These and other objects and features of the present invention will become more fully apparent when the following detailed description of the invention is read in conjunction with the accompanying figures.

## Brief Description of the Figures

Figure 1 shows preferred purine and pyrimidine structures used in forming polymer molecules of the invention;

Figure 2 shows preferred purine-like and pyrimidine-like recognition moieties used in forming the. polymer molecules;

Figure 3 shows preferred cyclic backbone moities used in forming the polymer molecules;

Figure 4 shows preferred acyclic backbone moieties used in forming the polymer molecules;

Figures 5A and 5B illustrate two preferred subunit assembly schemes for coupling $N_1$---$N_2$ type subunit backbones;

Figure 6 shows the backbone structures of subunit dimers A-A through D-D formed in accordance with the methods illustrated in Figures 5A and 5B, and two cyclic backbone structures formed in accordance with the methods illustrated in Figures 7A and 7B;

Figures 7A-7C illustrate three preferred subunit assembly schemes for coupling $N_1$---E type subunit backbones; and

Figure 8 shows preferred acyclic backbone structures of subunit dimers formed in accordance with the methods illustrated in Figures 7A-7C;

## Detailed Description of the Invention

The polymeric composition of the invention is designed to bind, with a selected binding affinity, to a polynucleotide containing a target sequence of bases. The composition is composed of non-homopolymeric substantially stereoregular molecules or species of the form:

$$\begin{array}{ccccccc} R_1 & & R_2 & & R_3 & & R_n \\ B & \sim & B & \sim & B & \sim \ . \ . \ . & B, \end{array}$$

where B-$R_i$ are a series of base-specific subunits containing a backbone moiety B and a recognition moiety $R_i$ which is selected to bind specifically by Watson/Crick base pairing to a corresponding, in-sequence base in the target sequence, and the subunits are joined through their backbone moieties predominantly achiral, substantially uncharged bonds. The design and selection of suitable subunits for use in constructing the polymer species will be be described in Section I below. Methods for coupling subunits through achiral linkages are described in Section II, with subunit-protective group strategies involved in subunit synthesis being discussed in Section II.

The polymer species are synthesized by sequential subunit coupling, to form polymer molecules of a selected length and sequence. As will be described in Section IV, the length of the target sequence and polymer sequence is selected to achieve a desired birding specificity. The binding affinity of the polymer for the target can be selectively varied by several strategies discussed in Section IV, including the choice of recognition moieties which are capable of forming either three (for greater binding affinity) or two (for lesser affinity) base-paired hydrogen bonds with the corresponding target base. The resulting composition contains polymer species or molecules, all having substantially the same sequence of subunits and substantially the same sequence of intersubunit linkage types. In functional terms, all of the polymer species have substantially the same binding affinity for the target polynucleotide. Methods for assembling the polymer, once a desired subunit sequence is selected, are given in Section IV.

The polymer composition is useful in a novel solid-phase diagnostic system which is described in Section IV. This system is based on the binding of analyte polynucleotide molecules to support-bound polymer molecules and subsequent elctrostatic attachment of multiple polycationic reporter molecules to the polynucleotide, to give an amplified reporter signal for each bound analyte molecule. The polymer is also useful in a variety of solution applications, including therapeutic applications, which are also considered in Section IV.

I. Subunit Structure

A. The Recognition Moiety

The design of the subunits B-$R_i$ which are suitable for use in forming the polymer species of the invention involves a number of structural and/or stereochemical criteria which must be met both by the backbone and recognition moieties and by the linkage between the two. The design requirement of the recognition moiety will be considered first.

The recognition moiety of each subunit must provide two or three hydrogen-bonding groups held in an unambiguous configuration adapted for hydrogen bonding to two or three of the Watson/Crick hydrogen bonding sites on a specified in-sequence base of the target genetic sequence. To avoid undesired mispairing between recognition moiety and its corresponding target base, under the conditions of use, (1) the tautomeric state of the recognition moiety should be relatively stable, and (2) the recognition moiety should have a structure which provides a relatively rigid arrangement of the hydrogen-bonding groups. Such rigidity is best afforded by a ring structure having the polar hydrogen-bonding groups either forming part of the ring or directly attached to the ring.

The preferred recognition moiety structures include purine, purine-like pyrimidine, and pyrimidine-like structures which are designed to form Watson/Crick base pairing, through either two or three hydrogen bonds, with selected polynucleotide bases. The group of subunits used in polymer synthesis includes at least two recognition moieties which are base-specific for different polynucleotide bases, and preferably one or more recognition moieties for each of the four bases in a natural DNA or RNA polynucleotide. Also, as will be seen below, it is desirable to provide one group of recognition moieties, $R_i$, which are capable of base-pairing with nucleotide bases through two hydrogen bonds, and a second group, $R_j$ which are capable of binding with the same bases through three hydrogen bonds. Figure 1 shows exemplary purine and pyrimidine type recognition moieties. The purine structures 1 and 2 are designed to bind to thymine or uracil bases, structures 3-6, to guanine bases; structures 7-9, to cytosine bases, and structures 10-12, to adenine bases Structures 1, 4, 5, 6, 8, and 10-12 are $R_i$ type moieties adapted to bind to corresponding in-sequence bases through two hydrogen bonds, and the remaining structures are $R_j$ type moieties which form three hydrogen bonds on base pairing. As will be seen below, purine and pyrimidine nucleosides are useful in synthesizing a variety of other subunits which are suitable for use in polymer synthesis. These subunits, modified if necessary with amine protective groups, can be obtained from commercial sources, or prepared according to known methods, such as those described or referenced in Example 1.

A number of purine-like or pyrimidine-like structures in which the recognition moiety is joined to the backbone through an annular carbon are shown in Figure 2. These structures have base pairing specificities and hydrogen bonding properties like those of the analogous structures shown in Figure. Although the binding properties of a polymer containing a carbon-linked recognition moiety may not be significantly different from that of a polymer whose recognition moities are nitrogen-linked, as in Figure 1, subunits incorporating the carbon-linked moieties generally require a greater synthetic effort than do subunits containing preformed purine and pyrimidines. However, for some subunit syntheses, such as the synthesis of the achiral acyclic backbone subunits described in Example 10, the carbon-linked recognition moieties provide a useful starting material.

B. The Backbone Moiety

The backbone moiety of each subunit has the general form $N_1$---E, or $N_1$---$N_2$, where $N_1$ and $N_2$ are nucleophilic groups and E is an electrophilic group. Based on ease of subunit coupling and required stability of the resultant intersubunit linkage, as discussed below, the preferred nucleophilic groups are amino, hydroxyl and hydrazino; and the preferred electrophilic groups, and/or electrophilic linking agents, are derivatives of carbonic, thiocarbonic, carboxylic, and sulfonic acids.

Backbone moieties can have either a cyclic or an acyclic structure. While the total number of possible backbone moiety structures is very large, nevertheless, only a rather limited number of structures are of actual practical value due to a number of factors. The initial procedure by which promising backbone moieties were selected was as follows.

As a first conditions, only those cyclic backbone moieties were considered which consisted of, or could be readily derived from, deoxyribose or ribose. This limitation is a practical one and reflects the difficulty and corresponding greater expense of de novo synthesizing ring structures having multiple chiral centers. In general prospective backbone moieties and intersubunit linkages expected to be suitable for polymers were selected on the basis of one or more of the following factors: feasibility of synthesis based on known

reactions; expected ease of synthesis from available starting materials; simplicity of structure; and expected stability of the final backbone.

Initial screening of promising backbone moieties and intersubunit linkages was performed as follows. Space-filling CPK molecular models of duplex DNA and RNA were constructed according to parameters determined by x-ray diffraction of oligodeoxyribo nucleotides in the B-Form and oligonucleotides in the A-form. In each of these constructed duplexes one of the two sugar-phosphate backbones was removed. Next, each prospective backbone was attached, if possible, to the sites on the bases form which the original sugar-phosphate backbone had been removed. Each resulting polynucleotide/polymer duplex was then examined for coplanarity of the Watson/Crick base-pairs, torsional and angle strain in the prospective polymer backbone, degree of distortion imposed on the nucleic acid strand, and interstrand and intrastrand nonbonded interactions. Special attention was paid to whether or not each amide-containing backbone could readily adopt a conformation wherein its amide moieties were planar. This is important because of the substantial energy cost required to force an amide into a nonplanar conformation.

These initial studies verified that the required unit backbone length (i.e., the $N_1$---E spacing in an $N_1$---E or activated $N_1$---$N_2$-E subunit) is 5-7 atoms for backbone moieties constituting or derived from deoxyribose or ribose (cyclic backbone structures), with a 6-atom length being optimal.

Subunit structure judged acceptable in the above modeling studies were then assessed for feasibility of synthesis (on the basis of key synthetic reactions reported in the literature, or reactions carried out on model compounds, and/or via actual synthesis of the subunits) and stability of the assembled polymer backbone (preliminary stability studies were generally carried out with suitably linked model compounds or subunit dimers). These types of studies were used to further restrict the number of candidate backbone structures. From this restricted candidate pool the cyclic backbone structures A-G shown in Figure 3 were ultimately selected as preferred structures.

In this figure, subunits A-D containing $N_1$----$N_2$ type cyclic backbone moieties include: 2'-deoxyribonucleosides (structure A); 2'-deoxyribonucleosides substituted at the 5' and 3' positions with an amino group (structures B and C, respectively); and morpholino derivatives of ribonucleosides (structure D). Subunits containing $N_1$---E-type backbone moieties include 2'-deoxyribonucleosides substituted at their 5' positions with one and two-carbon acids (structures E and F, respectively); and N-aminomorpholino derivatives of ribonucleosides substituted at their 5' positions with carboxylic acid (structure G) or sulfonic acid (structure H).

The same molecular modeling approach applied to acyclic (unbranched chain) backbone moieties showed that unit backbone lengths ranging from 4-6 atoms are acceptable, in terms of polymer conformation in base-pair hydrogen bonding to single-stranded polynucleotide, with a 5-atom backbone length being optimal This is in contrast to cyclic backbones, where a 6-atom unit backbone length is optimal. Further, the modeling work showed that the annular recognition moiety cannot be directly attached to the linear backbone moiety without severely inhibiting of coplanarity of the complementary bases and introducing undesirable interactions between the recognition moiety and the backbone the minimum binding strain occurred when the recognition moiety was linked to the backbone moiety by a one-atom spacer. Two-atom, but not three-atom, spacers may be tolerated, although increased degrees of freedom between the recognition moieties and the linear backbone may lead to mispairing with target bases with two-atom spacers.

The acylic backbone moiety structures I-N shown in Figure 4, all of which contain a one-atom methylene spacer between the backbone and recognition moieties, gave a good modeling prediction for favorable base-pairing to a complementray polynucleotide. Analogous structures containing a two-carbon spacer showed acceptable, but less favorable, binding conformation. The structures shown in the figure, and the analogous two-carbon spacer structures, are preferred acylcic backbone moieties because of their general ease of synthesis. It should be mentioned, however, that a number of other backbone moieties are also entirely feasible and suitable -- although generally not as easy and/or inexpensive to synthesize.

C. Backbone/Recognition Moiety Linkage

As indicated above, the linkage or spacer connecting the backbone and recognition moieties in the polymer subunit must meet certain design criteria which are effective to position the recognition moieties for base-pair binding to the polynucleotide bases. In the case of the cyclic backbone moieties shown in Figure 3, modeling studies indicate that the most favorable binding conformation in the polymer occurs when the recognition moiety is attached directly to the 1' carbon of the ribose or ribose-like structures, and to the analogous 1' position of the morpholino structures. That is, the moiety is attached at normal point of attachment and with the normal stereoisomeric configuration of purine or pyrimidine bases to the ribose or

deoxyribose groups of nucleosides. For the acyclic structures, one-and two-carbon atom spacers are required for placing the recognition moieties at favorable binding positions, with one-atom spacers being preferred, as discussed above.

In order to achieve a stereoregular polymer configuration required for uniform binding affinity of the polymer molecules to a polynucleotide target, it is also necessary that all of the backbone/recognition moiety linkages be either of define chirality or achiral The linkages are considered to be of defined chirality, for purposes of definition herein, if each of the linkages in any given subunit position in all of the polymer molecules has the same stereoisomeric configuration, or chirality That is, although the subunits in different sequence positions may have different internal stereoisomer configurations (including achirality at specific sequence positions), all of the linkages at a given sequence position among the polymer molecules have the same stereoisomeric configuration. Usually defined chirality is most easily achieved by using the same stereoisomeric configuration in all of the subunits.

For the cyclic backbone moieties, the most favorable binding occurs in nucleoside analogs having the natural D-steriosomeric configuration shown in Figure 3. Subunits of this type are also readily synthesized as will be discussed below, using natural D-nucleoside starting material. With reference to Figure 4, it is seen that only structures I and L have chiral linkages between the backbone and recognition moieties. These subunits are readily synthesized in homochiral form by using homochiral starting material as will be described. For all of the other acyclic structures shown in Figure 4, the one-atom linkages are attached to backbone nitrogen atoms, are are therfore achiral. Also, of course, the methylene spacers are themselves achiral.

II. Protective Group Strategies

Because of the rather high reactivity of the compounds used For activating and/or coupling the subunits, it is generally desirable, and often necessary, to protect the exocyclic ring nitrogens of the recognition moieties. Selection of these protective groups is primarily determined by the type of intersubunit linkage to be used in the polymer assembled from these subunits, and secondarily determined by the relative reactivity of the nitrogen to be protected.

Base-protected nucleosides are also useful starting reagents in a number of the subunit synthesis reactions to be described below. Methods for base protecting a number of the more common ribo- and deoxynucleosides from Example I are illustrated in Example 2. The methods detailed in the example are generally applicable for forming nucleosides with amine-protective groups.

When the intersubunit linkage to be used is relatively stable to nucleophiles, and particularly to ammonium hydroxide, then the standard base-protective groups used for nucleic acid chemistry are suitable. Such nucleophile-insensitive intersubunit linkages include carbamate, amide, and sulfonamide linkages. The corresponding nucleophile-sensitive protective groups for the recognition moieties include: benzoyl for the N4 of C; benzoyl or p-nitrobenzoyl for the N6 of A; acetyl or isobutyryl for the N2 of G; and N2,N6-bisisobutyryl for 2,6-diaminopurine residues. Removal of these groups after completion of polymer assembly is effected by treatment with ammonium hydroxide.

In contrast, when the intersubunit linkage to be used is sensitive to nucleophiles, such as ammonium hydroxide, suitable protective groups are those which can be removed by strong non-nucleophilic bases -- via a $\beta$ elimination mechanism. Such nucleophile-sensitive intersubunit linkages include: carbonate; ester; and, to a lesser extent, triocarbamate linkages. Suitable protective groups for the recognition moieties removable via $\beta$ elimination include: 2-(4-nitrophenyl)ethoxy carbonyl or 2-(phenyl sulfonyl)ethoxycarbonyl for both the N4 of C and the N6 of A; and the 9-fluorenyl methoxycarbonyl for the N2 of G and the N2 and N6 of 2,6-diaminopurine residues. Removal of these groups after completion of polymer assembly is effected by treatment with the strong nonucleophilic base 1,8-diazabicyclo[5.4.0]undec-7-ene (DBU), under stringently anhydrous conditions.

In regard to temporary protection of a nucleophile of the backbone moiety (generally N1 in the above structures), in the general polymer assembly strategy employs selected backbone-protective groups which are readily cleared by mild acid. One primary criterion for selection of protective groups is that they be adequately stable, but not so stable that conditions required for their removal would damage the growing polymer. A principal problem in the case of polymers assembled from cyclic backbone moieties is the particular acid sensitivity of the glycosidic bond linking protected purine residues to the C1 of their backbone moieties. A secondary criterion for selection of the backbone protective group is that it be easily introduced. Based on the above, the following backbone-protecting groups are preferred: for primary hydroxyls, the di(p-methoxy)trityl group; for primary amines, p-methoxytrityl; and for a secondary amine (as in morphilino-type backbone moieties), the phenylisopropoxycarbonyl group. These protective groups can

be readily removed by treatment with 0.2 M dichloroacetic acid in dichloromethane.

III. Subunit Synthesis

A. Cyclic Backbone Moieties

Subunits having a deoxydeoxynucleoside subunit structure (structure A in Figure 3) can be obtained from commercial sources or prepared via literature methods, as described in Example I. The subunits include the following ribosides and deoxyribosides, which are identified according to the struture numbers of the recognition moieties given in Figure 1: adenosine and deoxyadenosine (structure 1); 2,6-diaminopurine riboside and deoxyriboside (structure 2); cytodine and deoxycytodine (structure 3); 4-methoxy-2-pyrimidinone deoxyriboside (structure 4); 2-hydroxy-5-methyl pyrimidine deoxiriboside (structure 5); 2-hydroxypyrimidine riboside (structure 6); guanosine and deoxyguanosine (structure 7); inosine and deoxyinosine (structure 8); thioguanosine and deoxythioguanosine (structure 9); uradine and deoxyuridine (structure 10); thymidine and 5-methyluridine (structure 11;) and 5-halouridines and 5-halodeoxyuridines (structure 12).

The 5'-amino-2',5'-dideoxyribonucleosides (structure B in Figure 3) are prepared according to methods detailed in Example 3. Briefly, the selected deoxyribonucleosides, base-protected if necessary, are reacted with triphenyl phosphine, carbon tetrabromide, and lithium azide to form the corresponding 5'-azidonucleoside, which is then reduced by hydrogenation in the presence of a palladium on carbon catylyst. The nucleosides may be obtained as in Example 1, and base-protected as in Example 2. The stereochemistry of the reactant nucleosides is preserved in forming the 5' amino nucleoside analogs.

An alternative reduction method is used in reducing the azide group of 5'-azido-5-bromo uridine, as described in Example 3.2. Here non-catalytic hydrogenation is needed to prevent removal of the ring bromine atom. In Forming the 5'-amine guanosine compound, the azide is placed on the 5' position by first tosylating the 5' hydroxyl group, then displacing with azide, as detailed in Example 3.2.

The 3'-amino-2',3'-dideoxyribosnucleosides (structure C in Figure 3) are prepared according to methods detailed in Example 4. Briefly, thymidine which is protected at its 5' hydroxyl is tosylated at its 3' hydroxyl, and this is followed by an intramolecular displacement involving the 2 oxy base substituent. The resulting ring is now opened by treatment with azide, yielding a 3' azido analog of the correct stereoisomeric form. This analog can be be converted to a selected-base analog by reacting the azide compound with a selected purine or pyrimidine base. The latter may be base protected, if necessary, for subsequent subunit coupling reactions, as described below. The thymidine or other azide analog is then reduced to produce the desired 3' amine nucleoside. The stereochemistry is of the thymidine starting material is preserved in the synthesis.

Synthesis of the morpholino-type subunit derivatives represented by structure D in Figure 3 are detailed in Example 5 for a variety of different recognition moieties. Briefly, a selected nucleoside, base-protected if necessary, is dissolved in an ammonium salt, such as ammonium biborate, and then reacted with sodium periodate to form transient 2' 3' dialdehydes, which then close upon an ammonium ion to form morpholino ring having 2' and 4' hydroxyls. The compound is then treated with sodium cyanoborohydride to remove the ring hydroxyls. The ring nitrogen is preferably protected as a 2-phenylisopropylcarbamate for subsequent subunit coupling. The sterochemistry of the nucleoside starting material is retained.

Deoxyribose subunit structures having a 5' acetic acid group (structure F in Figure 3) can be prepared according the general synthetic scheme described in Example 6, which details the synthesis of the 5' acetic acid compounds represented by structure F. Here a selected deoxyribonucleoside which is protected at the 3' hydroxyl is converted to the 5' aldehyde, and subsequently treated with a 2-carbon Wittig reagent to give the unsaturated acetic acid derivative. Reduction of the side-chain olefin gives the desired 5' acetic acid compound. The reaction preserves the sterochemistry of the starting nucleoside material

The analogous 5' formate compound (structure E in Figure 3) is formed by treating the above 5' nucleoside aldehyde with a one-carbon Wittig reagent, and hydroslysing the resulting enol to form form the corresponding formaldehyde derivative, which is oxidized to the desired acid. The reaction preserves the stereochemistry of the starting nucleoside material.

B. Subunit Synthesis-Acyclic Backbones

The 5-atom chain amino acid subunit represented by Structure I in Figure 4 is prepared according to the general procedures outlined in Examples 7-9. Briefly, an (S)-5 carboxy pyrrolidone was converted to the corresponding stereochemically pure (S)-5-tosylmethyl-2-pyrrolidone by known methods, and then reacted with a selected purine or pyrimidine, to form the corresponding (S)-5-methylpurine or pyrimidine pyrrolidone. The displacement reaction retains the original stereospecificity, so that the subunits which are formed are homoisomeric at the site of attachment of the recognition moiety to the backbone carbon.

The purine used in subunit synthesis is preferably contains an electron-withdrawing group at the 6 position, to reduce the reactivity of the 1 and 3 ring nitrogens, i.e., to minimize pyrrolidone coupling at a ring nitrogen. The pyrrolidone-derivized purine or pyrimidine may then be converted to the amine derivative, and base protected, if necessary, prior to the ring opening step which will be described below. Example 7.1 and 7.2 detail methods for forming the cytosine pyrrolidone and its base-protected derivative. The adenosine pyrrolidone formed in Examples 7.3-7.5 employs 6-chloropurine as the starting material, and the corresponding pyrrolidone is converted to the adenosine derivative through azide formation as described. The adenosine is base-protected, as in Example 7.6, prior to ring opening. A similar approach is used to generate base protected guanine pyrrolidone, starting from 2-amino-6 chloropurine, as described in Examples 7.7-7.9. Similar methods are used in synthesizing 2,6-diaminopurine pyrrolidone, inosine pyrrolidone and 2-hydroxypyrimidine pyrrolidone, also as detailed in Example 7.

The pyrrolidone is then treated via a series of reactions which cleave the pyrrolidone ring to form an amino acid with a t-BOC protected amine. Examples 8.1-8.4 describe the synthesis of such t-BOC amino acids having one of a number of selected recognition moieties. In a final step (Example 5), the t-BOC protective group may be removed to form the corresponding amino acid represented by structure I in Figure 4. The amino acid may be used directly for subunit coupling, according to reactions given below. Alternatively, the t-BOC protected compound may be activated at its acid group for use in subunit coupling reactions.

The strategy for forming the subunit represented by structure J in Figure 4 is outlined in Examples 10.1 and 10.2, for forming the cytodine and uridine analog subunits, respectively. Briefly, in forming a pyrimidine type subunit, a 6-member heterocycle, such as a pyrimidine or nicotinate, is modified to contain a reactive methylene on the carbon ring position at which attachment to the backbone is desired. As detailed in Example 10.1, the cytosine analog is formed by reacting 5-amino-5-bromopyrimidine to form the corresponding 5-position carboxaldehyde. In Example 12, the uridine analog is formed by converting 6-hydroxynicotinic esters to the corresponding benzylic alcohols. This compound is converted to the aldehyde by reaction in the presence of manganese dioxide.

The aldehyde compound is then reacted with the desired amino acids, such as 4-aminobutyric acid, with reaction at the amine group producing an unstable imine which is reduced to form the stable amine subunit. The secondary amine can be protected, for subunit coupling involving activation of the acid group, by a tBOC group as described in Example 10.1. The base attachment to the backbone at a backbone nitrogen, through a methylene spacer, insures that the subunits lack chiral centers and so cannot be stereoregular.

To form the subunit structures shown at K in Figure 4, the C-carbon backbone analog of the above compound is prepared, using 3-aminoproprionic acid, and this compound is further treated with nitrous oxide to form the corresponding N-nitroso compound, which is subsequently reduced with $H_2$IPd in the presence of iron salts to give the hydrazino acid subunit.

## II. Backbone Coupling Reactions

The coupling reactions used in forming the polymer molecules of the invention are stepwise reactions which join one selected subunit or subunit sequence to another selected subunit or subunit sequence. For purposes of the present discussion, the coupling reactions will be described generally with respect to coupling a single subunit B-$R_1$ having a selected recognition moiety $R_1$ to another single subunit B-$R_2$ having the same or a different selected recognition moiety $R_2$, to form a dimer of the form

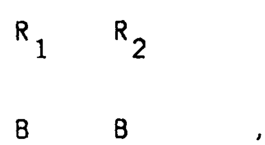

where $R_1$ and $R_2$ have the specific sequence shown.

### A. Subunit Coupling: $N_1$----$N_2$ Backbone Configurations

General methods for coupling subunits having an $N_1$--------$N_2$ backbone configuration are illustrated in

Figures 5A and 5B. As will be recalled from Section I above, $N_1$ and $N_2$ are nucleophilic backbone groups, such as hydroxyl and amine groups, which can be activated with an electrophile E, to form an activated $N_1$-E or $N_2$-E backbone group which can then react with a second $N_1$----$N_2$ type backbone moiety to form an $N_1$----$N_2$-E-$N_1$----$N_2$ backbone-linked dimer. The subunit backbones of this types which have been described specifically above are the cyclic backbone structures A-D in Figure 3. In structures A and B, the activated $N_2$ nucleophile is the 3' hydroxyl group in structure C is the 5' hydroxyl and in structure D, the 6' position hydroxyl corresponding to the 5' position hydroxyl in structure C.

In a preferred coupling method, which is illustrated in Figure 5A, a subunit having a selected recognition moiety $R_1$ is activated with an electrophile E. The star at the recognition moiety indicates any required base protection. As seen in the figure, the selected subunit is protected at its $N_1$ nucleophile, to ensure that (a) only the $N_2$ nucleophile is activated and (b) the activated subunit cannot self-polymerize. In structures A, C, and D in Figure 3, in which the backbone protection group is on the 5' hydroxyl, the protective group is preferably an acid-labile group, such as dimethoxytrityl (DMTO).

The activating reagent shown in Figure 5A has the general form:

$$\overset{X}{Y{-}{-}C{-}{-}Y}\,,$$

where X is oxygen or sulfur, and C is an active electrophile capable of reacting with a nucleophile, such as a hydroxyl oxygen or amine nitrogen, with displacement of Y, to form the activated subunit:

Activating agents, such as bis-p-nitrophenyl carbonate, which give the carbonyl activated subunit (X = O), are used in forming carbonate and carbamate subunit linkages. Similarly, activating agent, such as thiocarbonyl-di-(1,2,4-triazole) (X = S) are used in forming thiocarbamate linkages.

Subunit activation reactions involving carbonyl activated subunits are detailed in Example 11 for the 5'-protected 2' deoxynucleosides represented at A in Figure 4; in Example 12 for the 5'-protected amino nucleosides represented at B in Figure 4; and in Example 13 for the N-protected morpholino-type subunits shown at D in Figure 4. The general reaction conditions used to activate the hydroxyl groups in these structures are generally applicable; subunit activation reactions involving thiocarbonyl-activated subunits are detailed in Example 14 for the 5'-amino nucleosides represented at B; and in Example 15, for the morpholino-type subunits shown at D in Figure 3.

Following the activation reaction, the activated complex is purified by conventional methods, such as silica gel chromoatography, and then reacted with a second subunit whose selected recognition moiety $R_2$ will form the next in-sequence recognition moiety in the completed polymer. The coupling reaction is preferably carried out under mild conditions in which the activated group can react with backbone $N_2$ amine groups, but not $N_1$ hydroxyl groups. Therefore, the method is suitable for coupling subunits of the type represented by structures B-D in Figure 4, but not subunit structure A. An advantage of this coupling method is that the second subunit -- which contains an amine $N_1$ nucleophile and a hydroxyl $N_2$ nucleophile -- will be coupled to the first activated subunit only through the $N_1$ amine, so it is not necessary to protect the $N_2$ backbone group. The resulting dimer subunits are therefore coupled through an $N_2$-E-$N_1$ bond, as indicated in the figure.

The oligomer can be extended by repeating the above steps of (a) activating the free $N_2$ nucleophile in the second subunit, separating the activated species from the activating agent, and coupling the activated compound with the next-in-sequence subunit, whose backbone is unprotected. This method is used particularly in forming short oligomer blocks by solution methods which will be described below and which are suitable for solid-phase block assembly.

For forming a polymer by solid-phase sequential subunit addition, the second coupling method outlined in Figure 5B is much preferred. The second method differs from the first method in that polymer growth occurs by addition of an excess of activated subunit to an existing subunit or polymer chain, rather than, as in the first method, by addition of an unactivated subunit to an activated chain. In Figure 58, the existing subunit or subunit chain is shown by a subunit whose recognition moiety is $R_1$ (second line in Figure 6B). This subunit has a free $N_1$ backbone nucleophile and an $N_2$ nucleophile which is protected by a preferably acid-stable linkage to a solid support or by virtue of being linked to a chain of subunits which are linked to a solid support. Methods of forming cyclic backbone subunits which are $N_2$ protected in this manner are described generally in Examples 12-15. The first subunit (the most recently added subunit in a growing polymer) is now reacted with an activated subunit which thereby becomes the next-in-sequence subunit in the polymer. This activated subunit, which is activated at its $N_2$ backbone site, and protected at its $N_1$ site,

preferably by an acid-labile protective group, is prepared by methods described above with reference to Figure 6.

As seen in Figure 5B, the coupling reaction adds the $N_2$-activated second subunit to the $N_1$-protected first subunit to couple the two through an $N_2$-E-$N_1$ bond, and form a compound which is protected at both free backbone nucleophile sites. This compound is now treated, for example by reaction with acid, to deprotect the acid-labile $N_1$ protective group on last-added subunit, and the procedure is repeated to build up a desired sequence polymer.

It can be appreciated from the above that the $N_2$-activated subunit which will form the last-in-sequence subunit must be protected at its $N_1$ backbone site, to allow selective activation at the $N_2$ site and to prevent self-polymerization of the activated compound. Also the $N_1$-deprotected subunit which is to couple to the activated subunit should be protected at its $N_2$ site to allow selective reaction of its $N_1$ moiety with the $N_2$-activated subunit. Therfore, since the reacting subunits are both protected at one backbone site, the method is suitable for coupling nucleosides (structure A in Figure 4) as well as subunits whose cyclic backbones contain both amine and hydroxyl backbone nucleophiles, such as structures B-D in this figure. The reaction methods used in coupling structure A subunits through a carbonate bond are detailed in Example 12, Briefly, a subunit containing a 5' protected backbone moiety is activated at its 3' hydroxyl, and reacted with another subunit (or growing chain) which is protected at its 3' hydroxyl. The coupling reaction is carried out in the presence of a catalyst, such as N-methylimidazole or N,N-dimethylaminopyridine, which is necessary for forming the carbonate bond. For coupling subunits containing structure B-D cyclic backbones, where intersubunit carbamate or thiocarbamate bonds are formed, much milder uncatalyzed coupling conditions, such as those described with reference to the first method above, are suitable.

The advantage of this second coupling method, for forming a polymer by solid-phase subunit addition, is that a substantial molar exess of the activated subunit can be added to the growing support-bound polymer at each polymer-addition step, to achieve subunit coupling to a very high percentage of the support-bound polymers. This insures that a large percentage of the support-bound polymers will contain the complete sequence of subunits desired. By contrast in the first method, where the growing polymer is activated at its last-added subunit, the efficiency of subunit addition is limited by the efficiency of the activations steps.

Figure 6 shows the dimeric structures produced by coupling the cyclic backbone subunits indicated at A-A through D-D in Figure 4. The nucleoside subunits in structure A in the figure are joined by a carbonate (Y = O). In the remaining three structures B-D, the subunits are joined by carbamate (Y = O) or thiocarbamate (Y = S) bonds. As seen from the figure, all of the subunit linkages are uncharged and achiral, i.e., do not contain chiral centers. In addition, the linkages are stable in aqueous medium, as judged by the ability of polymers to resist hydrolysis over an extended period in neutral aqueous solution.

According to another important feature of the invention, the structures, when joined to form polymers show acceptible Watson/Crick base pairing with complementary polynucleotides.

Finally, according to another important feature of the invention, polymers formed from the subunits are stereoregular. This is achieved in the structures shown by (a) using natural nucleosides or nucleoside derivatives or synthetic nucleosides having the natural stereoisomeric configuration as subunits, and (b) joining the subunits by achiral intersubunit linkages. Exemplary coupling methods are detailed in Examples 11-15.

B. Subunit Coupling: $N_1$----E Backbone Configurations

General methods for coupling subunits having an $N_1$-----E backbone configuration are illustrated in Figures 7A, 7B, and 7C. As will be recalled from Section I above, $N_1$ is a nucleophilic backbone group, such as a hydroxyl or amine group, and E is an electrophile, such as a carboxyl or sulfonyl group, which, when activated, can react with a second $N_1$-----E type backbone to form an $N_1$-----E-$N_1$ -----E backbone-linked dimer. The subunit backbones of this types which have been described specifically above are the cyclic backbone structures E-H in Figure 3, and the acyclic backbone structures I-N in Figure 4. In the cyclic structures E and F, the $N_1$ nucleophile is the 3' hydroxyl group, and in structures G and H, the amine attached to the 3' nitrogen on the morpholino ring. The E group in all of the cyclic structures is the carboxyl or corresponding sulfonyl group attached to the 5' position of the ribose ring, or to the 6' position of the morpholino ring. In all of the structures shown in Figure 4, the $N_1$ and E groups are respectively, the backbone amine or hydrazine and carboxylic or sulfonic acid groups on the backbone moiety ends

The first coupling method, which is illustrated in Figure 7A, is analogous to the method described above with reference to Figure 5A, where a first $N_1$-protected subunit is activated, then coupled directly to a backbone-unprotected subunit which forms the next-in-sequence subunit in the growing polymer. The $P_1$

protective group on the subunit $N_1$ is preferably an acid-labile protective group, such as t-butoxycarbonyl or a clevable linker bound to a solid support. Methods for $N_1$-protecting cyclic backbone structures are analogous to procedures described above for cyclic structures A-D. Similar methods are effective to protect the amine nitrogens in the acyclic bonebone structures. Alternatively, if the polymer is to be constructed on a solid phase support, as described below, the backbone $N_1$ site may be protected by its attachment to a solid support, with subunit addition to the growing polymer occuring via the activated E electrophile of the last-added subunit.

The activation reaction is designed to yield an activated or chain-terminal moiety of the form $N_1$-----E-X, where X is as described above with reference to Figure 6, and the activated subunit has much the same reactivity toward backbone nucleophilic groups as does the activated subunit in the previously described method. That is, the activation is designed to produce much the same active coupling group as in the $N_1$----$N_2$ type backbones, but where the reactive carbonyl or sulfonyl group is provided by the backbone itself rather than by a carbonyl or sulfonyl activating reagent, as in the methods shown in Figure 6.

The activation can be performed readily by reacting the $N_1$----E type subunit with a carbodimide in the presence of p-nitrophenol where the backbone electrophile is a carbonyl group, or by reacting with a carbodiimide in the presence of imidazole, 1,2,4 triazole or tetrazole, where the backbone is a sulfonyl. Subunit activation reactions involving carbonyl electrophilic groups are detailed in Examples 16 and 17. The general reaction conditions used to activate the N-amino-morphilino and linear chain backbone carbonyl groups are generally applicable to the other $N_1$----E type backbone structures shown in Figures 3 and 4.

Following the activation reaction, the activated complex is purified by conventional methods, such as silica chromatography, or, in the case of a support-bound activated chain, simply washed, and then reacted with a second subunit whose selected recognition moiety $R_2$ will form the next in-sequence recognition moiety in the completed polymer. The coupling reaction yields a dimer whose subunits are therefore coupled through an E-$N_1$ bond, as indicated in the figure. As above, both subunits must be suitably base protected during the activation and coupling reactions.

The polymer can be extended by repeating the above steps of (a) activating the free E electrophile in the second subunit, (b) separating the activated species from the activating agent, and (c) coupling the activated compound with the next-in-sequence subunit, whose backbone is unprotected.

The second coupling method, which is illustrated in Figure 7B, is directly analogous to the method described above with respect to Figure 58. Here a First subunit having an E-protected backbone is reacted with a second subunit with an activated E group and a protected $N_1$ nucleophile, to form a dimer linked through an E-$N_1$ bond and protected at both free backbone end groups. Where the polymer is being formed by solid-phase synthesis the $P_2$ protective "group" takes the form of an acid-stable linkeage to a solid support. The $N_1$-protected subunit is prepared and activated as above. Coupling conditions generally follow those used in the above cyclic subunit coupling reactions.

In the third coupling method, shown at Figure 7C, the $n_1$-protected and E-unprotected subunits (or polymer units) are reacted together in the presence of a suitable E-activating agent, such as a carbodiimide, as indicated

Figure 8 shows the dimeric structures produced by coupling the cyclic amd acyclic backbone subunits indicated at E-K in Figures 3 and 4, and indicated at E-E through K-K, repsectively. The nucleosides subunits in structure F-F in Figure 3 are joined by an ester linkage. In remaining cyclic backbone structure, G-G, the subunits are joined through a hydrazid or sulfonyl hydrazide linkage. All of the acyclic backbone subunits, shown at I-I, J-J and K-K, are linked through amide (E = carbonyl) or sulfonamide (E = sulfonyl) bonds. As in the above-discussed cyclic backbone subunit structures, all of the subunit linkages are uncharged and achiral, and all of the bonds are reasonably stable in aqueous medium, as judged by the known stability of ester, hydrazide and amide linkages in solution.

According to another important feature of the invention, the structures, when joined to form polymers show Watson/Crick base pairing with complementary polynucleotides.

Finally, as with the $N_1$-----$N_2$ backbone subunits described above, all of the $N_1$----E backbone structures shown are stereoregular. This is due to (a) the homochiral (structures E-H in Figure 3 and I and L in Figure 4) or achiral (structures J, K, M, and N in Figure 4) nature of the recognition moiety attachment to the backbone moiety, and (b) the achiral subunit linkage.

C. <u>Subunit Coupling: Alternating Charged and Uncharged Achiral Linkages</u>

In some applications, it may be desirable to introduce one or more charged achiral subunit linkages in the polymer molecules. The backbone charge may be used to enhance polymer solubility in many solution applications or to adjust polymer-target binding constants, as will be described further below. Polymers

having regularly spaced charged achiral backbone linkages, e.g., between every second or third subunit, are suitable for the novel diagnostic system which is described below. For purposes of the present discussion, it is assumed that polymeric units composed of two or more subunits which are internally joined by achiral uncharged linkages are to be linked by a charged achiral linkage. These uncharged units are formed by one of the methods described in Sections A and B above.

The dimer subunits which are to be linked are selected such that that the free $N_2$ group of one dimer can be coupled to the free $N_1$ group of the other dimer through a suitable charged achiral linkage. The preferred linkage is a phosphodiester linkage which requires that the free $N_2$ group on one subunit and the free $N_1$ group on the other subunit both be hydroxyls. As can be seen in Figure 3, this condition is met if dimers formed from either structure A or structure B subunits, which both have a free $N_2$ hydroxyl, are linked to dimers formed from structure A, C, or D subunits which all have a free $N_1$ hydroxyl.

In a typical coupling method, dimer A, which is protected at its $N_1$ position, as indicated, is carried through a series of reactions which lead to an reactive phosphoester coupling species linked to the $N_2$ hydroxyl through a phosphoester bond, as indicated in Figure 9. The preferred coupling method, which is detailed in U.S. patent application for "Polynucleotide Assay Reagent and Method", Serial Number 712, 396, filed March 15, 1985, and which follows literature methods for forming phosphodiester bonds between base-protected nucleosides or nucleoside analogue subunits, involves formation of a reactive $N_1$ protected $N_2$-(p-chlorophenyl2-cyanoethyl)-phosphate. The activated dimer is then reacted with the second dimer to form a tetramer which is linked alternately by uncharged and charged achiral intersubunit linkages.

The phosphodiester linkage method may be incorporated, either is solution or support-type polymer synthesis method, according to the general procedures outlined above for polymer coupling involving achiral, but uncharged linkage reactions.

Although for diagnostic purposes, polymers with alternating charged and uncharged achiral bonds may be suitable, it is generally preferred in diagnostic and particularly therapeutic uses, to introduce as few charged linkages as are necessary for achieving adequate polymer solubility in an aqueous medium. The solubility enhancement is generally achieved with 1-3 charged backbone linkages per polymer of about 20 subunits, and preferably no more than about one charged linkage per four subunits. As defined herein, achiral backbone linkages are considered substantially uncharged if they contain less than about one charge backbone linkage per dimeric unit, and preferably one or fewer backbone linkage per tetrameric unit.

## IV. Polymer Targeting Considerations

### A. Diagnostic Applications

An important application of the uncharged or substantially uncharged polymers of the invention is in a novel solid-support diagnostic system for use in determining the presence and/or amount of a polynucleotide analyte. The assay system includes a solid support reagent composed of a multiple polynucleotide-binding polymers which are constructed according to the invention linked to a solid support. The polymers are constructed, according to targeting strategies detailed below, for binding specifically to target polynucleotide analyte at a selected melting temperature ($T_m$). The sytstem also includes polycationic reporter molecules which are designed to bind to the fully charged analyte backbone, but not the uncharged or only partially charged polymer backbone, under selected binding conditions. Each reporter molecules carries one or more reporter groups, and each polynucleotide can accomodate binding of typically several thousand or moe reporter molecules. Thus the system has an ampliication factor, in terms of reporter signal per bound analyte molecule, of several orders or magnitude. The assay system and its method of practice are more fully described in co-owned U.S. Patent application for "Polynucleotide Assay Reagent and Method", Serial No. 712,396, filed March 15, 1985.

The particular merits of using sequence-specific polynucleotide binding polymers having achiral predominatly uncharges intersubunit linkages for diagnostic applications derives from two factors. First, their target binding is relatively insensitive to salt and so the polymer/target annealing step can be carried out under low salt conditions wherein there is no competitive annealing between the target and complementary nucleic acid sequences in the solution. Second, the polymer's reduced charge or uncharged backbone allows use of the above-described reporter molecules which are designed at high density to the target analyte's polanionic backbone.

The design considerations applied in preparing a polynucleotide binding polymer for use as a diagnostic reagent are governed by the nature of the target analyte and the reaction conditions under which the analyte is to be assayed. As a first consideration, there is selected a non-homopolymeric target base sequence against which the polymer is directed. This target sequence is preferably unique to the analyte

being assayed, i.e., is calculated to occur only with some defined small probability (such as 1% or less) in an assay mixture containing a given number of unique-sequence bases. The probability of occurrence of a given n-base target sequence is approximately $1/4^n$--that is, a given n-base target sequence would be expected to occur approximately once in a polymer containing $4^n$ bases. Therefore, the probability P that a given n-base sequence will occur in polynucleotides containing a total of N unique-sequence bases is approximately $P = N/4^n$. To illustrate, the probability P that d 9-base target sequence will be found in a 20 kilobase polynucleotide is about $20x10^3/2x10^5$ or 0.08, the probability that a 16-base target sequence will be present is about $20x10^3/4.\ 3x10^9$ or 0.0000047. From these calculations, it can be seen that a polymer having 9-16 recognition moieties specific for a defined 9-16 base target sequence should have high specificity for the target sequence in an assay mixture containing only viral genomes, whose greatest complexities correspond to about 400K of unique-sequence bases.

Similar types of calculations show that a 12 to 16 subunit polymer can provide adequate specificity for a viral or bacterial target sequence in an assay mixture containing viral and bacterial genomic material only (largest genomic sizes about 5,000 kilobases), and that a 16 to 22 subunit polymer can provide adequate specificity for a target sequence in a polynucleotide mixture containing mammalian genomic DNA material (genomic sizes of about 5 billion base pairs of unique-sequence DNA).

The polymer/analyte binding affinity, and particularly the temperature at which the polymer just binds with the target sequence (the melting temperature, or Tm) can be selectively varied according to (a) polymer length, (b) the number of hydrogen bonds that can be formed between the recognition moieties and the corresponding, in-sequence bases of the analyte target sequence, (c) backbone charge density, and (d) a concentration of denaturants, such as formamide, which reduces the temperature of melting. From a number of studies on model homopolymer duplexes it is known that the melting temperature of oligonucleotide duplexes in the 10 to 20 bp range increases roughly 3°c per additional base pair where the complementary bases are capable of forming two hydrogen bonds, and about 6°C per additional base pair where the complementary bases are capable of forming three hydrogen bonds. Therefore, the length of a target sequence, which is initially selected to insure high binding specificity with the polymer may be extended to achieve a desired melting temperature with the complementary-base polymer, under selected assay conditions. Also, where the recognition moieties used in constructing the polymer are the standard nucleic acid bases, as illustrated above, the target sequence may be selected to have a high percentage of guanine plus cytosine bases for achieving a relatively high polymer/analyte melting temperature, or a relatively high percentage of adenine plus thymine bases, for achieving a relatively low melting temperature.

The backbone charge density of the polymer must be substantially less than that of the polynucleotide analyte, to allow preferential binding of polycationic reporter molecules to the analyte, under conditions where reporter binding to the polymer does not occur. This requirement is met where the spacing between the adjacent negative charges in the polymer backbone is at least twice as great as the spacing between adjacent charged phosphodiester linkages in the analyte. The charge density of the backbone also has an important effect on the polymer/analyte melting temperature, particularly under low salt conditions, where any charge-charge repulsion between the analyte and polymer backbones can act to lower the temperature at which the two can anneal. In general, therefore, a polymer having a less-charged backbone would be expected to show (1) a higher analyte/polymer melting temperature, and (2) less dependence of the melting temperature on salt concentration. Based on these considerations, an uncharged polymer, such as an all-carbamate-linked or all amide-linked polymer, will allow the analyte/polymer annealing reaction in the diagnostic method to be carried out under a wider range of salt-concentrations than a partially charged polymer, wherein some of the linkages constitute achiral but charged phosphodiester linkages.

B. Therapeutic Applications

Requirement for Adequate Specificity

1. Information Requirements

An ideal therapeutic is one which efficiently inactivates a targeted structure unique to the pathogenic state, but which does not detrimentally affect normal structures in the patient. Thus, sequence-specific gene inactivating agents should only effect inactivation of a targeted genetic sequence which contains an amount of sequence information adequate to assure a high probability of that target sequence being unique to the pathogenic state. For example, an agent which can effect target inactivation by binding to only three or four contiguous bases of its target sequence would invariably also attack many mRNAs required for normal cellular functions. In contrast, an agent which requires binding to 30 or more bases in order to effect target

inactivation would have an exceedingly small probability of also attacking non-target messengers involved in normal cellular functions.

A reasonable estimation of the desired amount of sequence information which a gene-inactivating agent should recognize when that agent is targeted against a disease-specific mRNA can be calculated as follows. The human genome contains roughly 5 billion base-pairs of unique-sequence DNA. However, essentially all of this material exists in the duplex state and so is largely unavailable for binding to single-strand-directed polynucleotide-binding agent. Thus, polymer binding is largely restricted to RNA transcripts of the genomic DNA. Of that genomic DNA, something on the order of 1% is transcribed in any given cell type. Further, when all cell types in the patient are considered, it is unlikely that a composite of more than 4% of the entire genome is transcribed in the adult human (i.e., post-embryogenesis). Thus, the sequence complexity of all rna sequences inthe adult human is likely to constitute no more than about 200 million bases -- and probably far fewer.

For a gene-inactivating agent to have an expectation of recognizing no target sequences in a cellular pool of 200 million bases of unique sequence genetic material, it should recognize at least n bases in its target, where n is calculated as $2 \times 10^8 = 4^n$ - giving a minimal target recognition requirement of 14 bases. This suggests that a gene-inactivating agent recognizing in excessof 14 bases in its target will likely have no targets in cellular pools of normal RNA. Obviously, as the number ob bases recognized in the target sequence increases over this value, the probability that the agent will recognize no inherent cellular RNA sequences also increases. It is noteworthy that as the number of bases recognized in the target increases linearly, this "safety factor" increases exponentially. For example, below are tabulated the number of bases recognized in a target sequence and the corresponding expected number of targets in a pool of 200 million bases of unique-sequence single-stranded genetic material:

| No. of bases recognized | Expected no. of targets |
|---|---|
| 12 | 12.0 |
| 14 | .75 |
| 16 | .047 |
| 18 | .0029 |
| 20 | .00018 |

From the above one can conclude that for a sequence-specific gene-inactivating agent destined for therapeutic applications, safety considerations probably mandate that the agent recognize at least 14, and preferably 16 to 20, bases in its target sequence.

2. The Concept of a Minimum Inactivation Length (MIL)

In regard to the amount of sequence information actually "recognized" by a sequence-specific gene-inactivating agent, a sueful concept in this regard is that of a minimum inactivating length (MIL), which we define as the minimum number of contiguous bases of a target sequence to which an agent must bind in order to effect gene inactivation in vivo. Irrespective of whether or not the agnet binds a longer sequence of bases, it is this MIL value which determines the number of bases in the targer "recognized" by that agent -- which, in turn, determines the agent's level of sequence specificity.

To illustrate this concept, suppose a gene-inactivating agent has a 16-subunit length but an MIL value of only 10. Such an agent would be expected to inactivate any genetic sequences containing any of the component 10-contiguous-base target sequences. In a cellular pool comprising 200 million bases of unique-sequence single-stranded genetic material, there are an estimated 1000 such target sequences. Further, because the number of base-pairing moieties in the agent is substantially greater than the MIL value, the agent would also be expected to forma variety of partially mispaired complexes with sequences lacking even the required 10-contiguous-base target sequences -- with some of those mispaired complexes having sufficient stability to effect inactivation of the participating nontarget genetic sequences.

In contrast, in the case of an agent 16 subunits in length and having an MIL value of 16, by definition, such an agent cannot inactivate nontarget sequences because any partially mispaired complexes with nontarget sequences would not have sufficient stability to effect gene inactivation -- only perfect 16-base-pair agent/target duplexes would have adequate stability to effect gene inactivation.

In regard to determining actual MIL values, when a series of gene-inactivation agents, all targeted against the same genetic sequence but varying in number of subunits, are tested in living cells known to

contain a functioning single-stranded target sequence, the activity of the targeted gene will show a dramatic reduction at the subunit length corresponding to the MIL value. That is, the MIL value corresponds to that number of subunits just sufficient to block the activity of the targeted genetic sequence.

To illustrate further, below is described an exemplary method for determining the minimum inactivating length for a polynucleotide-binding polymer having any selected backbone linkage type.

First, prepare a regular series of binding polymers having the selected backbone type, targeted against the region 57 to 80 of the rabbit β-globin in mRNA (5'-GUGCAUCUGUCCAGTGAGGAGAAG-3'), and ranging in length from 5 to 24 subunits. Next, following the detailed procedures of Black, Murakimi, and Miller, Biochemistry, 24:6132 (1985), add each of these polymers to a separate portion of a commercial rabbit reticulocyte lysate preparation labeled with [$^{35}$S]-methionine. Thereafter, add rabbit globin mRNA and incubate 1 hour at 37°C. Work each sample up, fractionate by gel electrophoresis, and guantitate the radioactivity incorporated in β-globin as per Blake et al., above. The minimum inactivating length is that minimum polymer subunit length which effects essentially complete blockage of new β-globin synthesis.

It should be reemphasized that this MIL will vary at small amount depending on the targeted sequence, but in general this relatively simple extracellular method serves to give a quite good approximation of the intracellular MIL for any given backbone type. Once this MIL value has been determined for a selected backbone type, the methods described below for MIL adjustment can be rationally applied for optimizing the MIL for a selected target sequence.

### 3. Tactics for Adjusting the MIL

As a rule, it is often necessary to vary an agent's target-binding affinity over a substantial range in order to ultimately achieve an acceptable MIL value. To this end, a variety of methods, and combinations thereof, can be used for varying the stability of the agent/target duplex, including:

(i) increasing or decreasing the number of base-pairing moieties in the agent;

(ii) selecting a target having a sequence providing greater or lesser stacking contributions (see Tnoco et al (31) for a detailed analysis of the effects of base sequence on duplex stability);

(iii) selecting a target sequence containing a greater or lesser proportion of strong-binding bases (G and C) to weak-binding bases (A and U or T);

(iv) replacing one or more weak-binding base-pairing moieties with stronger-binding moieties having the same Watson/Crick pairing specificities (e.g., a 2,6-diaminopurine moiety can be used instead of adenine to effect a substantially stronger bond to U or T in the target sequence; a 5-bromouracil moiety can be used instead of U or T to effect a substantially stronger bond to a in the target sequence; and a 5-bromocytosine moiety can be used instead of cytosine to effect a somewhat stronger bond to G in the target sequence); or replacing one or more strong-binding base-pairing moieties of the agent with a weaker-binding moiety having the same W/C pairing specificities (e.g., a 2-pyrimidinone moiety can be used instead of cytosine to effect a substantially weaker bond to G in the target sequence; and a 6-thioguanine or hypoxanthine moiety can be used instead of guanine to effect a substantially weaker bond to C in the target sequence); and

(v) selecting a backbone structure so as to somewhat inhibit or enhance Watson/Crick pairing to the target sequence.

It should be noted that the first three of the above methods actually comprise target selection procedures, while the latter two methods are effected during synthesis of the agents.

A difficulty with method (i) is that sequence specificity requirement generally preclude reduction of the agent's length to less than about 14 subunits. The difficulty with methods (ii) and (iii) is that other more critical targeting criteria can so severely limit the optimal target region that these two NIL adjustment methods have little latitude in which to achieve a significant effect. Because of these limitations, most MIL adjustments are preferably achieved by methods (iv) and (v).

## V. Polymer Assembly Strategies

### A. Geometric assembly

After selecting a desired polymer length and recognition moiety sequence, according to factors considered in Section IV, the polymer is assembled, using the general subunit coupling procedures detailed above. One method of polymer assembly involves initial preparation of an appropriate set of dimers, linking selected dimers to form tetramers, linking of these to form octamers, and so on. This method is carried out in solution, substantially according to methods described with reference to Figures 5A and 7A above. It

should be noted that all couplings need not necessarily be between oligomers of equal size. For example, often it is desirable in the last coupling to link a hexadecamer with a tetramer to give a 20-mer or to link a hexadecamer with an octomer to give a 24-mer.

A particular merit of this assembly method is that each coupling product is roughly twice the mass of the precursors and so purification of the product of each coupling is a simplified matter. Example 18 below details the the assembly of an 8-subunit carbamate-linked polymer formed by this method. (b)

B. Stepwise Assembly on a Solid Support

One preferred method for polymer synthesis is stepwise assembly on a solid support. Here the first subunit in the polymer is attached through its $N_2$ backbone group to a solid support. Typically, a solid support, such as glass beads derivatized with cleavable, preferably acid-stable, long-chain linkers are employed as the support material, and prepared for attachment of $N_2$ nucleophile, such as a 5' hydroxyl of a nucleoside, as descibed in Example 19. The glass beads are reacted with a subunit which has a preferably acid-labile $N_1$ protected group, and an activated $N_2$ or E backbone group, as detailed in Section III above. The coupling of various types of subunits to a glass-bead support is described generally in Examples 20-22.

After coupling the second subunit (or polymer unit which may be assembled in solution) to the support, any unreacted linker nucleophiles are capped by addition of a suitable capping reagent, such as p-nitrophenyl acetate, and thereafter the support is washed and filtered. The protecting group on the $N_1$ terminal subunit is removed, typically by acid treatment, and after neutralization, the support is then reacted with an excess of the next-in-sequence subunit (or polymer unit) which is activated at its free $N_2$ backbone moiety. The excess of activated subunit maximizes the number of support-bound subunits which are chain-elongated. That is, one feature of the solid support assembly method is the need for high coupling efficiencies at each subunit addition step, and the concentration of added activated subunit is selected to maximize this efficiency. Chain elongation is continued in this manner, with capping of failure sequence, after each subunit addition, until the polymer of the desired length and sequence is achieved. The general method is illustrated in Example 20 for the preparation of a 14-subunit carbonate-linked polymer, in Example 21, for the synthesis of a 19-subunit carbamate-linked polymer, and in Example 22, for the assembly of a 19-subunit amide-linked polymer.

After addition of the last-in-sequence subunit, the polymer may be capped with a suitable charged or uncharged group coupled to the terminal $N_1$ group. If the backbone assembly has been carried out exclusively with uncharged achiral linkages, the capping reagent is preferably a charged group which imparts an end terminal charge to the polymer. After capping the polymer, the polymer is cleaved from the support, e.g. , by treatment with a nucleophile, such as ammonium hydroxide, and the polymer is purified, such as by anion exchange chromatography. These post-assembly operations are described in Example 23, for synthesis of a carbonate-linked polymer, in Example 25, for synthesis of a carbamate -linked polymer, and in Example 27, for synthesis of an amide-linked polymer.

For diagnostic applications involving the above solid-support reagent with bound polymer molecules, the assembled, purified polymers are attached to a solid support, such as a cellulose support by known coupling methods, such as those detailed in Example 20. Examples 24, 26, and 28 are illustrative.

The following examples illustrate a variety of subunit synthetic schemes, polymer construction methods, and applications and compositions made and used according to the invention. The examples are in no way intended to limit the scope of the invention.

Example 1

Preparation of ribonucleosides and deoxyribonucleosides

The following nucleosides are obtained from Sigma Chemical Co., St. Louis, MO: deoxyuridine, deoxyguanosine, thymidine, deoxyadenosine, deoxycytidine, 5-bromodeoxyuridine, deoxyinosine, 2,6-diamino-9-(2-deoxy-β-D-erythro-pentofuranosyl)9H-purin e (2,6-diaminopurine deoxyriboside), uridine, guanosine, 5-methyluridine, adenosine, cytidine, 5-bromouridine, inosine.

2,6-Diamino-9-(β-D-ribofuranosyl)-9H-purine (2,6-diaminopurine riboside) is obtained from Pfaltz and Bauer, Inc., Division of Aceto Chemical Co., Inc., Waterbury, CT.

The following nucleosides are prepared by the literature methods indicated:

1-(2-Deoxy-β-D-erythro-pentofuranosyl)-2-pyrimidinone (2-hydroxypyrimidine deoxyriboside) is prepared by the method of P. Kohler, E. Volz, U. Sequin, and C. Tamm in Nucleic Acid Chemistry (L.B. Townsend

and R.S. Tipson, eds) John Wiley and Sons, Inc. (1976).

1-(2-Deoxy-β-D-erythro-pentofuranosyl)-4-methoxy-2-pyrimidinone is prepared by the following procedure:

1-(3'5'-Di-O-benzoyl-2-deoxy-β-D-erythro-pentofu ranosyl)-4-methylthio-2-pyrimidinone (prepared as in D. Cech and A. Holy, Collection of Czechoslov Chem Comm (1977) 42:2246) is treated with 200 mL of 0.2M sodium methoxide solution. The resulting solution is allowed to stand overnight at room temperature. This solution is then neutralized with Dowex 50X8 (H+ form) and filtered; the residue is dissolved in water (100 mL) extracted with ether (2.50 mL) and the aqueous phase evaporated. The residue is an amorphous material which isused directly in succeeding reactions.

2-Amino-9-(2-deoxy-β-D-erythro-pentofuranosyl)-1, 9-dihydro-6H-purine-6-thione (deoxythioguanosine) is prepared by the procedure of R.H. Iwamoto, E.M. Acton, and L. Goodman, J Med Chem (1963) 6:684.

1-(β-D-Ribofuranosyl)-2-pyrimidinone (2-hydroxypyrimidine riboside) is prepared by the procedure of U. Niedballa and H. Vorbruggen, J Org Chem (1974) 39:3668.

1-(2-Deoxy-β-D-ribofuranosyl)-4-methoxy-2-pyrimid inone (2-hydroxypyrimidine deoxyriboside) is prepared by the procedure of R. Wightman and D. Holy, Collection of Czechoslov Chem Comm (1973) 38:1381.

2-Amino-9-(β-D-ribofuranosyl)-1,6-dihydro-6H-purine-6-thione (thioguanosine) is prepared by the procedure of J.J. Fox, I. Wempen, A. Hampton and I.L. Doerr, J Amer Chem Soc (1958) 80:1669.

## Example 2

### Preparation of Base-Protected Nucleotides

Dimethoxytrityl chloride, N-benzoyladenosine, N-benzoyl-2'-deoxyadenosine, N-benzoylcytidine N-benzoyl-2'-deoxycytidine and N-isobutyryl-2'-deoxyguanosine are obtained from Sigma Chemicals, St. Louis, Missouri.

9-Fluorenylmethoxycarbonyl chloride (FMOC chloride), trimethylchlorosilane, isobutyric anhydride, 4-nitrobenzoyl chloride and all organic solvents for reactions and chromatography are obtained from Aldrich Chemical Co., Milwaukee, Wisconsin. Silica Gel is obtained from EM Science, Cherry Hill, New Jersey.

### 2.1 Guanosine

The N-2 9-fluorenylmethoxycarbonyl derivative is prepared by the procedure below which is general for the protection of nucleoside amino groups:

Guanosine (1 mmol) is suspended in pyridine (5 mL) and treated with trimethylchlorosilane (5 mmol). After the solution is stirred for 15 minutes 9-fluorenylmethoxycarbonyl chloride (5 mmol) is added and the solution maintained at room temperature for 3 hours. The reaction is cooled in an ice bath and water (1 mL) is added. After stirring for 5 minutes conc. ammonia (1 mL) is added and the reaction stirred for 15 minutes. The solution is evaporated to near dryness and the residue dissolved in chloroform (10 mL). This solution is washed with sodium bicarbonate solution (5 mL, 10%), dried over sodium sulfate and evaporated. The residue is coevaporated several times with toluene and the product chromatographed on silica gel using a gradient of methanol in methylene chloride (0-50%).

N-Isobutyrylguanosine is prepared by the method of Letsinger and Miller, J Amer Chem Soc (1969) 91:3356.

N-2 Acetylguanosine is obtained by the method of C.B. Reese and R.S. Saffhill, J Chem Soc Perkin Trans (1972) 1:2937.

### 2.2 Deoxyguanosine

The N-2 9-fluorenylmethoxycarbonyl derivative is prepared by the method of J. Heikkla and J. Chattopadhyaya, Acta Chem Scand (1983) B37:263.

The N-2 acetyl derivative is obtained from Research Plus Inc., Bayonne, N.J.

### 2.3 Deoxyadenosine

The N-6 2-(4-nitrophenyl)-ethoxycarbonyl derivative is prepared by the method of F. Himmelsbach and W. Pfleiderer, Tetrahedron Lett (1983) 24:3583.

N-6 4-Nitrobenzoyl-2'-deoxyadenosine is prepared using the procedure above for FMOC-guanosine

except that 4-nitrobenzoyl chloride is substituted for FMOC chloride.

The N-6 2-(phenylsulfonyl)-ethoxycarbonyl derivative is prepared by the procedure for FMOC guanosine except the 2-(phenylsulfonyl)-ethyl chloroformate (obtained from N. Balgobin, S. Josephson and B. Chattopadhyaya, Tetrahedran Lett (1981) 22: 3667) is used as the acylating agent and N-methylimidazole or pyridine is used as the solvent.

2.4 Adenosine

The N-6 2-(4-nitrophenyl)-ethoxycarbonyl derivative is prepared by the method of F. Himmelsbach and W. Pfleiderer, Tetrahedron Lett (1983) 24:3583.

N-6 4-Nitrobenzoyladenosine is prepared using the procedure above for FMOC-guanosine except that 4-nitrobenzoyl chloride is substituted for FMOC chloride.

The N-6 2-(phenylsulfonyl)-ethoxycarbonyl derivative is prepared by the procedure for FMOC guanosine except the 2-(phenylsulfonyl)-ethyl chloroformate (obtained from N. Balgobin, S. Josephson and B. Chattopadhyaya Tetrahedron Lett (1981) 22:3667) is used as the acylating agent and N-methylimidazole or pyridine is used as the solvent.

2.5 Deoxycytidine

The N-4 2-(4-nitrophenyl)-ethoxycarbonyl derivative is prepared by the method of F. Himmelsbach and W. Pfleiderer, Tetrahedron Lett (1983) 24:3583.

The N-6 2-(phenylsulfonyl)-ethoxycarbonyl derivative is prepared by the procedure for FMOC guanosine except the 2-(phenylsulfonyl)-ethyl chloroformate (obtained from N. Balgobin, S. Josephson and B. Chattopadhyaya Tetrahedron Lett (1981) 22:3667) is used as the acylating agent and N-methylimidazole or pyridine is used as the solvent.

2.6 Cytidine

The N-4 2-(4-nitrophenyl)-ethoxycarbonyl derivative is prepared by the method of F. Himmelsbach and W. Pfleiderer, Tetrahedron Lett (1983) 24:3583.

The N-6 2-(phenylsulfonyl)-ethoxycarbonyl derivative is prepared by the procedure for FMOC guanosine except the 2-(phenylsulfonyl)-ethyl chloroformate (obtained from N. Balgobin, S. Josephson and B. Chattopadhyaya Tetrahedron Lett (1981) 22:3667) is used as the acylating agent and N-methylimidazole or pyridine is used as the solvent.

2.7 2,6-diaminopurine riboside

The N-2,N-6-bis(9-fluorenylmethoxycarbonyl) derivative of 2,6-diaminopurine riboside is prepared by the general procedure.

The N-2,N-6-bis(isobutyryl) derivative is prepared by the general procedure.

2.8 2,6-diaminopurine-2'-deoxyriboside

The bis N-2,N-6-(9-fluorenylmethoxycarbonyl) derivative of 2,6-diaminopurine-2'-deoxyriboside is prepared by the general procedure.

2.9 Thioguanosine

The N-2 9-fluorenylmethoxycarbonyl derivative of thioguanosine is prepared by the general procedure.

2.10 2'-Deoxythioguanosine

The N-2 9-fluorenylmethoxycarbonyl derivative of 2'-deoxythioguanosine is prepared by the general procedure.

Example 3

Preparation of 5'-amino-2',5-dideoxyribonucleoside subunits.

Carbon tetrabromide, sodium azide, p-toluenesulfonyl chloride (tosyl chloride), triphenyl phosphine and 10% palladium on carbon are purchased from Aldrich Chem Co. Lithium azide is obtained from Kodak Laboratory and Specialty Chemicals.

3.1 General Method

The nucleosides employed in this example are thymidine, N6-benzoyl-2'-deoxyadenosine, N4-benzoyl-2'-deoxycytodine and 2'-deoxyinosine, 2-hydroxy-pyrimidine-2'-deoxyriboside and the N2-N6-bisisobutyryl derivative of 2,6-diamino purine-2'-deoxyriboside (see Example 1). The required dried nucleoside (1 mmol) is weighed into a reaction vessel containing triphenyl phosphine (1.01 mmol) and lithium azide (5 mmol). After the solids are suspended in DMF, carbon tetrabromide (1.0 mmol) is added to the vessel. The solution is stirred at room temperature for 24 h. After quenching the reaction with methanol, the solution is evaporated to dryness. The residue is chromatographed on silica gel eluting with methanol/chloroform mixtures to afford the desired 5'-azido-2', 5'-deoxynucleoside.

The 5'-azido nucleoside (1 mmol) is dissolved in ethanol. Hydrogenation in the presence of 10% palladium on carbon (catalytic amount) under a hydrogen atmosphere (35 psi) occurs over 10 h. The solution is filtered and evaporation under reduced pressure affords a crude solid. The solid is purified by trituration with the appropriate solvent.

3.2 5'-Azidouridine derivativ

The 5'-azido derivative of 5-bromo-2'-deoxy uridine is prepared via the method described above. The 5'-azido-t-bromo-2'-deoxyuridine (1 mmol) is treated with triphenyl phosphine (1.5 mmol) in pyridine at room temperature for 2 h. Concentrated ammonia (1 mL) is added to the reaction vessel. After 14 h the solvent is removed under vacuum. The residue is dissolved in THF and this solution is added to hexanes. The precipitate is collected and the solid triturated with the appropriate solvent to afford the 5'-amino-5-bromo-2'-deoxyuridine.

3.5 5'-Azido guanosine

An alternate preparation of 5'-amino-2',5'-dideoxyguanosine is to treat the protected 2'-deoxyguanosine (protected at either the N-2-acetyl or the N-2-FMOC derivative) (1 mmol) with tosyl chloride (1.3 mmol) in pyridine at 0 °C overnight. The solution is evaporated to dryness and the residue is dissolved in chloroform. The resulting solution is washed twice with aqueous sodium bicarbonate and dried over sodium sulfate. The solvent is evaporated and the residue chromatographed on silica gel eluting with methanol/chloroform mixtures. The resulting tosylate (1 mmol) is treated with sodium azide (6 mmol) in DMF at 80-100 °C for a few h. The solvent is removed by rotovap and the residue is chromatographed on silica gel eluting with chloroform/methanol solvent mixtures. The azide is reduced to the desired amine using the above procedure.

Alternate protecting groups for the base nitrogens of 2'-deoxycytidine, 2'-deoxyadenosine, 2'-deoxyguanosine, and 2,6-diaminopurinedeoxyriboside are 2-(phenylsulfonyl)ethoxycarbonyl for 2'-deoxycytidine and 2'-deoxyadenosine and the use of the 9'-fluorenylmethoxycarbonyl (FMOC) to protect 2'-deoxyguanosine and 2,6-dianinopurine deoxyriboside, as in Example 2.

Example 4

Preparation of 3'-amino-2',3'-dideoxyribonucleoside subunits.

Thymidine is transformed to 5'-O-acetyl-3'-azido-2',3'-dideoxythymidine and this is converted to 3'-azido-2',3'-dideoxyadenosine and 3'-azido-2',3'-dideoxyguanosine via the methods of M. Imazawa and F. Eckstein, J Org Chem (1978) 43:3044. The base moiety of the 3'-azidoadenosine is protected as either the benzoyl or the 2-(phenylsulfonyl)ethoxycarbonyl derivative (as shown in Example 1). N-2 of the 3'-azidoguanosine is protected as either the acetyl or FMOC derivative (see Example 1). The reduction of these 3'-azido functions to the 3'-amino-2',3'-dideoxyribonucleosideS is performed as shown in Example 3.

Example 5

Preparation of morpholino-type subunits derived from ribonucleosides.

Ammonium biborate, sodium m-periodate, sodium cyanoborohydride, uridine, N4-benzoyl cytidine, and N6-benzoyl adenosine are obtained from Sigma Chemical Co. N2-isobutyryl guanosine is prepared by the general method of Letsinger and Miller (J Amer Chem Soc (1969) 91:3356). 2-Phenyl-2-propanol and bis(p-nitrophenyl) carbonate are obtained from Aldrich Chemical Co.

The morpholino derivative of uridine is prepared by dissolving 1 mmol of uridine plus 2 mmol of ammonium biborate, $(NH_4)_2B_2O_7$, in 5 ml of water. While stirring in an ice bath protected from direct light 1.2 mmol of sodium m-periodate in 5 ml of water is added. After 90 min, 0.2 ml of 1,2-propanediol is added and stirring is continued for 10 min at rt. Thereafter, while stirring in a well-ventilated hood, 0.25 g of sodium cyanoborohydride dissolved in 3 ml of water is added and the mixture stirred For 4 hr at rt. Thereafter, the reaction mixture is reduced to an oil under vacuum in a warm water bath, resuspended in a minimal volume of methanol, layered on a Silica gel chromatography column, and the column eluted with methanol/1% triethylamine. The morpholino product elutes from the column as a sharp band moving significantly slower than the original ribonucleoside and its oxidation products. The morpholino product has a UV spectrum essentially identical to the parent ribonucleoside, but its mass is lower by 17 daltons (determined by mass spectral analysis using fast atom bombardment activation)

The morpholino nitrogen is next protected by reacting the product with 2-phenylisopropyl phenyl carbonate. The required active carbonate is prepared and reacted with the morpholino subunit essentially by the methods of Sandberg and Ragmarsson (Int J Peptide Protein Res (1974) 6:111). Finally, if desired (depending on the method to be used for subunit assembly into polymers) the hydroxyl at the position corresponding to the original 5' can be activated by dissolving the N-protected subunit in a minimal volume of dry dimethylformamide and adding 2 equivalents of bis(p-nitrophenyl)carbonate plus 0.2 equivalent of triethylamine N-methylimidazole, or N,N-dimethylamino pyridine. After 3 h at rt, the reactions mixture is reduced in volume under vacuum in a warm water bath. The thick syrup is resuspended in a small volume of dichloromethane and layered on a silica gel column, which is subsequently eluted with a mixture of dichloromethane/ether (1:1 by vol) 0.2% in N,N-dimethylaniline by volume. With this solvent system the activated product moves substantially slower than p-nitrophenol and bis(p-nitrophenyl)carbonate but much faster than the unreacted starting material. The activated product is readily visualized on silica gel TLC plates simply by exposing to ammonia fumes, whereupon a yellow nitrophenolate ion is generated within one to two minutes.

Other ribonucleosides (bases protected where necessary as in Example 2) are converted to their corresponding morpholino derivatives by essentially the same methods -- though varying amounts of methanol must be added to effect dissolution of the protected ribonucleosides before the initial periodate oxidation step.

When the morpholino-type subunits are to be coupled via thiocarbamate linkages, the preferred base-protective groups for the common starting ribonucleosides are: the phenylsulfonylethoxycarbonyl moiety for cytidine and adenosine and the 9-fluorenyl methoxycarbonyl moiety for guanosine.

## Example 6

### Preparation of 5'-acetic acid-2',5'-dideoxyribonucleoside subunits.

Triethylamine pyridine/sulfur trioxide complex, p-nitrophenol, dicyclohexylcarbodiimide, and 40% aqueous dimethylamine are obtained from Aldrich.

3'-O-tert-Butyldimethylsilyl-N-2-(phenylsulfonyl) ethoxycarbonyladenosine (1 mmol), prepared as in Example 12.1, is dissolved in methylene chloride and dimethylsulfoxide (10 mL, 1/1, v/v) at 0°C and triethylamine (3 mmol) and the pyridine/sulfur trioxide complex (3 mmol) is added. The mixture is stirred at this temperature for 1 h, then washed with water, dried over sodium sulfate, and evaporated under reduced pressure. The residue is dissolved in dry pyridine (10 mL) and treated with benzyloxycarbonylmethylenetriphenylyphosphorane (1.5 mmol) For 24 h at 37°C. The mixture is then evaporated to dryness under reduced pressure and the residue dissolved in methylene chloride, washed with dilute HCl and water, then the organic phases are dried over sodium sulfate and solvent removed under reduced pressure. The residue is chromatographed on silica using a gradient of methanol in chloroform (0-20%).

The unsaturated ester (1 mmol) from the previous paragraph is dissolved in ethyl acetate/ethanol (40 mL, 1/1, v/v) containing cyclohexadiene (5 mL) and 10% palladium on charcoal (100 mg) and the suspension is vigorously agitated under a nitrogen atmosphere for 5-24 h. The mixture is filtered and the solvents removed under reduced pressure. The residue is chromatographed on silica gel using a gradient of methanol in chloroform (5-50%).

To a 0.2-0.5 M solution of the acid from the previous paragraph in ethyl acetate (or methylene chloride)

p-nitrophenol is added in about 20% excess. The calculated amount of dicyclohexylcarbodiimide is added to the solution at 0°C. After 0.5 h the mixture is allowed to come to room temperature and was held there for 1 h. The dicyclohexylurea which separated is filtered and bashed with solvent. The combined filtrate and washings are evaporated to dryness under reduced pressure. The ester may be used directly for coupling reactions or may be purified by short column chromatography on silica using an isopropanol in chloroform gradient (0-50%).

The analogous series of reactions may be carried out on the other properly protected 2'-deoxynucleosides.

The active ester (1 mmol) is treated with 40% aqueous dimethylamine (2 mmol) in methanol to form the N,N-dimethylamide of the N-protected 3'-O-tert-butyl dimethylsilyl-2', 5'-dideoxyadenosine-5'-acetic acid. The solvents are removed by evaporation under reduced pressure and the residue is desilylated by using the 2M HF/1M tert-butylammonium fluoride (TBAF) reagent described in B.L. Gaffney and R.R. Jones, Tetrahedron Lett (1982) 23:2257. To the 2M HF/1M tert-butylammonium fluoride in pyridine (1 mmol of TBAF) is added the deprotected nucleoside from the previous paragraph. After stirring for 24 h the reaction is partitioned between methylene and aqueous sodium bicarbonate. The organic layer is washed with water, dried over sodium sulfate, and evaporated to dryness. The residue is purified by chromatography on silica using a gradient of methanol in methylene chloride (5-25%).

## Example 7

### Preparation of Purine and Pyrimidine Pyrrolidones

Potassium t-butoxide, anisoyl chloride, 6-chloropurine, 10% palladium on charcoal, phthaloyl chloride, 2-amino-6-chloropurine, 20% aqueous tetraethylammonium hydroxide, 25% aqueous trimethylamine, 2-pyrimidinone N-bromosuccinimide, trifluoroacetic acid, 4-nitrophenol, and N,N-disuccinimydyl carbonate are obtained from Aldrich. Lithium azide is obtained From Eastman Kodak, Rochester, New York. C18 reverse phase silica gel is obtained from Whatman, Hillsboro, Oregon.

### 7. 1 Preparation of (S)-5-[4-amino-2-oxopyrimidinyl)methyl]-pyrrolidone (henceforth referred to as the cytosine pyrrolidone)

Cytosine (2.2 mmol) is dissolved in dimethyl sulfoxide (2 mL) which contained potassium tert-butoxide (2 mmol). This solution is added to a flask which contained (S)-5-(tosyloxymethyl)-2-pyrrolidone (1 mmol, prepared by the procedure of E. Hardegger and H. Ott, Helv Chim Acta (1955) 38:312). After dissolution, the mixture is stirred at 25°C for 6 h. The mixture is neutralized with acetic acid (2 mmol) and evaporated under reduced pressure. The residue is taken up in dimethyl formamide (5 mL) and evaporated under reduced pressure, and this procedure repeated three times.

### 7.2 Preparation of N-4 anisoylcytosine pyrrolidone

The mixture from the previous paragraph is dissolved in pyridine or N-methylimidazole (10 mL) and treated with anisoyl chloride (2.8 mmol) at room temperature. After stirring for 2 h, the mixture is quenched with ice (0.5 g). After 5 min, 1 mL of 29% ammonia was added. After 15 min at rt the solution is dissolved in ethyl acetate (15 mL) and washed twice with brine (15 mL). The washings are combined and washed with ethyl acetate (2 x 30 mL), the combined organic layers dried with sodium sulfate and evaporated under reduced pressure. The residue is coevaporated several times with toluene and the residue chromatographed on silica gel with a gradient of methanol in methylene chloride (0-20%).

### 7.3 Preparation of 6-chloropurine pyrrolidone

This compound is prepared as for the alkylation of cytosine except that the alkylation is performed on 6-chloropurine and the mixture was heated at 35-95°C for several h. The mixture is cooled to room temperature, neutralized with acetic acid (2 mmol) and evaporated under reduced pressure, shaken with a mixture of 20% methanol/chloroform and 10% sodium bicarbonate. The aqueous layer is washed with chloroform, the combined organic layers aree dried over sodium sulfate and evaporated under reduced pressure. The residue is chromatographed on silica gel using a gradient of methanol in chloroform (0-25%).

### 7.4 Preparation of 6-azidopurine pyrrolidone

The 6-chloropurine pyrrolidone (1 mmol) is treated with lithium azide (2 mmol) in dimethyl sulfoxide (2 mL) at 30-100 °C for several h. At the end of this time the mixture is evaporated under reduced pressure, dissolved in chloroform, washed with 10% sodium bicarbonate, and the organic layer dried over sodium sulfate and concentrated under reduced pressure. The residue is chromatographed on silica using a gradient of methanol in chloroform (0-25%).

7.5 Preparation of adenosine pyrrolidone

The azide from the previous example is hydrogenated by shaking the azide (1 mmol) with 30 pounds of hydrogen pressure in a solution of ethanol (10 mL) containing palladium on charcoal (100 mg, 10% by weight Pd) for 24 h. The solution is filtered through celite and the solvent evaporated. The residue is used directly for the next step.

7.6 Preparation of N-6 phthaloyladenosine pyrrolidone

The phthaloyl group is introduced at the N-6 position by the acylation of the adenosine pyrrolidone (1 mmol) with phthaloyl chloride (1.4 mmol) using the procedure above for the anisoylation of cytidine except that no ammonia was added in the workup.

7.7 Preparation of 2-amino-6-chloropurine pyrrolidone

The pyrrolidone tosylate is alkylated with 2-amino-6-chloropurine as per the preparation of the 6-chloropurine derivative.

7.8 Preparation of guanine pyrrolidone

The chloropurine pyrrolidone (1 mmol) from the previous paragraph is dissolved in diglyme (10 mL) and 25% aqueous trimethylamine (2 mL). The solution is stirred at rt for 5 h, water (10 mL) is added, and the mixture concentrated to 10 mL under reduced pressure. Acetic acid (2 mL) is added, and the mixture was evaporated under reduced pressure to an oil. The residue which contains tetraethylammonium acetate is used directly for the next step.

7.9 Preparation of N-2 acetylguanine pyrrolidone

Guanine pyrrolidone (1 mmol) from the previous paragraph is reacted with acetic anhydride (5 mL) at reflux for 2 h. The solvent is evaporated and the residue coevaporated with dimethylformamide. The residue is dissolved in methanol (5 mL) saturated with ammonia at 0 °C, and the solution is stirred for 2 h at this temperature. The solvent is evaporated under reduced pressure and the residue purified by chromatography on a short column of silica using a gradient of methanol in chloroform (5-50%).

7.10 Preparation of 2-amino-6-azidopurine pyrrolidone

This compound is prepared from the 2-amino-6-chloropurine pyrrolidone by use of the procedure of Example 7.4, except that the reaction requires longer times and was performed at a higher temperature.

7.11 Preparation of 2,6-diaminopurine pyrrolidone

This compound is prepared as for the adenosine pyrrolidone by reduction of the 2-amino-6-azido derivative.

7.12 Preparation of N-2 acetyl N-6 phthaloyl 2,6-diamino purine pyrrolidone

The acetyl group is introduced by the method above for N-2 acetylguanine pyrrolidone except that the methanolic ammonia treatment is carried out for 8 h. The solvents are evaporated and the residue is phthaloylated as for the adenosine derivative.

7.13 Preparation of inosine pyrrolidone

6-Chloropurine pyrrolidone (1 mmol) was converted to the inosine derivative by the method used for the preparation of the guanine pyrrolidone derivative. The compound is purified by chromatography or silica using a gradient of methanol in chloroform (5-25%).

7.14 Preparation of 2-hydroxypyrimidine pyrrolidone

This compound is obtained by the alkylation of 2-pyrimidinone by the procedure used for the preparation of the 6-chloropurine derivative

Example 8

Preparation of Purine and Pyrimidine T-BOC Amino Acids

The general procedure described below for the cytosine pyrrolidone may be applied to all properly protected pyrrolidone derivatives. The product, referred to as the t-BOC acid is formed by cleavage of the pyrrolidone ring to an acid and a t-BOC amine.

8.1 Preparation of the cytosine t-BOC acid

N-4-Anisoylcytosine pyrrolidone (1 mmol) is dissolved in methylene chloride (0.5 M solution) containing di-tert-butyl dicarbonate (2 mmol), triethylamine (1 mmol), and dimethylaminopyridine (1 mmol). The solution is stirred for 10 h at rt. The volatiles are removed and the residue purified on silica using a gradient of methanol in chloroform (0-20%). The residue is dissolved in tetrahydrofuran (0.2 M solution) to which is then added lithium hydroxide ( 3 mmol, as a 1 M solution). After 5 h at rt, the solution is neutralized by the addition of 10% acetic acid (3 mmol) and the solvents removed under reduced pressure. The residue was dissolved in 9N ammonia and stirred at room temperature for 1-10 h. The solvent is removed under reduced pressure and the residue purified by chromatography on C18 reverse phase silica gel using water and methanol (or trifluoroethanol) mixtures.

8.2 Preparation of uracil t-BOC acid

This is prepared from the cytosine t-BOC acid by the following procedure:

The cytosine t-BOC acid (1 mmol) is dissolved in aqueous acetic acid and treated with sodium nitrite (1 mmol) at 4°C. The mixture is stirred for 1 h and evaporated to dryness under reduced pressure. The product is purified by chromatography in C18 reverse phase silica gel using water and methanol (or trifluoroethanol) mixtures.

8.3 Preparation of 5-bromouracil t-BOC acid

This compound is obtained from the uracil t-BOC acid by the following procedure:

The uracil t-BOC acid (1 mmol) is dissolved in DMF (3 mL) and treated with N-bromosuccinimide (1.2 mmol). The solution is allowed to stand at room temperature for 16 h. After removal of the solvents under reduced pressure the product is purified by chromatography on C18 reverse phase silica gel using water and methanol (or trifluoroethanol) mixtures.

8.4 preparation of N-protected t-BOC acid

This general procedure for acylating the exocyclic amino groups of the recognition moiety is also applicable to the protection of the adenosine derivative.

The cytosine t-BOC acid (1 mmol) from the previous paragraph is dissolved in N-methylimidazole or pyridine (5 mL) and treated with chlorotrimethylsilane (5 mmol). After 15 min at room temperature the solution is treated with 2-(4-nitrophenyl)ethylchloroformate (3 mmol, obtained from F. Himmelsbach and W. Pfleiderer, Tetrahedron Lett (1983) 24:3583). The reaction iss maintained at room temperature for 8 h, then cooled in an ice bath, and water (1 mL) was added. After 5 min, 1 mL of concentrated ammonia is added and the mixture stirred at rt for 15 min. The reaction is then evaporated to dryness and the residue dissolved in water. The solution is made acidic with 2 M HCl and extracted with chloroform. The combined organic extracts are dried over sodium sulfate and evaporated under reduced pressure. The residue is purified by chromatography on silica using a gradient of methanol in chloroform (5-50%).

For protection of the guanine and 2,6-diaminopurine derivatives, the above procedure was modified so that the reaction was carried out in pyridine and the acylating agent was 9-fluorenylmethyl chloroformate.

Example 9

Preparation of Purine and Pyrimidine Amino Acids

The following procedure is applicable to all the derivatives. It removes the t-BOC group and frees the primary amino group of the backbone.

The t-BOC acid (1 mmol) was dissolved in 20% trifluoroacetic acid in methylene chloride (5 mL) and stirred at rt for 30 min. Toluene (3 mL) is added and the solvents are evaporated under reduced pressure to give the amino acid trifluoroacetate salt. This is used directly for coupling reactions.

Example 10

Preparation of Subunits for Assembly of Achiral Acyclic Polynucleotide Analogs

10.1 Preparation of the Cytidine Analog

Lithium borohydride, di-tert-butyl dicarbonate and bis(triphenylphosphine) palladium II chloride were obtained from Aldrich Chemical Co., Milwaukee, WI. 2-Amino-5-bromopyrimidine is prepared as per T. Nishikawa, Chem. Pharm. Bull, 9:38 (1961).

2-amino-5-bromopyrimidine is benzoylated at the exocyclic amino group by the general method in Example 2 using benzoyl chloride. The product benzamide (25 mmole) is added to a 45 mL pressure vessel containing triethylamine (10 mL), benzene (10 mL), and bis(triphenylphosphine) palladium II chloride (0.375 mmole). The bomb is flushed with argon, sealed, and pressurized to 600 psi with carbon monoxide and then pressurized to 1200 psi with hydrogen. The reaction vessel is heated in an oil bath with stirring at 145°C. after all gas absorption had ceased, the reaction is cooled, and the gases vented slowly. After addition of anhydrous ether, the reaction mixture is filtered and evaporated in vacuo. The product was isolated following chromatography in silica gel using a gradient of isopropanol in methylene chloride (0-20%).

The 2-benzamidopyrimidine-5-carboxaldehyde (1 mmol) from the previous paragraph is dissolved in ethanol (5 mL) and treated with 4-aminobutyric acid (1 mmole) and triethylamine (1 mmol). After stirring for one hour, the mixture is hydrogenated at 30 psi using 10% palladium on carbon (100 mg). After the absorption of hydrogen has ceased, the reaction is filtered and the solvents removed under reduced pressure. The residue is dissolved in methanol which is saturated with ammonia at 0°C and stirred at room temperature for 10 h. After evapoaration of the solvents, the residue iss purified by chromatography on C18 silica gel using methanol water mixtures buffered to pH 7 with ammonium acetate (0.2 M).

The amino acid (1 mmol) from the previous paragraph is dissolved in ethanol (3 mL) containing di-tert-butyl dicarbonate (1 mmol). Triethylamine (1.5 mmol) is added slowly at room temperature. After the evolution of carbon dioxide has ceased, the solvents are removed in vacuo and the residue used directly for the next reaction.

The t-BOC-amino acid from the previous paragraph is acylated on the exocyclic amino group by the general procedure in Example 2 using 2-(phenylsulfonyl)-ethoxycarbonyl chloride. The product may be purified by chromatography on silica gel using a gradient of methanol in chloroform (0-50%).

The 2-(phenylsulfonyl)-ethoxycarbonyl-protected BOC amino acid from the previous paragraph was converted into the p-nitrophenyl or N-hydroxysuccinimidoyl active ester by the procedures in Examples 11.3 and 11.4.

10.2. Preparation of the Uridine Analog

Ethyl 6-hydroxynicotinate is prepared as per H. Gault, J. Gilbert, and D. Briaucourt, C.R. Acad. Sci., Paris, Ser. C, 266:131 (1968). Lithium borohydride is obtained from Aldrich. Manganese dioxide is prepared as per Vereshchagin et al, Zhurnal Arganicheskoi Khimil, 8:1129 (1972). Dowex resin is obtained from Bio-Rad Laboratories, Richmond, CA.

Ethyl 6-hydroxynicotinate (49 mmole) in tetrahydrofuran (10 mL) is added dropwise to a suspension of lithium borohydride (100 mmol) in tetrahydrofuran (THF) (75mLO at room temperature under nitrogen. The viscous mixture is stirred at 25°C for 16 hours. Then, 60 mL of 20% acetic acid is added to the cooled

suspension. The THF is evaporated under reduced pressure and the remaining aqueous solution is applied to an ion exchange column (Dowex-50W-X8) to remove lithium ion. The column is washed carefully with 250 mL of water, which is then evaporated. Boric acid is removed from the resulting residue by repeated evaporation with methanol. The product is purified by distillation under reduced pressure.

The benzylic alcohol (66 mmol) prepared in the previous paragraph is dissolved in ether (250 mL) and treated slowly (exothermic) with a slurry of manganese dioxide (54 g) in ether (100 mL). After twelve hours at room temperature, the mixture is filtered, the solvent removed under reduced pressure, and the residue distilled in vacuo to give 6-hydroxynicotinaldehyde.

The aldehyde from the previous paragraph is reacted with 4-amino butyric acid as for the preparation of the cytidine analog in Example 11.1

The amino acid from the previous paragraph was converted to the corresponding BOC-derivative as for the preparation of the cytidine analog in Example 10.1

The BOC-amino acid from the previous paragraph iss converted into the p-nitrophenyl or N-hydroxysuccinimidoyl active ester by the procedures in Example 11.3 and 11.4.

## Example 11

### Preparation of 5'-protected, 3'-activated 2'-deoxynucleo- sides.

#### 11.1 2'-deoxynucleoside 5'-O-dimethoxytrityl derivatives

The procedure below is general for the preparation of 2'-deoxynucleoside 5'-O-dimethoxytrityl derivatives:

N-2 9-Fluorenylmethoxycarbonyl-2'-deoxyguanosine (1 mmol) is dissolved in anhydrous pyridine (2.0 mL) and kept at 0 ° C. Dimethoxytrityl chloride (1.2 mmol) is added in, portions (0.15 mmol) during an 8 hour period. After an additional 2 hours methanol (0.5 mL) was added and the solution is concentrated at reduced pressure. The residue is treated with a mixture of sodium bicarbonate (2 mL, 5%) and methylene chloride (5 mL).

The aqueous layer is extracted with methylene chloride (2x5 mL) and the combined organic layers are dried over sodium sulfate and evaporated. The residue is coevaporated several times with toluene and the product is purified by chromatography on silica gel using a gradient of methanol in methylene chloride (0-10%).

#### 11.2 5'-O-dimethoxytrityl-2'-deoxynucleoside 3'-O-(4-nitrophenyl)carbonates.

The preparation of 5'-O-dimethoxytrityl-2 -deoxycytidine 3'-O-(4-nitrophenyl)carbonates is described in the following procedure which is general for the preparation of the 4-nitrophenylcarbonates. Bis (4-nitrophenyl)carbonate and N,N-dimethylaminopyridine are obtained From Sigma.

5'O-Dimethoxytrityl-N-2-(4-nitrophenyl)ethoxycarbonyl-2'-deoxycytidine (1 mmol) is dissolved in anhydrous dimethylformamide (5 mL) at room temperature and treated with bis(4-nitrophenyl)carbonate (2 mmol) and then evaporated under reduced pressure. The residue is dissolved in dimethylformamide (5 mL) and N,N-dimethylaminopyridine (0.1 mmol) is added. The yellow solution is evaporated and allowed to react for 1 h. The reaction mixture is dissolved in chloroform (10 mL) and washed with aqueous HCl (5 mL, 0.1 M). The organic layer is washed twice with aqueous NaOH (5 mL, 0.1 M), water (2 mL), dried over sodium sulfate and evaporated. The residue is applied to a silica gel column and eluted with a gradient of isopropanol in chloroform (0-10%). The fractions containing the product are pooled, evaporated, dissolved in chloroform (10 mL) and the aqueous NaOH washes are repeated to remove traces of 4-nitrophenol. The organic layer is washed with water, dried over sodium sulfate and evaporated. The product is homogeneous by TLC and is used directly for preparation of the dimeric carbonates.

For the preparations of the carbonates of nucleosides containing the FMOC group it is sometimes advantageous to use N-methylimidazole as the catalyst rather than N,N-dimethylaminopyridine.

#### 11.3 N-protected t-BOC p-nitrophenyl ester

The following two procedures prepare active esters for coupling of subunits. It is general for all the t-BOC acid derivatives.

To a 0.2-0.5 M solution of the t-BOC acid from Example 8.4 in ethyl acetate (or methylene chloride), p-nitrophenol is added in about 20% excess. The calculated amount of dicyclohexylcarbodiimide is added to

the solution at 0 ° C. After 0.5 h the mixture is allowed to come to rt and was held there for 1 h. The dicyclohexylurea which separated is filtered and washed with solvent. The combined filtrate and washings are evaporated to dryness under reduced pressure. The ester may be used directly for coupling reactions or may be purified by short column chromatography on silica using an isopropanol-in-chloroform gradient (0-50%).

11.4 N-protected t-BOC N-hydroxysuccinimidoyl ester

To a solution of the t-BOC acid from Example 8.4 (1 mmol) in acetonitrile (5 mL) is added pyridine (1 mmol) and N,N'-disuccinimidyl carbonate (1 mmol). The solution ia stirred at rt for 3 h. The solvents are removed under reduced pressure and the residue dissolved in chloroform, the organic phase washed with 0.1 M HCl and water, dried over sodium sulfate, and evaporated to dryness. The esters are sufficiently pure to be used directly.

Example 12

Formation of a carbonate intersubunit linkage.

The preparation of cytidinyl-(3'-5')-cytidine carbonate is described in the procedures below. This is a general procedure for the formation of the carbonate intersubunit linkage and the deprotection of the oligocarbonate. Note that the base protecting groups must be 2-(4-nitrophenyl)ethoxycarbonyl 2-(phenyl sulfonyl)ethoxycarbonyl, or FMOC. Tert-butyl dimethylsilyl chloride is obtained from Aldrich.

12.1 Preparation of 2'-deoxynucleoside 3'-O-tert-butyldimethylsilyl derivatives.

The preparation of 3'-O-tert-butyldimethylsilyl-N-2-(4-nitrophenyl)ethoxycarbonyl-2'-deoxycytidine is described in the following procedure which is a general procedure for the preparation of 2'-deoxynucleoside-3'-O-tert-butyldimethylsilyl derivatives:

To a stirred solution of 5'-O-dimethoxytrityl-N-4-(4-nitrophenyl)-ethoxycarbonyl-2'-deoxycytidine (1 mmol) and imidazole (3.5 mmol) in dry dimethylformamide or tetrahydrofuran (10 mL) is added dropwise a solution of tert-butyldimethylsilyl chloride (3 mmol) in dry DMF (10 mL). The reaction is stirred for 1 h, quenched with ice, extracted with methylene chloride, washed with water and dried over sodium sulfate. The organic layer is evaporated to dryness under reduced pressure and purified by chromatography over silica gel with methylene chloride as eluant.

To a solution of the 5'-O-dimethoxytrityl derivative frown the previous paragraph (1 mmol) in methylene chloride (5 mmol) is added a solution of zinc bromide in methylene chloride (10 mL, 1 M) containing methanol (15 % v/v). The reaction mixture is stirred for 30 min, quenched with ice, and extracted with methylene chloride. The organic layer is washed with water, dried over sodium sulfate, and evaporated to dryness under reduced pressure. The product is purified by chromatography over silica gel using a gradient of methanol in methylene chloride (0-10%).

5'-O-dimethoxytrityl-N-2-(4-nitrophenyl)ethoxy carbonyl-2'-deoxycytidine-3'-O-(4-nitrophenyl)carbonate (1.5 mmol) and 3'-O-tert-butyldimethylsilyl-N-4-(4-nitrophenyl)ethoxycarbonyl-2'-deoxycytidine (1 mmol) are dissolved in dimethylformamide (5 mL) and the solvent removed under reduced pressure. This is repeated two times. The residue is redissolved in dimethylformamide (5 mL) and treated with N,N-dimethylaminopyridine (0.5 mmol) or N-methylimidazole (1 mmol). The solvent is removed by evaporation under reduced pressure and the reaction allowed to sit overnight at rt. The residue is dissolved in chloroform (20 mL) and washed with aqueous HCl (2 mL, 0.1 M), water (2 mL), aqueous NaOH (2 x 2 mL, 0.2 M) water (2 mL), and the organic layer dried over sodium sulfate and evaporated. The residue is purified on silica gel using a gradient of isopropanol in chloroform (0-30%). The isolated product is homogeneous on TLC and by 400 MMz NMR.

The residue is desilylated by using the 2 M HF/1M tert-butylammonium fluoride (TBAF) reagent described in B.L. Gaffney and R.A. Jones, Tetrahedron Lett (1982) 23:2257. To the 2M HF/1M tert-butylammonium fluoride in pyridine (1 mmol of TBAF) is added the deprotected nucleoside from the previous paragraph. After stirring for 24 h, the reaction is partitioned between methylene chloride and aqueous sodium bicarbonate. The organic layer is washed with water, dried over sodium sulfate, and evaporated to dryness. The residue is purified by chromatography on silica using a gradient of methanol in methylene chloride (5-25%).

The 3'-hydroxydinucleoside carbonates are converted to the 3'-(4-nitrophenyl)-carbonates by the

method in Example 11. These derivatives may be reacted with the 5'-hydroxyl of a nucleoside or oligonucleoside carbonate to prepare higher order oligonucleoside carbonates.

Example 13

Formation of a carbamate intersubunit linkage.

Bis(p-nitrophenyl)carbonate, p-anisoyldiphenyl methyl chloride, N,N-dimethylaniline (DMA), and triethylamine are available from Aldrich Chemical Co.

The required 5'-amino-2',5'-dideoxyribonucleoside (employing acetyl or benzoyl groups for base protection where necessary) (1 mmol) prepared in Example 3 above, is treated with p-anisoyl diphenylmethyl chloride (1.2 mmol) in pyridine. After 12 h the solvent is evaporated and the residue is redissolved in chloroform. This solution is washed once each with aqueous $NaHCO_3$ and water, then dried over $Na_2SO_4$. After evaporation of the solvent, the residue is chromatographed on silica gel eluting with chloroform/methanol/1% triethylamine mixtures.

The 5'-tritylated aminonucleoside (1 mmol) is evaporated twice from DMF, then treated with bis-(p-nitrophenyl)carbonate (2 mmol) in the presence of triethylamine (or N,N-dimethylaminopyridine, or N-methyl imidazole)(catalytic amount) using DMF as the solvent. After 3 h, the solvent is evaporated and the residue dissolved in chloroform. This solution is washed twice with 0.01 N aqueous NaOH, once with water, then dried over $Na_2SO_4$. The solvent is removed by rotovap and the residue is chromatographed on silica gel eluting first with a chloroform/0.1% DMA mixture, then with a chloroform/methanol/0.1% DMA solvent system. The appropriate fractions are combined and evaporated to dryness. The residue is dissolved in a minimum of THF and this solution is added to an excess of hexanes. The precipitated activated nucleoside is collected by filtration and dried under vacuum.

The required 5'-aminonucleoside (1.1 mmol), as prepared in Example 3 above, is twice evaporated from DMF. The activated nucleoside (1 mmol) is added to the reaction vessel and the solids are dissolved in DMF. The solution is concentrated to a small volume and allowed to stand overnight. The solvent is completely removed under vacuum and the resulting residue is dissolved in chloroform. This solution is twice washed with 0.01 N aqueous sodium hydroxide, once with water, then dried over solid sodium sulfate. The solvent is removed by rotovap, and the residue is chromatographed on silica gel eluting with the appropriate methanol/chloroform/1% triethylamine solvent system. The fractions containing the dimer nuclcoside are combined, then evaporated to dryness. The residue is dissolved in a minimum of THF and this solution is added to hexanes. The precipitate is collected and dried under vacuum.

Example 14

Formation of a thiocarbamate intersubunit linkage.

N,N-dimethylaminopyridine (DMAP) is purchased from Aldrich Chemical Co.

Formation of the thiocarbamate intersubunit linkage of the 5'-aminonucleosides prepared in Example 3 is achieved in the same manner as described above in Example 13 with the following alterations in procedure. The 5'-aminonucleosides employed have as base-protecting moieties either 2-(phenylsulfonyl)-ethoxycarbonyl (for deoxycytidine and deoxyadenosine) or 9-FMOC (for guanosine and 2,6-diamino-2'-deoxyriboside) groups. The preparation of these molecules are described above (Example 3). Activation of the 5'-tritylaminonucleoside (1 mmol) is achieved with p-nitrophenylchlorothioformate (as prepared by G Hilgetag and r. Phillippson, Monatsber Deut Akad Wiss Berlin (1964) 6:897) (1.2 mmol) in the presence of triethylamine (2 mmol) and the DMAP (catalytic amount) in DMF. The coupling step employs this activated monomer (1 mmol) with the requisite 5'-aminonucleoside (1.1 mmol) using DMAP or N-methyl imidazole as catalyst and DMF as the solvent. In this aqueous workup of these steps, the 0.01 N aqueous NaOH washes are omitted, employing in their place water washes.

Example 15

Formation of carbamate and thiocarbamate intersubunit linkages between morpholino type subunits.

N-methylimidazole is purchased from Aldrich Chemical Co.

The requisite dry, N'-protected, 5'-free alcohol morpholino type nucleoside (employing acetyl or benzoyl groups for base protection where necessary) (1 mmol), prepared in Example 5 above is treated with bis-(p-nitrophenyl)carbonate and triethylamine (or DMAP or N-methylimidazole) (catalytic amount) in DMF under anhydrous conditions. The solution is stirred for 3 h, then evaporated to dryness. The residue is dissolved in chloroform and the solution is washed twice with 0.01 N aqueous NaOH, once with water, then dried over $Na_2SO_4$. The solvent is removed by rotovap and the residue is chromatographed on silica gel eluting with an appropriate chloroform/isopropanol/0.1% DMA mixture. The fractions containing the desired activated nucleoside are combined, evaporated, and the resulting solid is dissolved in a minimum of THF. The THF solution is added to an excess of hexanes and the resulting precipitate is collected and dried.

The required 5'-free hydroxy, N'-free morpholino type nucleoside (using the identical base protecting groups listed above in this example) (1.1 mmol), after evaporation twice from DMF, is treated with the 5'-(p-nitrophenoxycarbonyl)morpholino subunit (1 mmol) in DMF. The volume of the reaction solution is reduced under vacuum to a small volume. If required, a catalytic amount of N-methylimidazole or triethylamine is added to the reaction vessel. The resulting solution is allowed to stand overnight at rt. The remaining solvent is evaporated, and the residue is dissolved in chloroform. The chloroform solution is washed twice with 0.01 N aqueous NaOH, once with water, and then dried over $Na_2SO_4$. The solvent is removed by rotovap and the residue is chromatographed on silica gel eluting with chloroform/methanol solvent mixtures. The fractions containing the desired dimer are combined, then rotovaped to dryness. The residue is dissolved in a minimum of THF and the solution is added to an excess of hexanes. The precipitate is collected and dried under vacuum.

Morpholino subunits linked by thiocarbamate moieties are prepared as related above in this example with the following experimental alterations. The base-protecting groups are either 1-(phenylsulfonyl)-ethoxycarbonyl (for deoxycytidine and deoxyadenosine) or 9-FMOC (for deoxyguanosine and 2,6-diamino-2'-deoxyriboside) groups. The preparation of these molecules is described above (Example 5). Activation of the N-protected morpholino nucleoside (1 mmol) is achieved with p-nitrophenylchlorothioformate (1.2 mmol) in the presence of triethylamine (2 mmol) and DMAP or N-methyl imidazole (catalytic amount) in DMF. The coupling step employs this activated monomer (1 mmol) with the requisite N'-free morpholino nucleoside (1.1 mmol) using DMAP or N-methyl imidazole as catalyst and DMF as the solvent while warming the solution to 35-40 ° C. In the aqueous workup of these steps, the 0.01 N aqueous NaOH washes are omitted, employing in their place water washes.

## Example 16

### Preparation of an ester subunit linkage.

N,N-dimethylaminopyridine and N-methylimidazole are obtained from Aldrich.

The coupling of two subunits is performed by reaction of the 5'-N,N-acetamide-3'-hydroxy derivative with the with the 3'-silylated-5'-active ester. These are prepared as in Example 6. The reagents (1 mmol each) are coevaporated several times with dimethylformamide, then dissolved in dimethylformamide (5 mL) and treated with N,N-dimethylaminopyridine (0.2 mmol) or N-methylimidazole (1 mmol). The solvent is removed under reduced pressure and the reaction allowed to stand at room temperature for 2 h. The residue is dissolved in chloroform and washed with dilute HCl, water, and the organic phase dried with sodium sulfate arid solvents evaporated under reduced pressure. The residue is purified by chromatography on silica gel using a gradient of methanol in chloroform.

The chain may be elongated by desilylation as in Example 6, and treatment of the resulting 3'-free hydroxy compound with the active ester as in the previous paragraph.

## Example 17

### Formation of a t-BOC amide-linked dimer

A fully protected t-BOC N-hydroxysuccinimidoyl ester or p-nitrophenyl ester prepared as in Example 11.3 and 11.4 (1 mmol) is dried by several coevaporations with dry dimethyformamide. The protected amino acid trifluoroacetate salt from Example 9 (1 mmol) is treated similarly. The two components are separately dissolved in dry dimethylformamide (2 mL), mixed, and treated with diisopropylethylamine (1.0 mmol). The solution is stirred at room temperature for 1 h, then the solvent is removed by evaporation under reduced pressure, the residue dissolved in chloroform and washed with dilute HCl. The organic layer is dried over sodium sulfate, evaporated to dryness under reduced pressure, and the residue purified by chromatography on silica gel using a gradient of methanol in chloroform (5-50%). Alternatively the t-BOC dimer acid may be purified by chromatography on C18 reverse phase silica gel using water and methanol

(trifluoroethanol) mixtures.

17.1 Preparation of the t-BOC dimer active ester

The acid from the previous paragraph is converted into the N-hydroxysinccinimidoyl ester or p-nitrophenyl ester by application of the general methods in Example 11.3 and 11.4.

17.2 Preparation of the t-BOC dimer amino acid trifluoro acetate

The t-BOC dimer acid is treated with trifluoro acetate as per the general procedure in Example 9.

17.3 Preparation of a t-BOC tetramer acid

The t-BOC dimer active ester and the dimer acid trifluoroacetate are coupled to the t-BOC tetramer acid by the general procedure. Purification is best effected by chromatography on C18 reverse phase silica gel using water and methanol (or trifluoroethanol) mixtures buffered to pH 7.0 with triethylammonium acetate. In this manner chains of any length may be prepared by coupling an active ester with a free amine component.

Example 18

Geometric assembly of an 8-subunit carbamate-linked polymer

DEAE cellulose is purchased from Sigma Chemical Co. Preparative TLC plates are a product of EM Science purchased from VWR Scientific. HPLC equipment columns, and supplies are available from Beckman Instruments, Inc.

The requisite carbamate-linked dimer (0.2 mmol) prepared as described in example 12, is treated with 4 mL of a 1/1/1 mixture of methanol/THF/glacial acetic acid at rt for 12 h. The solvent is removed under vacuum and the residue is taken up in a minimum of THF. The THF solution is added to a large volume of hexanes and the resulting precipitate is collected and dried. The acetate salt is dissolved in a 4/1 THF/ethanol mixture and DEAE cellulose (0.8 mmol of the base) is added to the vessel. After stirring for 20 min, the heterogeneous mixture is filtered and the filtrate evaporated to dryness. The residue is dissolved in a minimum of 4/1 THF/ethanol and this solution is added to hexanes. The solid is collected and dried. The precipitation procedure is repeated once.

The required N-5'-trityl dimer (0.1 mmol) (as prepared in Example 12) is evaporated twice from DMF, then treated with bis(p-nitrophenyl)carbonate (2 mmol) in the presence of triethylamine (or DMAP or N-methyl imidazole) (catalytic amount) using DMF as the solvent. after 3 h the solvent is evaporated and the residue dissolved in chloroform. This solution is washed twice with 0.01 N aqueous NaOH, once with water, then dried over $Na_2SO_4$. The solvent is removed by rotovap and the residue is chromatographed on silica gel eluting with a chloroform/ isopropanol or methanol/0. 1% DMA mixture. The appropriate fractions are combined and evaporated to dryness. The residue is dissolved in a minimum of THF and this solution is added to an excess of hexanes. The precipitated activated dimer is collected by filtration and dried under vacuum.

The 5'-amino dimer nucleoside prepared above (0.11 mmol) is twice evaporated from DMF. The activated dimer (0.1 mmol) is added to the reaction vessel and the solids are dissolved in DMF. The solution is concentrated to a small volume and allowed to stand overnight. The solvent is completely removed under vacuum and the resulting residue is dissolved in chloroform. This solution is twice washed with 0.01 N aqueous sodium hydroxide, once with water, then dried over sodium sulfate. The solvent is removed by rotovap and the residue is chromatographed on a preparative TLC plate eluting with the appropriate methanol/chloroform/1% triethylamine solvent system. The band containing the tetramer nucleoside is eluted and the solvent is evaporated. The residue is dissolved in a minimum of THF and this solution is added to hexanes. The precipitate is collected and dried under vacuum.

The following transformations are performed using the procedures enumerated above in this example. The requisite N-5'-trityl tetramer nucleoside (0.06 mmol) is treated with 1/1/1 THF/methanol/glacial acetic acid, then purified to afford the 5'-aminotetramer nucleoside. Another aliquot of the N-5'-trityl tetramer nucleoside (0.05 mmol) is activated with bis(p-nitro phenyl)carbonate and subsequently purified. The two tetramers are coupled, worked up, and further purification is achieved either by chromatography on a preparative TLC plate or by HPLC chromatography. the chromatographed material is isolated as a solid,

dissolved in a minimum of THF, and then precipitated from hexanes. The octomer nucleoside is collected and dried.

The octamer is configured for further use as follows. The N-5'-trityl octomer nucleoside is treated with 1/1/1 THF/methanol/glacial acetic acid and purified employing the conditions listed above. The 5'-amino octomer nucleoside (0.01 mmol) is treated with succinic anhydride (0.02 mmol) in pyridine at room temperature for two hours. The solvent is removed by rotovap and the residue is evaporated several times from ethanol. The residue is taken up in 3/1 THF/ethanol and added to a 2/1 hexane/benzene mixture. The solid is collected and dried. The N,5'-succinylated octamer nucleoside (0.01 mmol) is treated with a 1/1 mixture of pyridine/concentrated ammonia (1 mL). After standing overnight the solvent is removed by rotovap and the residue is evaporated from ethanol several times. The crude solid is purified using reverse phase (RP-18) HPLC chromatography. The elutants are evaporated and the residue is taken up in DMSO and precipitated from a 1/1 hexane/benzene solvent mixture. The solid is collected and dried.

Example 19

Preparation of a support-bound cleavable linker suitable for stepwise and stepwise/block assembly of polymers: activation of support and attachment of linker.

Long-chain alkylamine-derivatized controlled pore glass (Cat. No. 24875) is obtained from Pierce Chemical Co. 2,2-sulfonyldiethanol and dicyclohexylcarbodiimide are obtained from Aldrich Chemical Co.

The amino groups of the glass support (approximately 40 mmol per grain of support) are reacted with succinic anhydride essentially by the method of Matteucci and Caruthers (J Amer Chem Soc (1981) 103:3185) to give free carboxylic acid termini. One-tenth mol of 2,2'-sulfonyldiethanol (60 % by weight in water) is dried by mixing with three volumes of dimethylformamide (DMF) reducing to a thick syrup under vacuum in a warm water bath, adding a second quantity of DMF, reducing to a thick syrup, and repeating the process a third time. Dry DMF is then added to give a final sulfonyldiethanol concentration of 2 M. A mixture of 1 ml of the sulfonyldiethanol solution plus 0.2 g of dicyclohexylcarbodiimdide is added to 1 g of the dry controlled pore glass support carrying carboxylic acid residues and the slurry mixed by rocking or tumbling (not stirring) overnight at rt. Thereafter, the glass support is thoroughly washed with methanol and dried.

Example 20

Preparation of a 14-subunit carbonate-linked polymer targeted against a conserved sequencein the AIDS viral genome: stepwise assembly on a solid support: addition of the first subunit and chain extension.

N-methylimidazole, 4-(N,N-dimethylamino)pyridine (DMAP), and 1,8-diazabicyclo[5.4.0]undec-7-ene (DBU) are obtained from Aldrich Chemical co. Methyl P-nitrophenyl carbonate is prepared from methyl chloroformate and p-nitrophenol.

The 2'-deoxycytidine subunit(0.1 mmol) prepared as in Example 4.2 (wherein the N4 carries a p-nitrophenethoxycarbonyl moiety, the 5' oxygen carries a di(p-methoxy)trityl moiety (DMT), and the 3' oxygen carries a p-nitrophenoxycarbonyl moiety) is dissolved in a minimal volume of dry tetrahydrofuran (THF) or dimethylformamide and added to 0.5 g of thoroughly dried controlled pore glass support carrying a cleavable linker, prepared as in Example 19. Catalyst (either 1 mmol of N-methylimidazole or 0.1 mmol of DMAP) is introduced and the slurry is mixed by rocking or trembling (not stirring) for 2 hours. The slurry is next filtered, and the solid washed thoroughly with THF.

Any unreacted hydroxyls are capped by adding two ml of THF 1 M in methyl p-nitrophenyl carbonate and 0.5 M in DMAP and mixing for 20 min at rt. Thereafter, the glass support is washed with THF and filtered.

The dimethoxytrityl at the 5' terminus is then removed by washing the support with dichloromethane followed by treatment with 5 ml of 0.2 M dichloroacetic acid in dichloromethane for 5 min at rt. The glass support is next washed with dichloromethane and filtered.

Subsequent subunits, prepared as in Example 1 and activated as in Example 2, are added in a like manner and in the following order: G,A,T,A,A,C,A,T,T, T,T,T,C, (G protected with the FMOC moiety, A and C protected with the p-nitrophenethoxy carbonylmoiety).

Example 21

Preparation of a 19-subunit carbamate-linked polymer targeted against a conserved sequence in the AIDS viral genome. Stepwise assembly of oligomer blocks on a solid support: addition of the first subunit and chain extension.

The 5'-amino-2',5'-dideoxycytidine subunit (0.2 mmol), prepared as in Example 3 (wherein the N4 carries a benzoyl moiety, the 5'-amine carries a p-methoxytrityl moiety, and the 3' oxygen carries a p-nitrophenoxycarbonyl moiety) is dissolved in a minimal volume of dry THF and added to 0.5 g of dried controlled pore glass support carrying a cleavable linker, prepared as in Example 19. Catalyst (either 1 mmol N-methylimidazole or 0.1 mmol of DMAP) is introduced and the slurry mixed by rocking or tumbling for 2 h. The slurry is next filtered, and the solid washed thoroughly with THF.

The mono-p-methoxy trityl is removed by washing with dichloromethane, followed by treatment with 5 ml of 0.2 M dichloroacetic acid in dichloromethane at rt for 1 min. The support is then washed with dichloromethane and filtered. The 5' amino termini are converted to their free amine form by a brief wash with 3 ml of THF containing 1% by volume of diisopropylethylamine, followed by washing with THF and filtration.

0.1 mmol of 3' p-nitrophenoxycarbonyl-activated diner having the sequence 5'-A-G-3', prepared as in Example 13 (wherein the guanine N2 is protected with an acetyl moiety and the adenine N6 is protected with a benzoyl or a p-nitrobenzoyl moiety), is dissolved in a minimal volume of dry THF and added to the glass support and mixing is carried out for 2 h (no catalyst is added for this and subsequent coupling steps). The slurry is next washed with THF and filtered.

Any unreacted amine moieties are capped by adding 2 ml of THF 2 M in p-nitrophenyl acetate and mixing at rt for 20 min. Thereafter, the glass support is washed with THF and filtered.

The mono-methoxytrityl at the 5' terminus is removed with dichloroacetic acid, as before.

Subsequent activated dimeric subunits, prepared as in Example 17 (wherein the exocyclic ring nitrogen of cytosine is protected with a benzoyl moiety and the exocyclic ring nitrogen of A is protected with a benzoyl or a p-nitrobenzoyl moiety), and added in a like manner and in the following order: A-T, C-A, T-A, T-T, T-T, A-C, A-C, C-A (5' to 3').

Subunits having base exocyclic ring nitrogen protective groups removable by strong nonnucleophilic bases (e.g., p-nitrophenethoxycarbonyl or phenylsulfonyl ethoxycarbonyl for A and C, and FMOC for G) can also be used for assembling polymers by the procedure above. However, polymers having these alternative protective groups are generally deprotected by treatment with DBU rather than by ammonium hydroxide.

Example 22

Preparation of a 19-subunit amide-linked polymer targeted against a conserved sequence in the AIDS viral genome. Stepwise assembly of oligomer blocks on a solid support: addition of the first subunit and chain extension.

A cytosine-containing acyclic-backboned subunit (0.2 mmol), prepared as in Example 11.3 (wherein the N4 of the cytosine carries a 2(P-nitrophenyl)ethoxycarbonyl moiety, the amine of the backbone carries a t-butoxycarbonyl moiety, and the carboxyl is in the form of a p-nitrophenyl ester) is dissolved in a minimal volume of dry THF and added to 0.5 g of dried controlled pore glass support carrying a cleavable linker, prepared as in Example 19. Catalyst (either 1 mmol of N-methylimidazole or 0.1 mmol of DMAP) is introduced and the support mixed for 2 h, filtered, and the solid is washed thoroughly with THF.

The t-BOC is removed by washing the support with dichloromethane followed by treatment at rt with 5 ml of 20% by volume trifluoroacetic acid in methylene chloride for 30 min. The support is washed with dichloromethane and filtered. The support-bound amino terminus is converted to the free amine form by a brief wash with 3 ml of THF containing 1% by volume of diisopropylethylamine, followed by a THF wash.

The activated dimeric subunit (0.1 mmol) having the sequence (amino terminus)-G-A-(carboxy terminus) prepared as in Example 17 (wherein the N2 of guanine carries an FMOC moiety, the N6 of adenine carries a p-nitrophenethoxycarbonyl moiety, the backbone amino terminus carries a t-BOC moiety, and the carboxy terminus is in the form of a p-nitrophenyl ester) is dissolved in a minimal volume of dry THF and added to the glass support and mixing is carried out for 2 h (no catalyst is added for this or subsequent coupling steps). The support is washed with THF.

Any unreacted amine moieties are capped by adding 2 ml of 2 M p-nitrophenyl acetate in THF and mixing at rt for 20 min. Thereafter, the glass support is washed with THF.

The terminal t-BOC moiety is removed with TFA and the support neutralized, as described above.

Subsequent dimeric subunits, prepared as in Example 17, are added in a like manner in the following order: (C-terminus) T-A, A-C, A-T, T-T, T-T, C-A, C-A, A-C (N-terminus).

Subunits having exocyclic ring nitrogen protective groups removable by good nucleophiles (e.g., benzoyl for C, benzoyl or nitrobenzoyl for A, and acetyl or isobutyryl for G) call also be used for assembling polymers by the above procedures. However, polymers having these alternative protective groups are generally deprotected by treatment with ammonium hydroxide rather than by DBU.

Example 23

Configuring the carbonate-linked polymer of Example 20 for a solution application.

The 5' oxygen of the support-bound polymer of Example 20 is succinylated with 5 ml of a THF solution 1 M in succinic anhydride and 0.1 M in DMAP for 10 min at rt, washing with THF, and filtering. Next, the polymer is both released from the support and its recognition moieties are deprotected by treatment with 3 ml of dry DMSO containing 0.3 g of DBU at rt for 12 h. The polymer is freed from DBU by two cycles of precipitation with anhydrous either and resuspension in anhydrous DMSO. Thereafter, the polymer is resuspended in aqueous DMSO containing a trace of pH 7.5 buffer to neutralize any residual DBU and purified by anion exchange chromatography.

Example 24

Configuring the carbonate-linked polymer of Example 20 for a solid phase application.

6-Aminocaproic acid is reacted with 2-phenylisopropylphenyl carbonate by the method of Sandberg and Ragmarsson (Int J Peptide Prot Res (1974) 6:111). The N-protected (10 mmol) product is then activated by reaction with 20 mmol of DCC and 50 mmol of p-nitrophenol in a minimal volume of DMF. the resultant product is purified or silica gel developed with ether.

The support-bound polymer of Example 20 is reacted with 1 mmol of the above aminocaproic product plus 0.1 mmol of DMAP in 2 ml of THF for 2 h at rt. The glass support is then washed with THF and filtered. the polymer is both released from the support and its recognition moieties deprotected by treatment with 3 ml of dry DMSO containing 0.3 g of DBU at rt for 12 h. The polymer is freed from DBU by two cycles of precipitation with anhydrous ether and resuspension in anhydrous DMSO.

The phenylisopropoxycarbonyl group is removed by adding 80% aqueous acetic acid and incubating at rt for 2 h. The deprotected polymer is precipitated with ether, resuspended in aqueous DMSO, and purified by cation exchange chromatography.

One gram of cellulose (Type 20, Cat. No. 3504, average particle size of 20 microns, from Sigma Chemical Co.), 1 g of bis(p-nitrophenyl) carbonate, and 0.2 ml of triethylamine are added to 5 ml of DMF and stirred overnight at rt. The slurry is filtered, washed once with 10 ml of DMF, then with 50 ml of methanol, and dried under vacuum. A typical level of activation obtained by this procedure is 23 µmol of p-nitrophenoxycarbonyl moiety bound per gram of cellulose.

The activated cellulose (0.5 g) is washed with DMSO, filtered, and 10 µmol of polymer in 1 ml of DMSO is added followed by 30 µmol of diisopropylethyl amine. This mixture is stirred slowly overnight at rt in a closed vessel, filtered, and washed with DMSO. The support is next washed with aqueous pyridine, rinsed with methanol, and dried.

Example 25

Configuring the carbamate-linked polymer of Example 21 for a solution phase application.

The 5' amino moiety of the support-bound carbamate-linked polymer of Example 21 is succinylated by reacting with 5 ml of a THF 1 M solution in succinic anhydride and 1 M in diisopropylethylamine for 10 min at rt, then washing with THF.

The polymer is released from the support by treatment with 3 ml of DMF containing 0.3 g of DBU. The polymer is freed from the DBU by 2 cycles of precipitation with ether and resuspension in DMF.

Protective groups are removed from the recognition moieties by precipitating the polymers with ether, resuspending in 1 ml of DMSO, and adding 1 ml of concentrated ammonium hydroxide. Generally, when the protective group on adenine is a benzoyl moiety, deprotection is carried out in a closed container at 30 °C for 12 h. Alternatively, when the protective group on adenine is a nitrobenzoyl and the protective group on guanine is an acetyl moiety, deprotection is carried out at rt for 6 h. Volatile components are removed under vacuum and the polymer solution neutralized with pH 7.5 buffer. Finally, the polymer,

diluted in aqueous DMSO, is purified by anion exchange chromatography.

Example 26

Configuring the carbamate-linked polymer of Example 20 for a solid phase application.

The activated 6-aminocaproic acid reagent (1 mmol) of Example 23 in 3 ml of THF is reacted with the support-bound carbamate-linked polymer of Example 21 for 20 min at rt. The support is washed with THF and filtered. The terminal phenylisopropoxycarbonyl moiety is removed by washing with dichloromethane, then reacting with 5 ml of 0.5% trifluoroacetic acid in dichloromethane for 15 min at rt, washing with THF, and filtering.

The polymer is released from the support and protective groups on the recognition moieties are removed as in Example 25, except that the final purification is by chromatography on a cation exchange column.

The purified polymer is linked to a cellulose support as in Example 20.

Example 27

Configuring the amide-linked polymer of Example 22 for a solution phase application.

The support-bound amide-linked polymer of Example 22 is succinylated as in Example 24.

The polymer is released from the support, its protective groups on the recognition moieties are removed, and it is purified as in Example 24.

Example 28

Configuring the amide-linked polymer of Example 22 for a solid phase application.

The support-bound amide-linked polymer of Example 22 is reacted with activated aminocaproic acid and the phenylisopropoxycarbonyl moiety removed as in Example 24.

The polymer is released from the support, its protective groups on the recognition moieties are removed, it is purified, and then linked to a cellulose support essentially as in Example 24.

While preferred embodiments of the invention have been described herein, it will be appraent to those in the art that various modifications and changes can be made without departing from the invention.

## Claims

**1.** A polymer composition effective to bind, with a selected binding affinity, to a single-stranded polynucleotide containing a target sequence of bases, comprising non-homopolymeric, substantially stereoregular polymer molecules of the form:

$$\begin{array}{ccccccc} R_1 & & R_2 & & R_3 & & R_n \\ | & & | & & | & & | \\ B & \sim & B & \sim & B & \sim & B \end{array}$$

where:

(a) R, - $R_n$ are recognition moieties selected from purine, purine-like, pyrimidine, and pyrimidine-like heterocycles effective to bind by Watson/Crick pairing to corresponding, in-sequence bases in the target sequence;

(b) n is such that the total number of recognition moieties is at least 5;

(c) B ≈ B are backbone moieties joined predominantly by chemically stable, substantially uncharged, predominantly achiral linkages selected from the group consisting of:

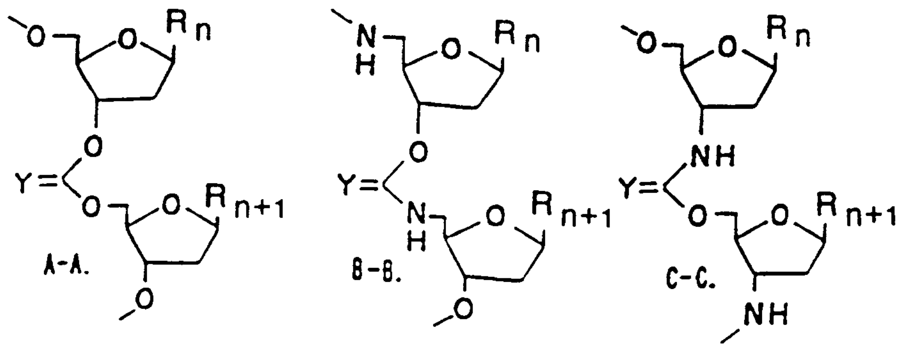

A-A. B-B. C-C.

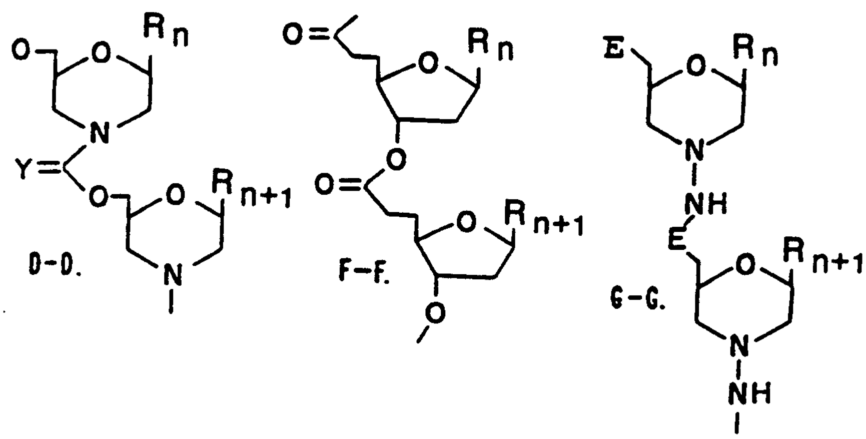

D-D. F-F. G-G.

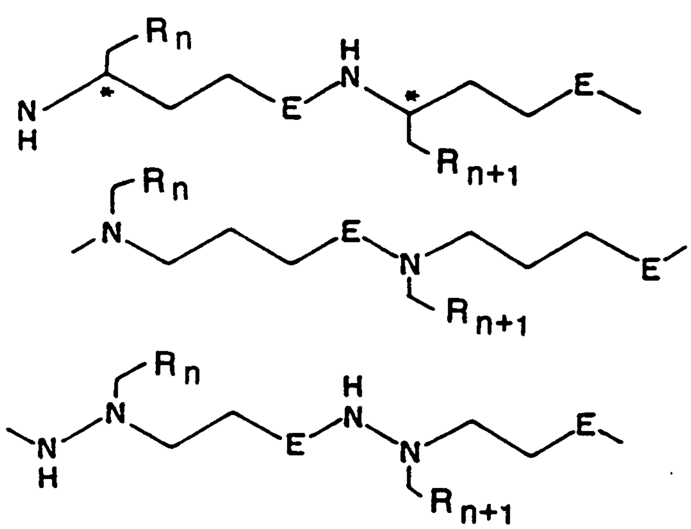

I-I, L-L

J-J, M-M

K-K, N-N

where Y = O or S, and

$$E = \overset{O}{\overset{\|}{C}} \text{ or } \underset{\underset{O}{|}}{\overset{O}{\overset{\|}{S}}} ;$$

and

(d) the backbone moieties support the recognition moieties at positions which allow Watson/Crick base pairing between the recognition moieties and the corresponding, in-sequence bases of the target sequences.

**2.** The composition of claim 1, wherein the recognition moieties are selected from the following group:

1. 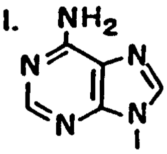
2. 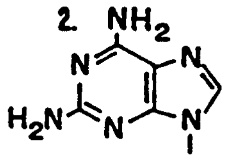
3. 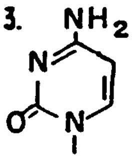
4. 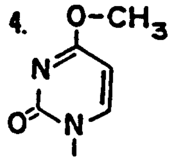
5. 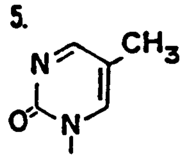
6. 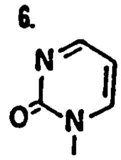
7. 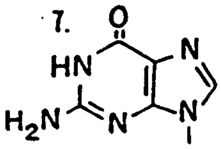
8. 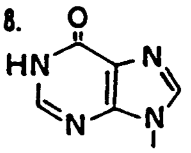
9. 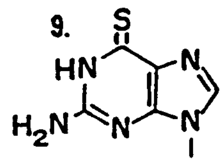
10. 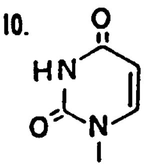
11. 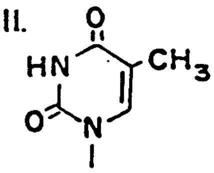
12. 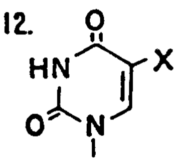 X= F, Cl, Br, I

**3.** The composition of claim 1, wherein the recognition moieties are selected from the following group:

1. 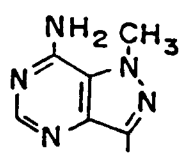
2. 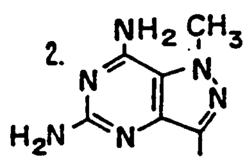
3. 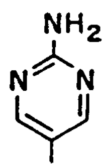
4. 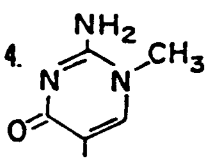
5. 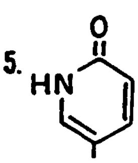
6. 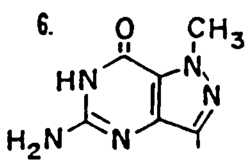
7. 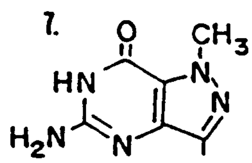
8. 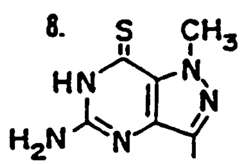

**4.** The composition of claim 1, wherein the backbone moiety B ≈ B has one of the following forms:

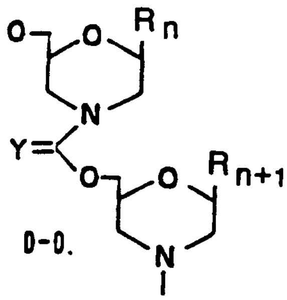

D-D.

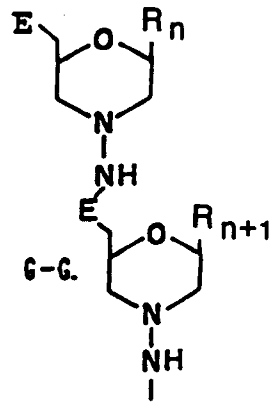

G-G.

where Y = O or S, and

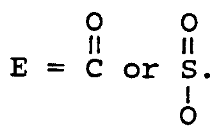

5. The composition of claim 1, wherein the backbone moiety B ≈ B has one of the following forms:

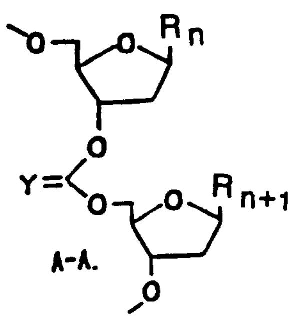

A-A.

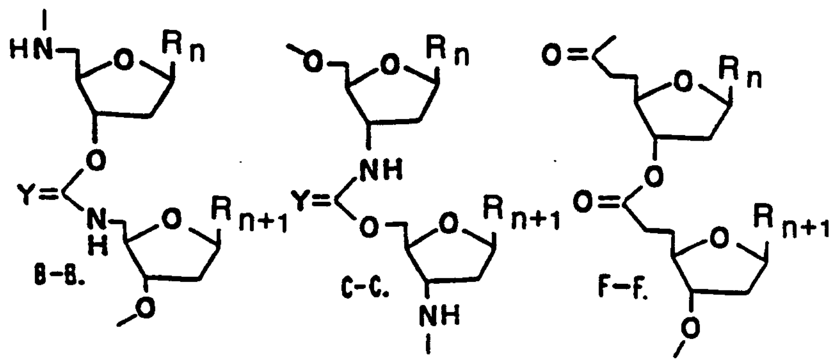

B-B. C-C. F-F.

where Y = S or O.

6. The composition of claim 1, wherein the backbone moiety B ≈ B has one of the following forms:

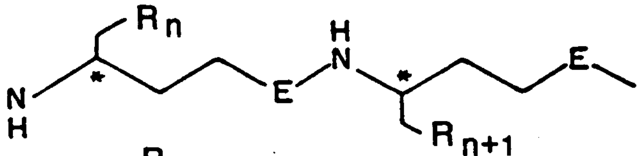

I-I, L-L

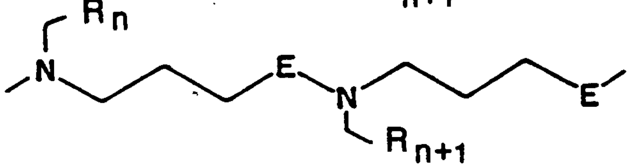

J-J, M-M

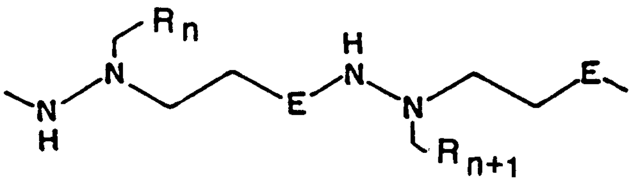

K-K, N-N

where

E = 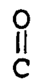 or 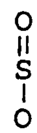.

7. The composition of claim 1, for use in binding to an intracellular target sequence, wherein the polymer molecules contain at most 1-3 ionic charges, and no more than about 1 charge per 4 subunits.

8. The composition of claim 7, wherein the polymer molecules contain charged groups at one or both molecule ends.

9. A method of preparing a substantially uncharged polymeric composition effective to bind specifically and with a substantially uniform binding affinity to a single-stranded polynucleotide, said method comprising:

(1) selecting a specific sequence of bases in the polynucleotide, said sequence constituting the target sequence;

(2) providing a group of subunits of the form B-R, where:

(a) R is a recognition moiety selected from purine, purine-like, pyrimidine, and pyrimidine-like heterocycles effective to bind by Watson/Crick pairing involving two or three hydrogen bonds to corresponding bases contained in the selected target-specific sequence;

(b) B is a backbone moiety which can be coupled to another backbone moiety B by a chemically stable, uncharged, achiral linkage selected from the group consisting of:

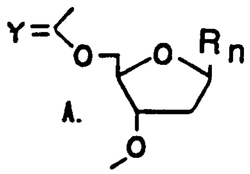
A.

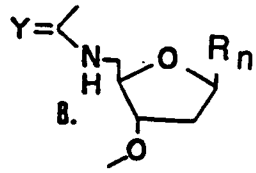
B.

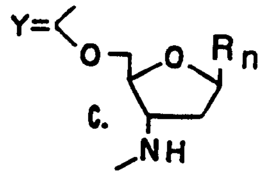
C.

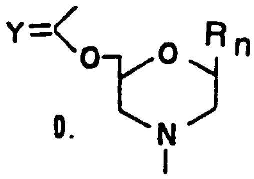
D.

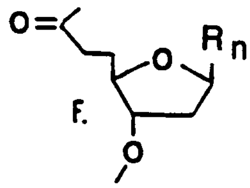
F.

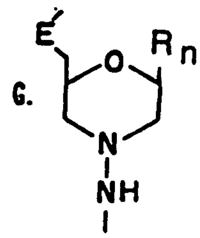
G.

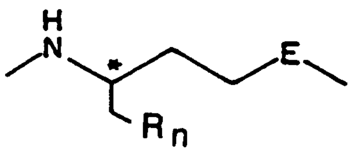

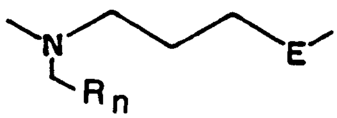

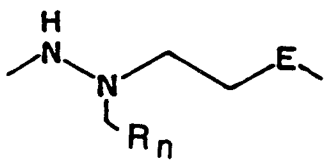

where Y = O or S, and

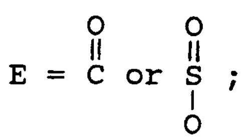

and

(c) the backbone moieties, when coupled, form a backbone which supports the recognition moieties at positions which allow Watson/Crick base pairing between the recognition moieties and the corresponding, in-sequence bases of the target sequences; and

(3) coupling at least 5 subunits in a defined sequence corresponding to the sequence of bases in the target-specific sequence.

**10.** The method of claim 9, which further includes providing recognition moieties for at least one base in the target-specific sequence, where the recognition moieties can form either 2 or 3 hydrogen bonds to corresponding bases contained in the selected target sequence, and adjusting the binding affinity of the composition for the target sequence by decreasing the ratio of recognition moieties which form 2 hydrogen bonds and recognition moieties which form 3 hydrogen bonds, to increase the binding affinity, and increase the ratio, to decrease the binding affinity.

**11.** The method of claim 9, which further includes introducing a group which carries an ionic charge at or near neutrality at one or both ends of the polymer molecules, to increase the solubility of the polymer in an aqueous medium.

**12.** The method of claim 10, wherein recognition moieties which form 2 hydrogen bonds are selected from the following group:

1. 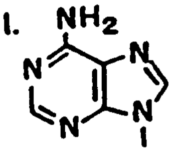
4. 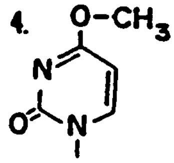
5. 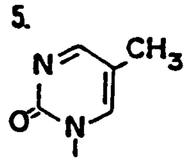
6. 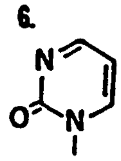

8. 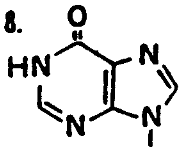
10. 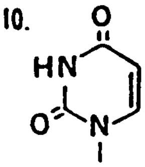
11. 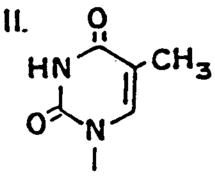
12. 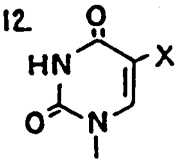

and recognition moieties which form 3 hydrogen bonds are selected from the following group:

2. 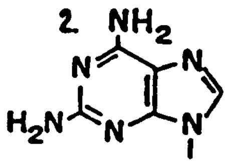
3. 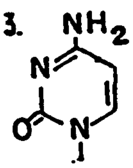

7. 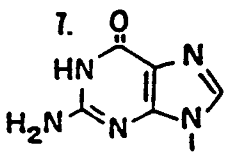
9. 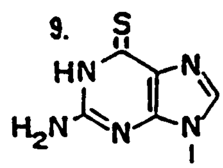

where X = F, Cl, Br, or I.

**13.** The method of claim 9, wherein B is a cyclic backbone moiety, and adjacent backbone moieties, when linked together, have one of the following forms:

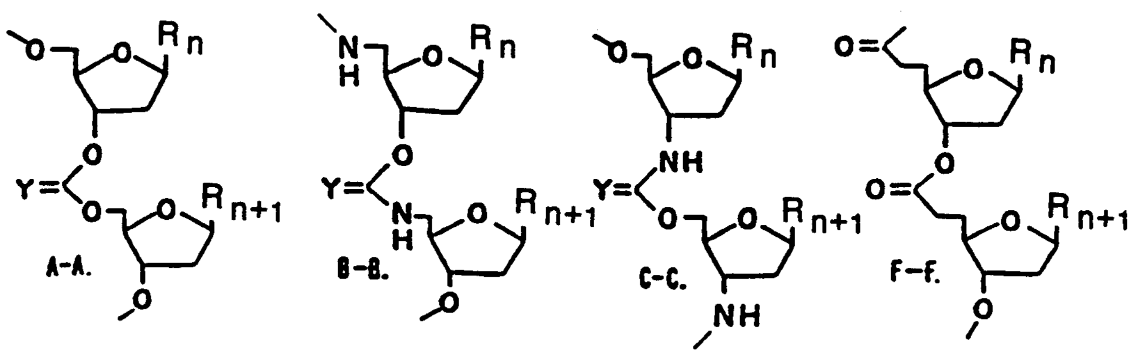

A-A. B-B. C-C. F-F.

where Y = S or O.

**14.** The method of claim 9, wherein B is a cyclic backbone moiety, and adjacent backbone moieties, when linked together, have one of the following forms:

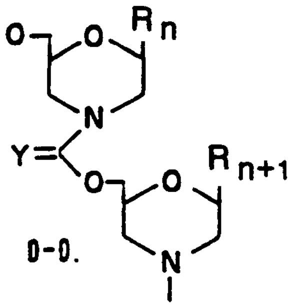

D-D.

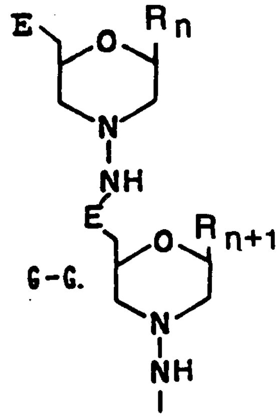

G-G.

where Y = S or O, and

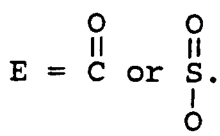

**15.** The method of claim 9, wherein B is an acyclic backbone moiety and adjacent backbone moieties, when linked together, have one of the following forms:

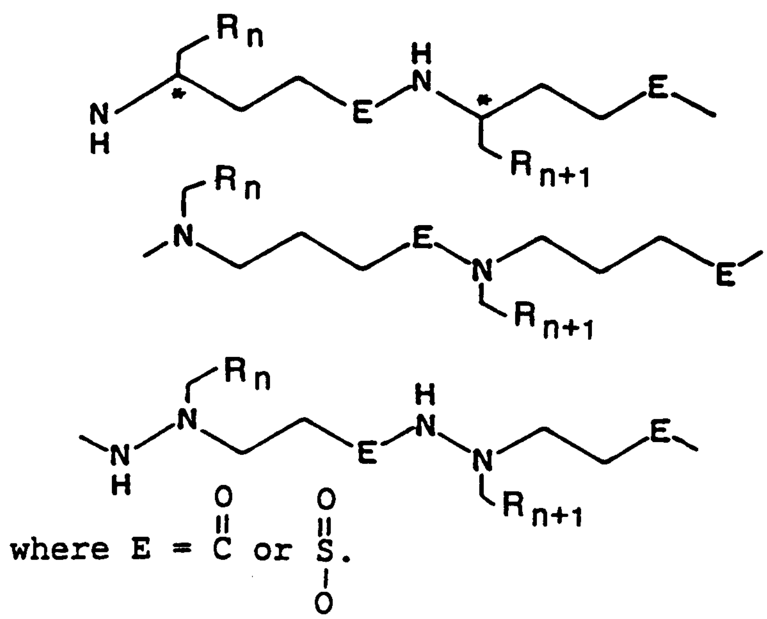

I-I, L-L

J-J, M-M

K-K, N-N

where E =

**16.** A method for inhibiting expression of a single-stranded polynucleotide contained in a system in which a selected activity is expressed, said method comprising:

(1) selecting a target sequence in the polynucleotide which is required for such expression,

(2) providing a group of subunits of the form: B ~ R, where:

(a) R is a recognition moiety selected from purine, purine-like, pyrimidine, and pyrimidine-like heterocycles effective to bind by Watson/Crick pairing involving two or three hydrogen bonds, to corresponding bases contained in the selected target sequence;

(b) B is a backbone moiety which can be coupled to another backbone moiety B by a chemically stable, uncharged, achiral linkage selected from the group consisting of:

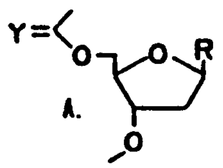
A.

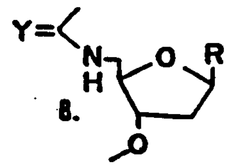
B.

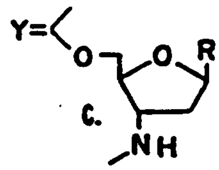
C.

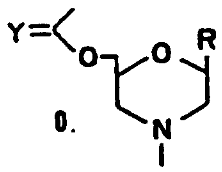
D.

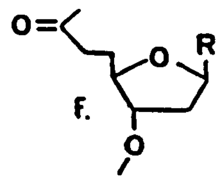
F.

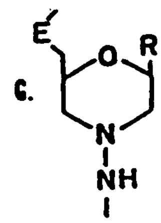
G.

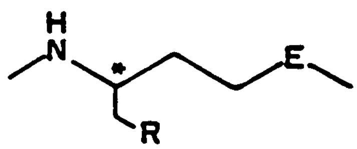

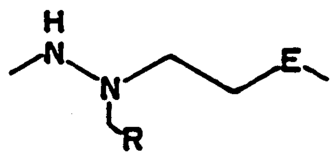

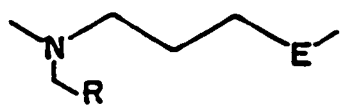

where Y = O or S, and

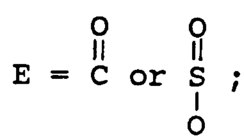

and

(c) the backbone moieties, when coupled, form a backbone which supports the recognition moieties at positions which allow Watson/Crick base pairing between the recognition moieties and the corresponding, in-sequence bases of the target sequences;

(3) coupling at least 5 subunits in a defined sequence to form a predominantly uncharged, stereoisomer polymer designed to bind specifically to the target sequence,

(4) adding the polymer to the system and observing the extent of inhibition of the polynucleotide expression,

depending on the degree of inhibition of polynucleotide expression observed, adjusting the binding affinity of the polymer for the target sequence by one or both of the steps of (a) varying the length of the target sequence and the corresponding polymer length, or (b) varying the ratio of recognition moieties which form three and two hydrogen bonds with corresponding in-sequence polynucleotide bases; and

by said adjusting producing a polymer whose binding affinity is substantially the lowest affinity which is required to effect such inhibition of polynucleotide expression.

**17.** A morpholino subunit of the form:

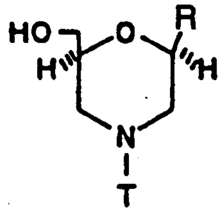

where R is a base-pairing moiety capable of binding by Watson/Crick pairing to complementary bases in a polynucleotide,

and where T is H or an N-protective group.

**18.** The morpolino subunit of claim 16, where R is selected from the group consisting of:

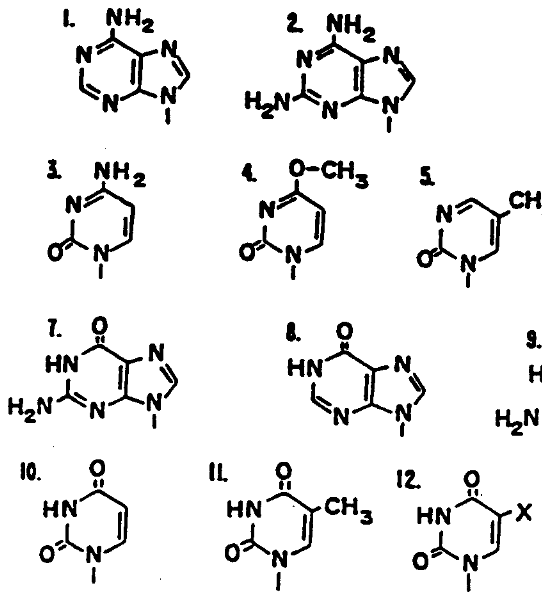

1. 2. 3. 4. 5. 6. 7. 8. 9. 10. 11. 12.

X=F, Cl, Br, I

**19.** The morpolino subunit of claim 17, wherein R contains an N-protected exocyclic amine.

## Patentansprüche

**1.** Polymerzusammensetzung, die mit einer ausgewählten Bindunasaffinität an ein Einzelstrang-Polynucleotid bindet, das eine Targetbasensequenz enthält, umfassend nichthomopolymere, im wesentlichen sterisch regelmäßige Polymermoleküle der Form:

$$\begin{array}{cccc} R_1 & R_2 & R_3 & R_n \\ | & | & | & | \\ B \sim & B \sim & B \sim & B \end{array}$$

worin:

(a) $R_1$ - $R_n$ Erkennungsgruppen sind, ausgewählt aus Purin, Purin-ähnlichen, Pyrimidin und Pyrimidin-ähnlichen Heterocylen, die mittels Watson/Crick-Paarung an korrespondierende, in-Sequenz Basen der Targetsequenz wirksam binden,

(b) n derart ausgewählt ist, daß die Gesamtzahl der Erkennungsgruppen mindestens 5 ist,

(c) B ≈ B Rückgratgruppen sind, die überwiegend mittels chemisch stabilen, im wesentlichen

ungeladenen, überwiegend achiralen Bindungen miteinander verknüpft sind, ausgewählt aus der Gruppe bestehend aus:

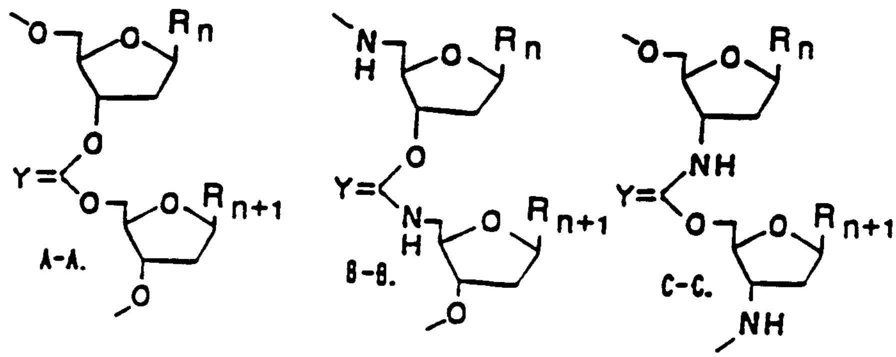

A-A. B-B. C-C.

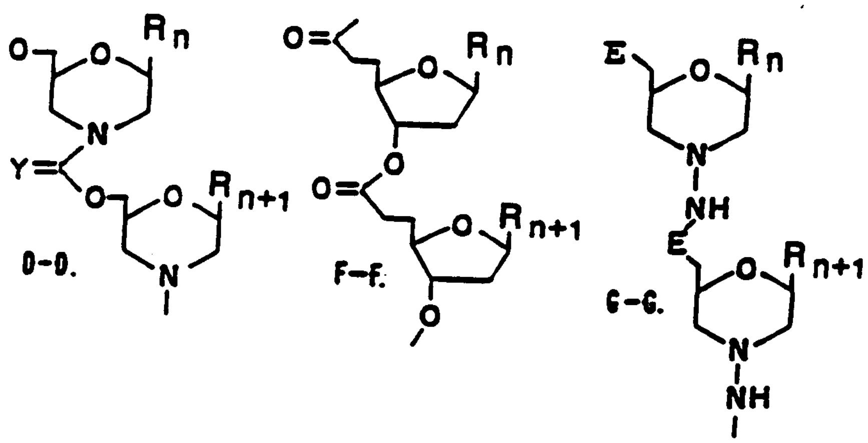

D-D. F-F. G-G.

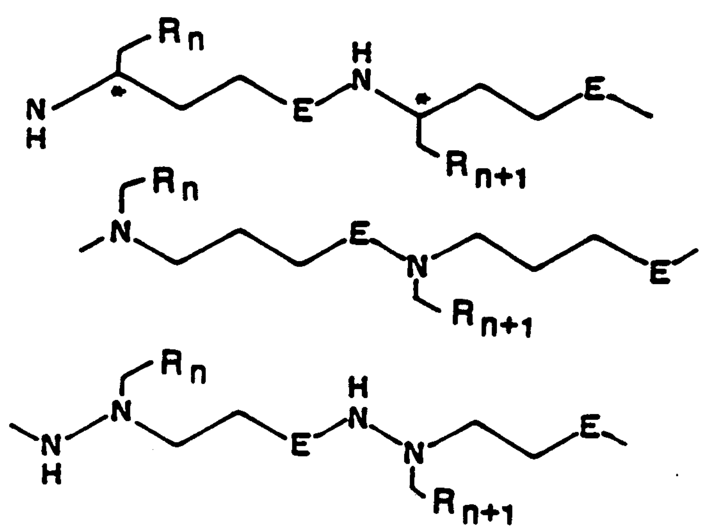

I-I, L-L

J-J, M-M

K-K, N-N

wobei Y = O oder S, und

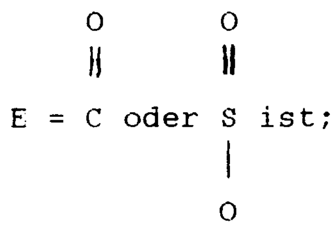

und

(d) diese Rückgratgruppen die Erkennungsgruppen an solchen Stellen tragen, die eine Watson/Crick-Basenpaarung zwischen den Erkennungsgruppen und den korrespondierenden in-Sequenz Basen der Targetsequenzen ermöglichen.

2. Zusammensetzung nach Anspruch 1, in der die Erkennungsgruppen aus der folgenden Gruppe ausgewählt sind:

1. 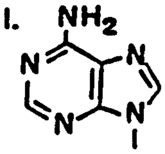
2. 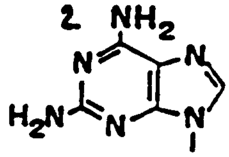

3. 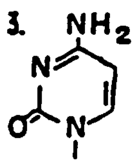
4. 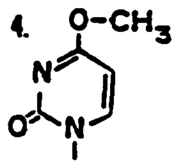
5. 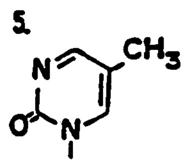
6. 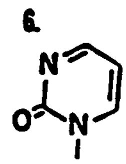

7. 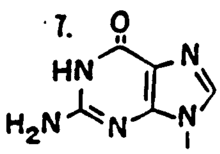
8. 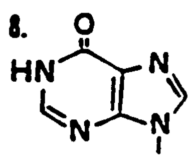
9. 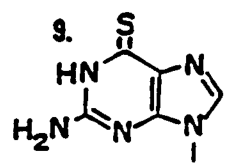

10. 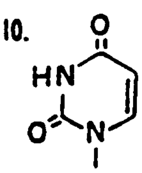
11. 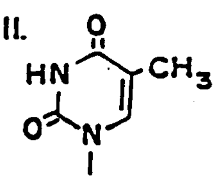
12. 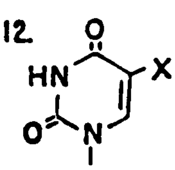
X=F, Cl, Br, I

3. Zusammensetzung nach Anspruch 1, in der die Erkennungsgruppen aus der folgenden Gruppe ausgewählt sind:

1. 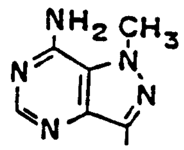

2. 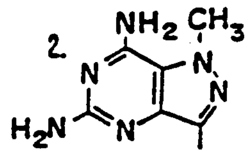

3. 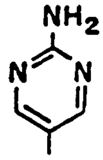

4. 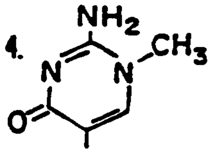

5. 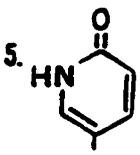

6. 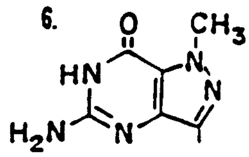

7. 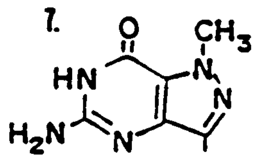

8. 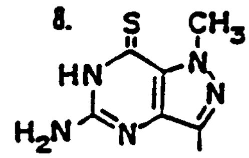

**4.** Zusammensetzung nach Anspruch 1, in der die Rüchgratgruppe B ≈ B eine der folgenden Formen aufweist:

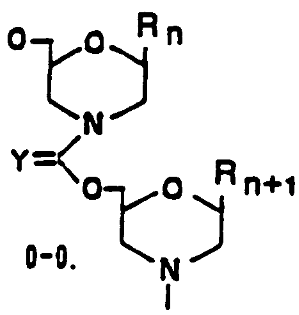

O-O.

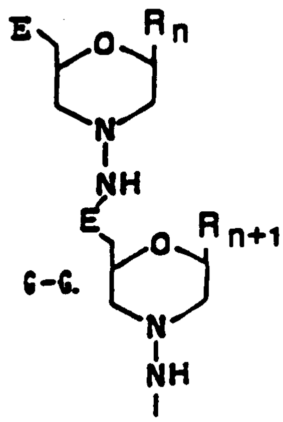

C-C.

wobei Y = O oder S, und

```
        O       O
        "       "
    E = C oder  S ist.
                ¦
                O
```

**5.** Zusammensetzung nach Anspruch 1, in der die Rückgratgruppe B ≈ B eine der folgenden Formeln aufweist:

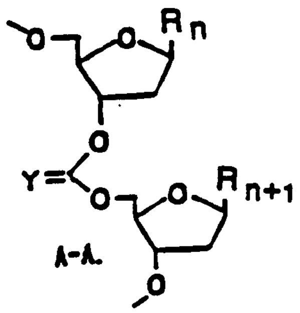

A-A.

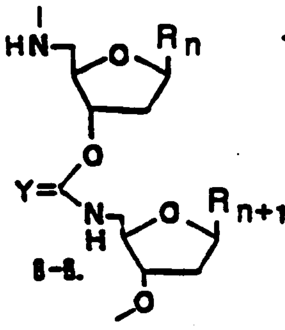

B-B.

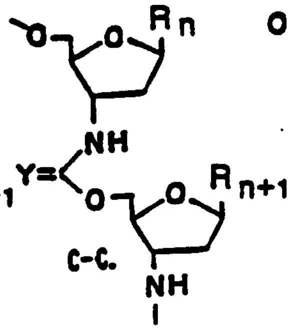

C-C.

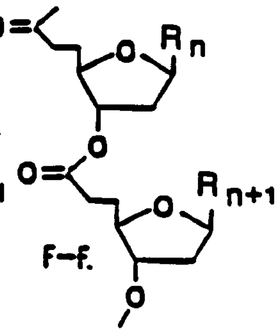

F-F.

worin Y = S oder O ist.

6. Verbindung nach Anspruch 1, in der die Rückgratgruppe B ≈ B eine der folgenden Formen aufweist:

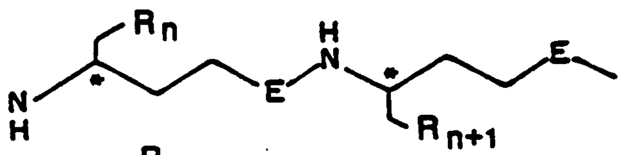

I-I, L-L

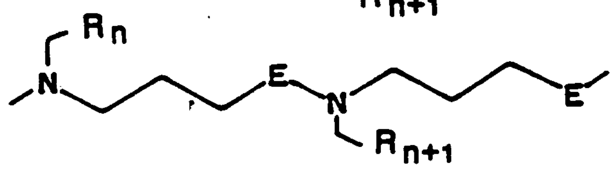

J-J, M-M

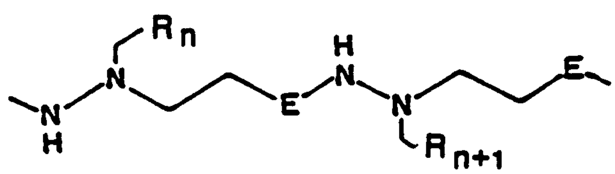

K-K, N-N

worin

$$E = \overset{O}{\overset{\|}{C}} \text{ oder } \overset{O}{\overset{\|}{\underset{\underset{O}{|}}{S}}} \text{ ist.}$$

7. Zusammensetzung nach Anspruch 1 zur Verwendung für das Binden an eine intrazelluläre Targetsequenz, worin die Polymermoleküle höchstens 1 bis 3 ionische Ladungen und nicht mehr als etwa eine Ladung pro 4 Untereinheiten enthalten.

8. Zusammensetzung nach Anspruch 7 worin die Polymermoleküle an einem oder an beiden Molekülenden geladene Gruppen aufweisen.

9. Verfahren zur Herstellung einer im wesentlichen ungeladenen polymeren Zusammensetzung, die spezifisch und mit einer im wesentlichen gleichförmigen Bindungsaffinität an ein Einzelstrang-Polynucleotid wirksam bindet, umfassend:

(1) das Auswählen einer spezifischen Basensequenz in dem Polynucleotid, wobei diese Sequenz die

Targetsequenz bildet,

(2) das Bereitstellen einer Gruppe von Untereinheiten der Form B-R, worin:

(a) R eine Erkennungsgruppe darstellt, die ausgewählt ist aus Purin, Purin-ähnlichen, Pyrimidin und Pyrimidin-ähnlichen Heterocyclen, die mittels einer Watson/Crick-Paarung unter Ausbildung von zwei oder drei Wasserstoffbrückenbindungen an die korrespondierenden Basen wirksam binden, die in der ausgewählten targetspezifischen Sequenz enthalten sind,

(b) B eine Rückgratgruppe darstellt, die mit einer anderen Rückgratgruppe B durch eine chemisch stabile, ungeladene, achirale Bindung verknüpft werden kann, ausgewählt aus der Gruppe bestehend aus:

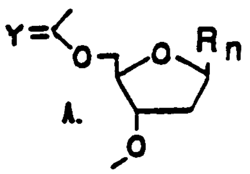
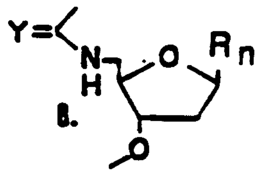
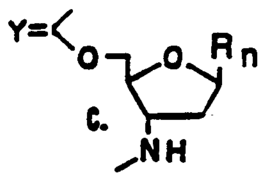

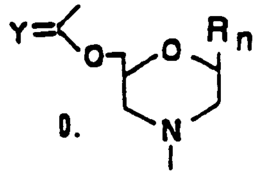
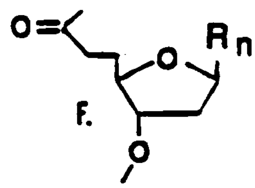
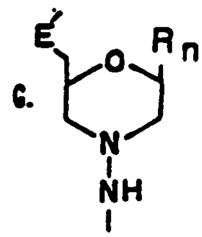

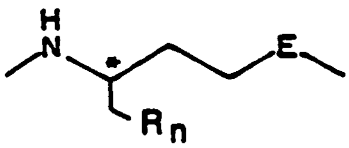

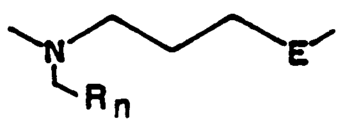

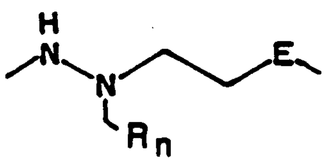

worin Y = O oder S und

$$E = \overset{O}{\overset{\parallel}{C}} \text{ oder } \underset{\underset{O}{\parallel}}{\overset{O}{\overset{\parallel}{S}}} \text{ ist,}$$

und

(c) die Rückgratgruppen, wenn sie verknüpft sind, ein Rückgrat bilden, das die Erkennungsgruppen an den Stellen trägt, die eine Watson/Crick-Basenpaarung zwischen den Erkennungsgruppen und den korrespondierenden in-Sequenz Basen der Targetsequenzen ermöglichen, und

(3) das Verknüpfen von mindestens 4 Untereinheiten in einer definierten Sequenz, die der Basensequenz in der targetspezifischen Sequenz entspricht.

**10.** Verfahren nach Anspruch 9, welches darüberhinaus die Bereitstellung von Erkennungsgruppen für mindestens eine Base in der targetspezifischen Sequenz umfaßt, wobei die Erkennungsgruppen mit den in der ausgewählten Targetsequenz enthaltenen Basen zwei oder drei Wasserstoffbrückenbindungen ausbilden können und welches das Einstellen der Bindungsaffinität der Zusammensetzung an die Targetsequenz durch Herabsetzen des Verhältnisses der Erkennungsgruppen, die zwei Wasserstoffbrückenbindungen ausbilden sowie von Erkenntingsgruppen, die drei Wasserstoffbrückenbindungen ausbilden, umfaßt, um so die Bindungsaffinität zu erhöhen, und daß man dieses Verhältnis erhöht, um die Bindungsaffinität herabzusetzen.

**11.** Verfahren nach Anspruch 9, das darüberhinaus die Einführung einer Gruppe unmfaßt, welche an einem oder beiden Enden des Polymermoleküls eine neutrale oder nahezu neutrale Ladung trägt, um die Löslichkeit des Polymers in einem wäßrigen Medium zu erhöhen.

**12.** Verfahren nach Anspruch 10, bei dem die Erkennungsgruppen, die zwei Wasserstoffbrückenbindungen ausbilden, ausgewählt sind aus der folgenden Gruppe:

1. 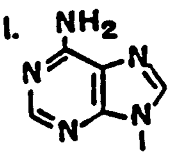
4. 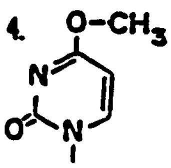
5. 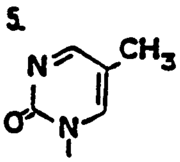
6. 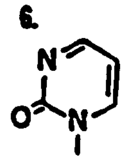

8. 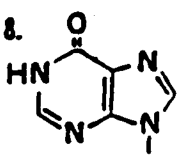
10. 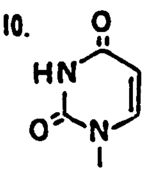
11. 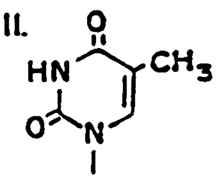
12. 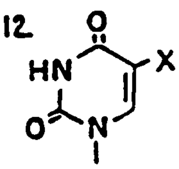

und die Erkennungsgruppen, die drei Wasserstoffbrückenbindungen ausbilden, ausgewählt sind aus der folgenden Gruppe:

2. 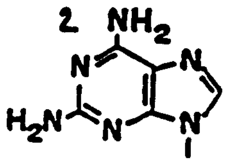
3. 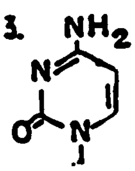

7. 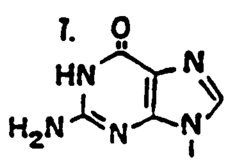
9. 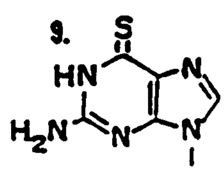

worin X = F, Cl, Br oder I ist.

**13.** Verfahren nach Anspruch 9, in dem B eine cyclische Rückgratgruppe ist und benachbarte Rückgratgruppen, wenn sie miteinander verknüpft sind, eine der folgenden Formen aufweisen:

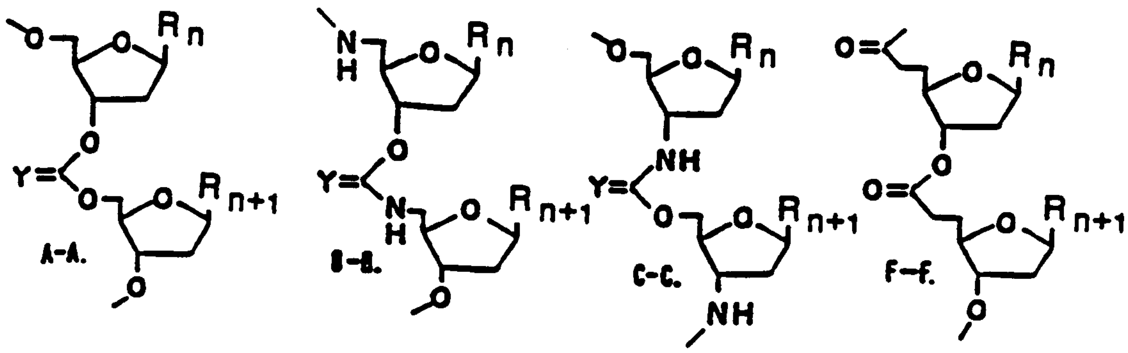

A-A.  B-B.  C-C.  F-F.

worin Y = S oder O.

**14.** Verfahren nach Anspruch 9, worin B eine cyclische Rückgratgruppe ist und angrenzende Rückgratgruppen, wenn sie miteinander verbunden sind, eine der folgenden Formen aufweisen:

D-D.

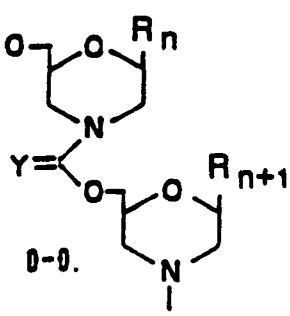

G-G.

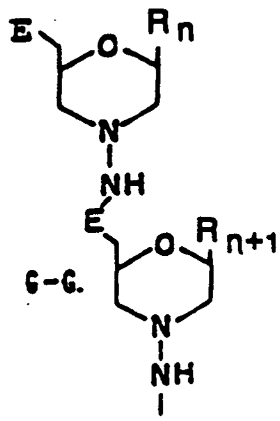

worin Y = S oder O und

O O<br>
" "<br>
E = C oder S ist.<br>
|<br>
O

**15.** Verfahren nach Anspruch 9, worin B eine acyclische Rückgratgruppe ist und benachbarte Rückgratgruppen, wenn sie miteinander verbunden sind, eine der folgenden Formen aufweisen:

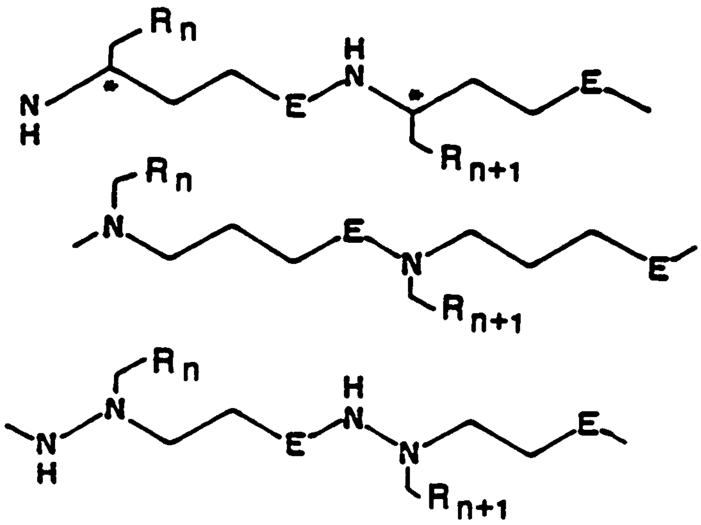

I-I, L-L

J-J, M-M

K-K, N-N

worin

$E = \overset{\overset{O}{\parallel}}{C}$ oder $\underset{\underset{O}{\parallel}}{\overset{\overset{O}{\parallel}}{S}}$ ist.

**16.** Verfahren zum Inhibieren der Expression eines Einzelstrang-Polynucleotids, das in einem System enthalten ist, in dem eine ausgewählte Aktivität exprimiert wird, umfassend:

(1) das Auswählen einer Targetsequenz in dem Polynucleotid, das für eine solche Expression benötigt wird,

(2) das Bereitstellen einer Gruppe von Untereinheiten der Form: B ~ R, worin:

(a) R eine Erkennungsgruppe ist, ausgewählt aus Purin, Purin-ähnlichen, Pyrimidin und Pyrimidin-ähnlichen Heterocyclen, die durch Watson/Crick-Paarung unter Ausbilden von zwei oder drei Wasserstoffbrückenbindungen wirksam an die korrespondierenden Basen binden, die in der ausgewählten Targetsequenz enthalten sind,

(b) B eine Rückgratgruppe darstellt, die mit einer anderen Rückgratgruppe B mittels einer chemisch stabilen, ungeladenen, achiralen Bindung verknüpft werden kann, ausgewählt aus der Gruppe bestehend aus:

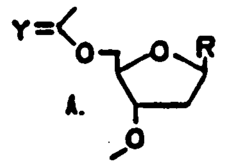
A.

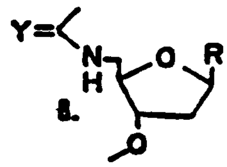
B.

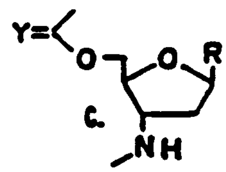
C.

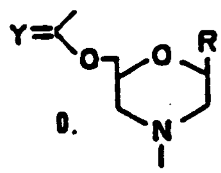
D.

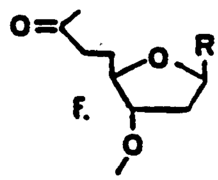
F.

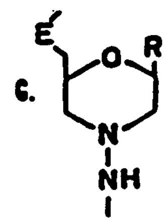
G.

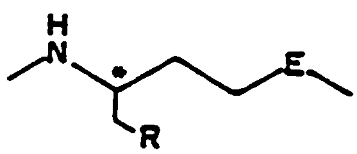

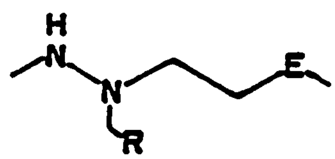

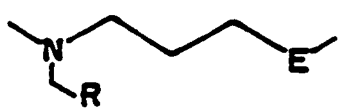

worin Y = O oder S und

$$E = \overset{\overset{O}{\|}}{C} \text{ oder } \underset{\underset{O}{|}}{\overset{\overset{O}{\|}}{S}} \text{ ist,}$$

und

(c) die Rückgratgruppen, wenn sie verknüpft sind, ein Rückgrat ausbilden, welches die Erkennungssequenzen an den Stellen trägt, die eine Watson/Crick-Basenpaarung zwischen den Erkennungsgruppen und den korrespondierenden in-Sequenz Basen der Targetsequenz ermöglichen,

(3) das Verknüpfen von mindestens drei Untereinheiten in einer definierten Sequenz, um ein überwiegend ungeladenes, sterisch isomeres Polymer zu bilden, das zur spezifischen Bindung an die Targetsequenz entworfen wurde,

(4) das Zugeben des Polymers zu dem System und das Beobachten des Ausmaßes der Inhibierung der Polynucleotidexpression,

je nach dem Grad der Inhibierung der beobachteten Polynucleotidexpression, das Einstellen der Bindungsaffinität des Polymers für die Targetsequenz durch einen oder beide der folgenden Verfahrensschritte, (a) Variieren der Länge der Targetsequenz und der korrespondierenden Polymerlänge oder (b) Variieren des Verhältnisses der Erkennungsgruppen, die drei oder zwei Wasserstoffbrückenbindungen mit den korrespondierenden in-Sequenz Polynucleotidbasen bilden und

wobei man durch dieses Anpassen ein Polymer herstellt, dessen Bindungsaffinität im wesentlichen die niedrigste Affinität aufweist, die notwendig ist, um eine solche Inhibierung der Polynucleotidexpression zu bewirken.

17. Eine Morpholino-Untereinheit der Form:

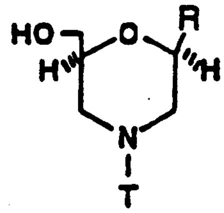

worin R eine mit Basen paarende Gruppe darstellt, die an die komplementären Basen in einem Polynucleotid durch Watson/Crick-Paarung binden kann,
und worin T eine N-protektive Gruppe oder H darstellt.

18. Morpholino-Untereinheit nach Anspruch 16, worin R ausgewählt ist aus der Gruppe bestehend aus:

1. 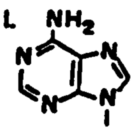 2. 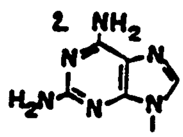

3. 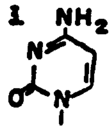 4. 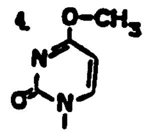 5. 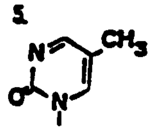 6. 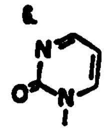

7. 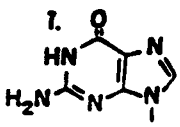 8. 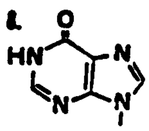 9. 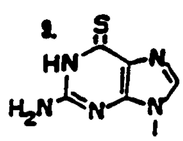

10. 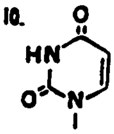 11. 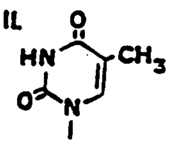 12. 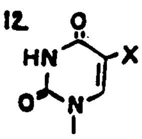 X=F, Cl, Br, I

19. Morpholino-Untereinheit nach Anspruch 17, worin R ein N-geschütztes, exocyclisches Amin enthält.

## Revendications

1. Composition de polymère susceptible de se lier, avec une affinité de liaison donnée, à un polynucléotide à un seul brin contenant une séquence de bases cible, comprenant des polymères non homopolymériques, sensiblement stéréoréguliers, de forme

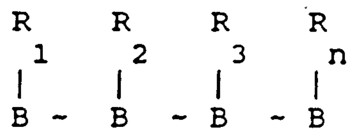

dans laquelle :

(a) $R_1$ -$R_n$ sont des fractions de reconnaissance choisies parmi la purine, les hétérocycles de type purine, la pyrimidine et les hétérocycles de type pyrimidine susceptibles de se lier par un appariement de type Watson/Crick à des bases correspondantes en séquence dans la séquence cible ;

(b) n est tel que le nombre total de fractions de reconnaissance soit d'au moins 5 ;

(c) B ≈ B sont des fractions de squelette liées principalement par des liaisons chimiquement stables, sensiblement non chargées et principalement achirales, choisies dans le groupe constitué par :

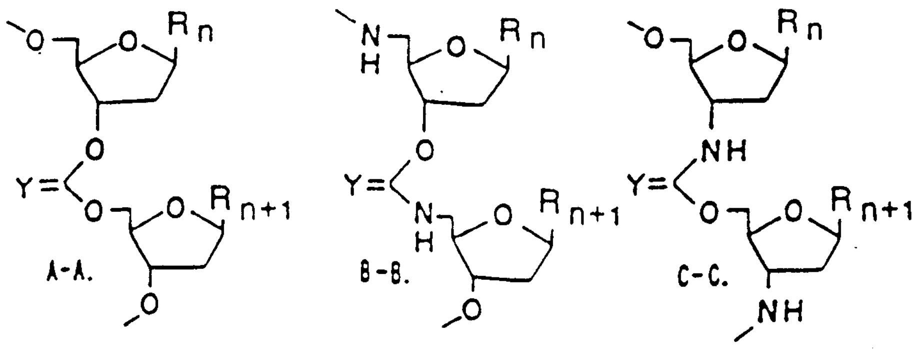

A-A. B-B. C-C.

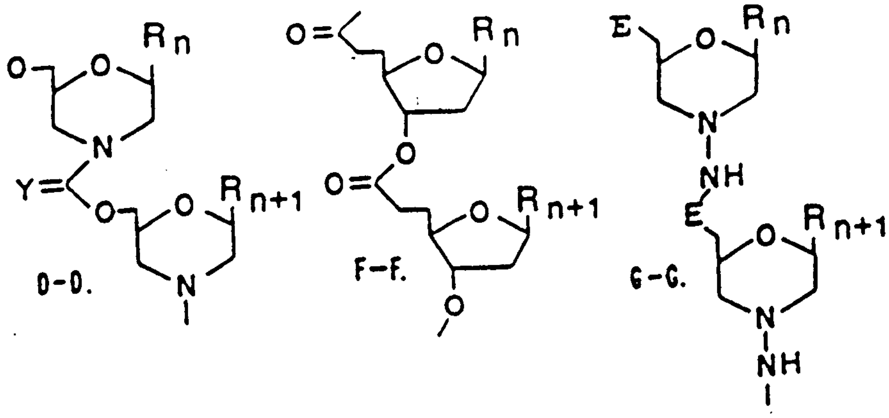

D-D. F-F. G-G.

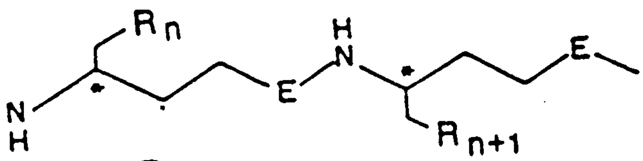

I-I, L-L

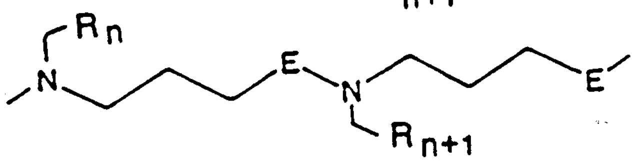

J-J, M-M

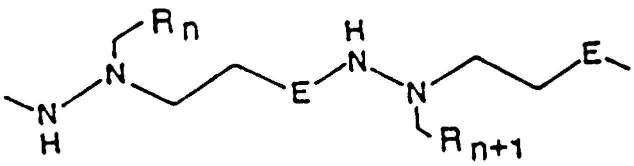

K-K, N-N

où Y = O ou S, et

E = 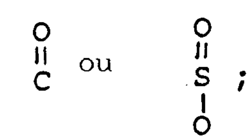 ;

et

(d) les fractions de squelette supportent les fractions de reconnaissance aux positions qui permettent l'appariement des bases de type Watson/Crick entre les fractions de reconnaissance et les bases correspondantes en séquence des séquences cibles.

2. Composition de la revendication 1, dans laquelle les fractions de reconnaissance sont choisies dans le groupe suivant :

1. 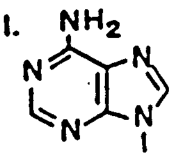
2. 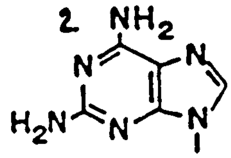

3. 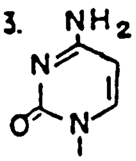
4. 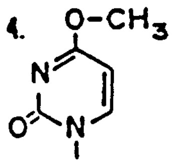
5. 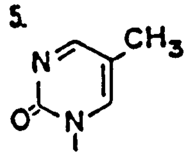
6. 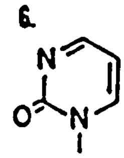

7. 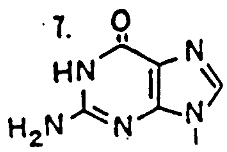
8. 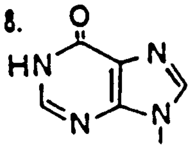
9. 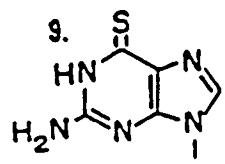

10. 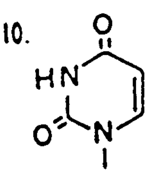
11. 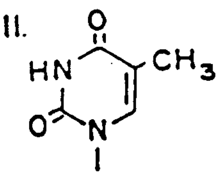
12. 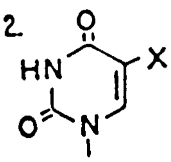 X= F, Cl, Br, I

3. Composition de la revendication 1, dans laquelle les fractions de reconnaissance sont choisies dans le groupe suivant :

1. 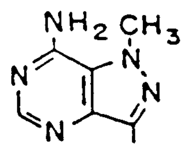
2. 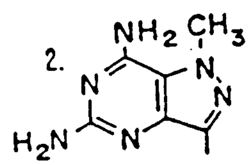

3. 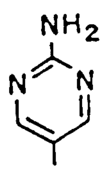
4. 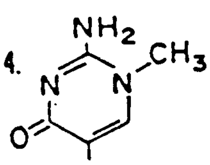
5. 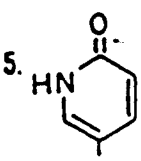

6. 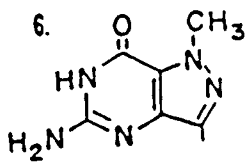
7. 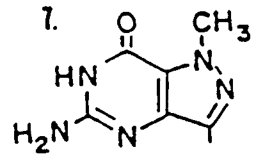
8. 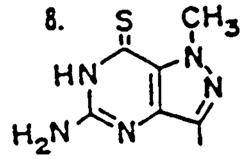

4. Composition de la revendication 1, dans laquelle la fraction de squelette B ≈ B a l'une des formes suivantes :

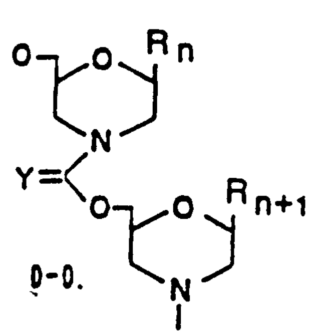

D-D.

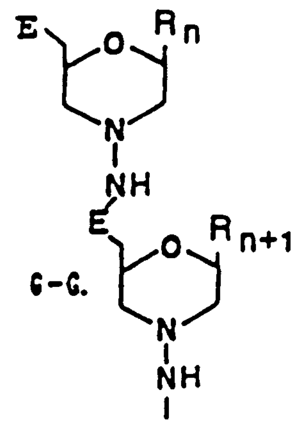

C-C.

où Y = O ou S, et

E = 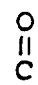 ou 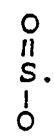

5. Composition de la revendication 1, dans laquelle la fraction de squelette B ≈ B a l'une des formes suivantes :

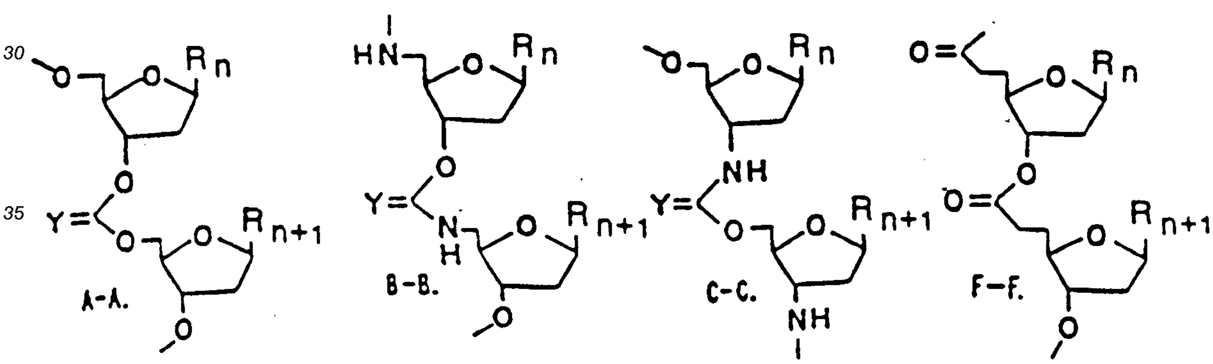

A-A. B-B. C-C. F-F.

où Y = S ou O.

6. Composition de la revendication 1, dans laquelle la fraction d'ossature B ≈ B a l'une des formes suivantes :

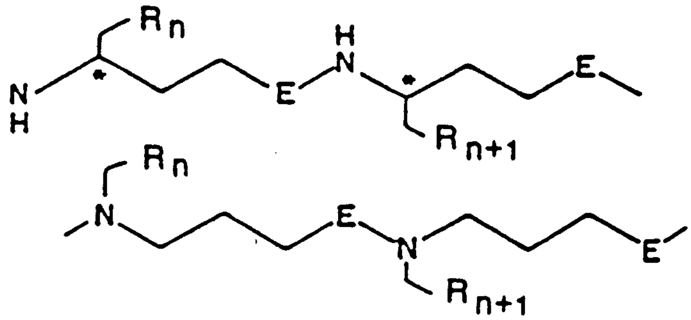

I-I, L-L

J-J, M-M

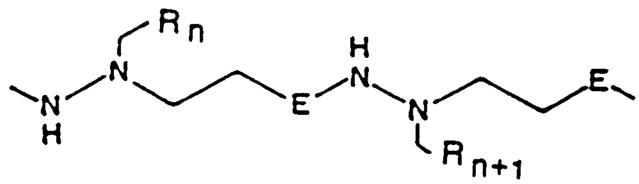

K-K, N-N

où

E = 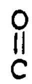 ou 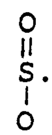.

7. Composition de la revendication 1, pour utilisation dans une liaison à une séquence cible intracellulaire, où les molécules de polymère contiennent au plus 1 à 3 charges ioniques, et pas plus d'environ 1 charge pour 4 sous-unités.

8. Composition de la revendication 7, où les molécules de polymère contiennent les groupes chargés à l'une des extrémités des molécules ou aux deux extrémités.

9. Procédé de préparation d'une composition polymérique essentiellement non chargée susceptible de se lier spécifiquement et avec une affinité de liaison sensiblement uniforme à un polynucléotide à un seul brin, ledit procédé comprenant :

(1) la sélection d'une séquence spécifique de bases dans le polynucléotide, ladite séquence constituant la séquence cible ;

(2) la fourniture d'un groupe de sous-unités de forme B-R, où :

(a) R est une fraction de reconnaissance choisie parmi la purine, les hétérocycles de type purine, la pyrimidine, et les hétérocycles de type pyrimidine susceptible de se lier par un appariement de type Watson/Crick faisant intervenir deux ou trois liaisons hydrogènes à des bases correspondantes de la séquence cible spécifique sélectionnée ;

(b) B est une fraction de squelette qui peut être couplée à une autre fraction de squelette B par un lien chimiquement stable, non chargée, achirale, choisie dans le groupe constitué par :

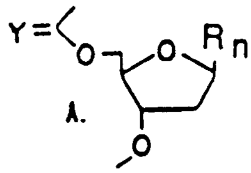 A. 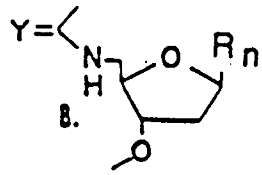 B. 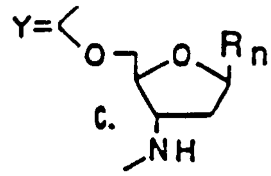 C.

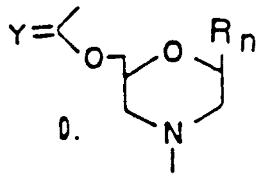 D. 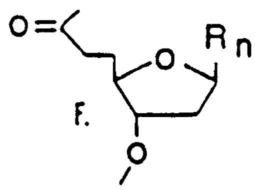 F. 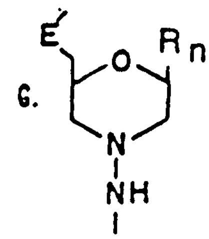 G.

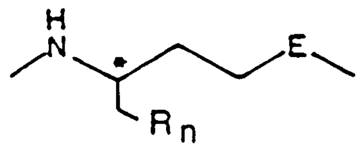

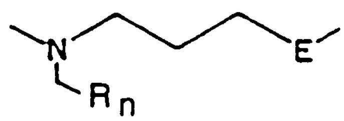

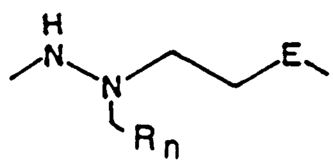

où Y = O ou S, et

E = 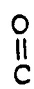 ou 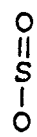

et

(c) les fractions de squelette, une fois couplées, forment un squelette qui supporte les fractions de reconnaissance aux positions qui permettent un appariement de bases de type Watson/Crick entre les fractions de reconnaissance et les bases correspondantes en séquence des séquences cibles ; et

(3) le couplage d'au moins 5 sous-unités dans une séquence définie correspondant à la séquence de bases dans la séquence cible spécifique.

**10.** Procédé de la revendication 9, qui comprend en outre la fourniture de fractions de reconnaissance pour au moins une base dans la séquence cible spécifique, où les fractions de reconnaissance peuvent former deux ou trois liaisons hydrogènes à des bases correspondantes des séquences cibles sélectionnées et où l'on ajuste l'affinité de liaison de la composition pour la séquence cible soit en diminuant la quantité de fractions de reconnaissance qui forment deux liaisons hydrogènes par rapport à la quantité de fractions de reconnaissance qui forment trois liaisons hydrogènes, pour ainsi augmenter l'affinité de liaison soit en augmentant le rapport, pour ainsi diminuer l'affinité de liaison.

**11.** Procédé de la revendication 9, qui comprend en outre l'introduction d'un groupe qui porte une charge ionique à ou au voisinage de la neutralité à l'une des extrémités, ou les deux, des molécules de

polymère, pour augmenter la solubilité du polymère dans un milieu aqueux.

**12.** Procédé de la revendication 10, dans lequel les fractions de reconnaissance qui forment des liaisons hydrogènes sont choisies dans le groupe suivant :

1. 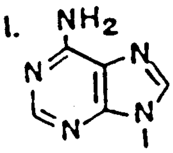
4. 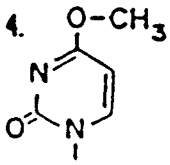
5. 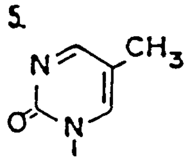
6. 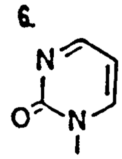

8. 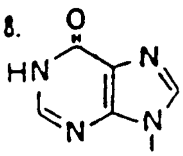
10. 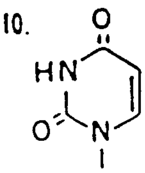
11. 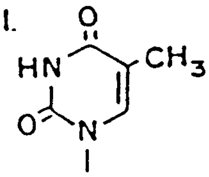
12. 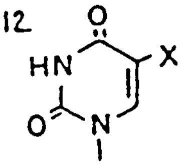

et les fractions de reconnaissance qui forment trois liaisons hydrogènes sont choisies dans le groupe suivant :

2. 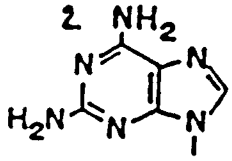
3. 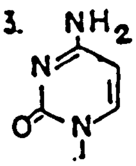

7. 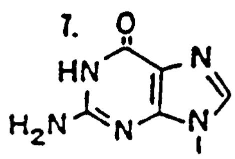
9. 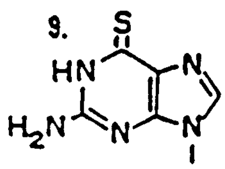

où X = F, Cl, Br ou I.

**13.** Procédé de la revendication 9, dans lequel B est une fraction de squelette cyclique, et les fractions de squelette adjacentes, lorsqu'elles sont réunies, ont l'une des formes suivantes :

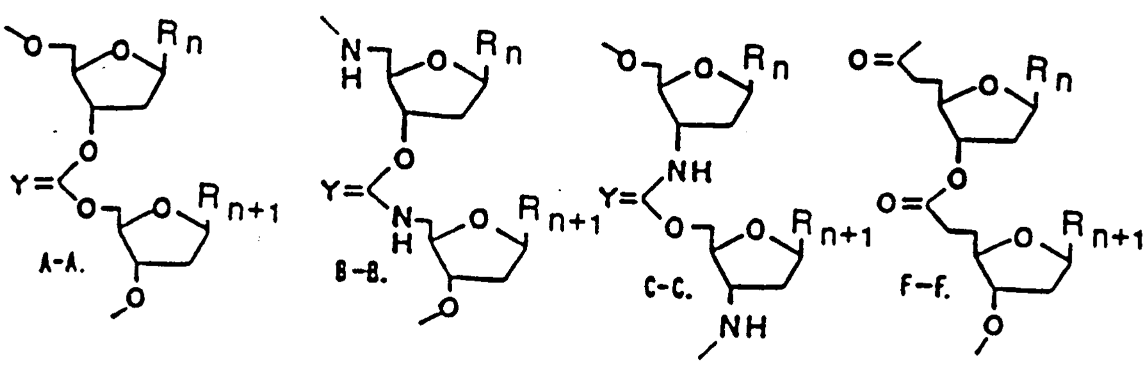

A-A. B-B. C-C. F-F.

où Y = S ou O.

**14.** Procédé de la revendication 9, dans lequel B est une fraction de squelette cyclique, et les fractions de squelette adjacentes, lorsqu'elles sont réunies, ont une des formes suivantes

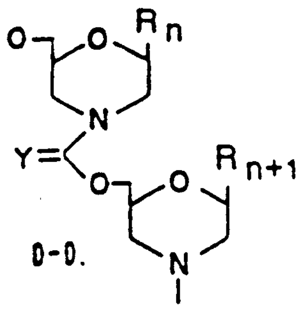

D-D.

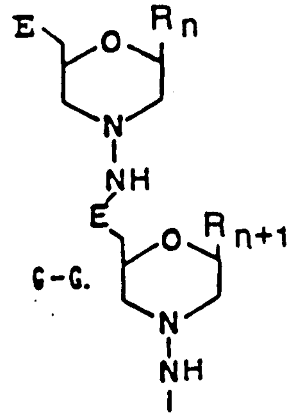

G-G.

où Y = S ou O, et

E = 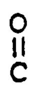 ou 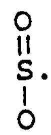

**15.** Procédé de la revendication 9, dans lequel B est un fraction de squelette acyclique et les fractions de squelette adjacentes, lorsqu'elles sont réunies, ont l'une des formes suivantes :

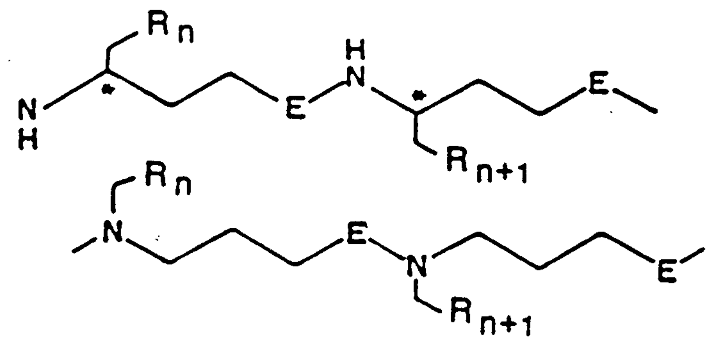

I-I, L-L

J-J, M-M

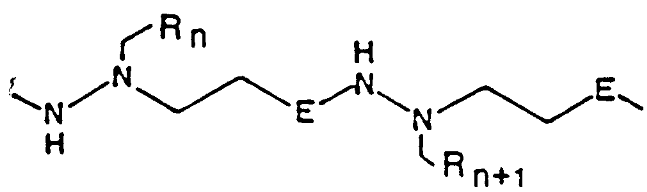

K-K, N-N

où

E = 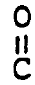 ou 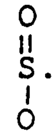

**16.** Procédé pour inhiber l'expression d'un polynucléotide à un seul brin contenu dans un système dans

lequel une activité donnée est exprimée, ledit procédé comprenant :

(1) la sélection d'une séquence cible dans le polynucléotide nécessaire à une telle expression ;

(2) la fourniture d'un groupe de sous-unités de forme : B ~ R, où :

(a) R est une fraction de reconnaissance choisie parmi la purine, les hétérocycles de type purine, la pyrimidine, les hétérocycles de type pyrimidine susceptible de se lier par un appariement de type Watson/Crick faisant intervenir deux ou trois liaisons hydrogènes, à des bases correspondantes de la séquence cible sélectionnée ;

(b) B est une fraction de squelette qui peut être couplée à une autre fraction de squelette B dans un lien chimiquement stable, non-chargée, achirale, choisie dans le groupe constitué par :

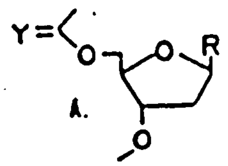
A.

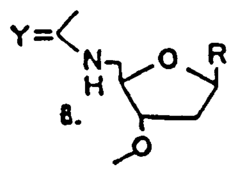
B.

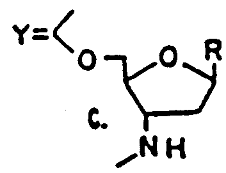
C.

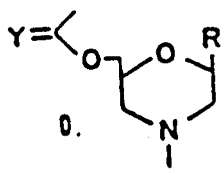
D.

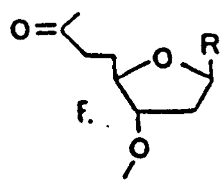
F.

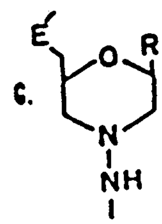
G.

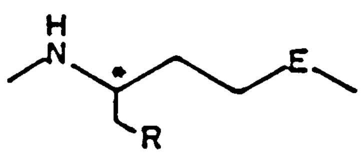

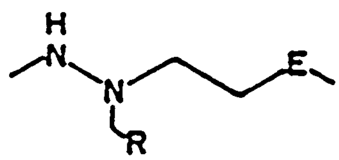

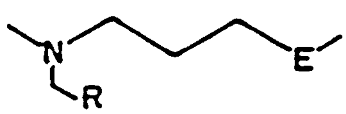

où Y = O ou S, et

E = 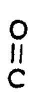 ou 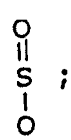 ;

et

(c) les fractions de squelette, lorsqu'elles sont couplées, forment un squelette qui supporte les fractions de reconnaissance aux positions qui permettent un appariement de bases de type Watson/Crick entre les fractions de reconnaissance et les bases correspondantes en séquence des séquences cibles.

(3) le couplage d'au moins 5 sous-unités dans une séquence définie pour former un polymère stéréoisomérique principalement non-chargé conçu pour se lier spécifiquement à la séquence cible,

(4) l'addition du polymère au système et l'observation de la mesure de l'inhibition de l'expression du

polynucléotide,

selon le degré d'inhibition de l'expression du polynucléotide observée, l'ajustement de l'affinité de liaison du polymère pour la séquence cible par une ou deux des étapes consistant à (a) faire varier la longueur de la séquence cible et la longueur du polymère correspondant, ou (b) faire varier la quantité de fractions de reconnaissance qui forment deux liaisons hydrogènes par rapport à la quantité de fractions de reconnaissance qui en forment trois avec des bases polynucléotidiques en séquences correspondantes ; et

par ledit ajustement, la production d'un polymère dont l'affinité de liaison est sensiblement la plus faible affinité nécessaire à obtenir cette inhibition de l'expression d'un polynucléotide.

**17.** Sous-unité morpholino de forme :

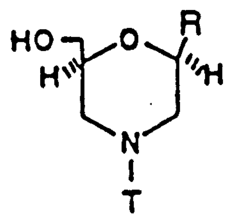

dans laquelle R est une fraction d'appariement de base capable de se lier par un appariement de type Watson/Crick à des bases complémentaires dans un polynucléotide,

et où T représente H ou un groupe protecteur de N.

**18.** Sous-unité morpholino de la revendication 17, dans laquelle R est choisi dans le groupe constitué par :

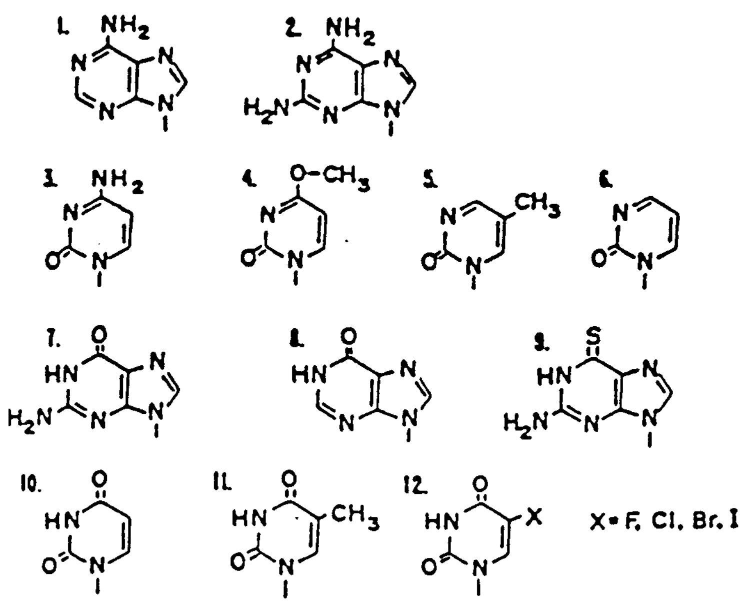

**19.** Sous-unité morpholino de la revendication 17, dans laquelle R contient une amine exocyclique N-protégée.

FIG. 1

1. 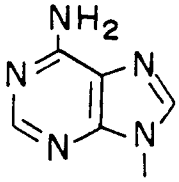

2. 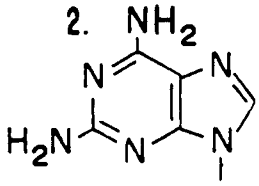

3. 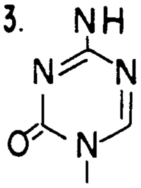

4. 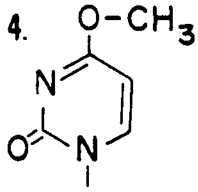

5. 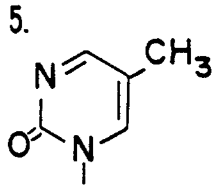

6. 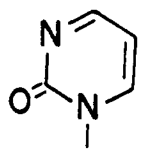

7. 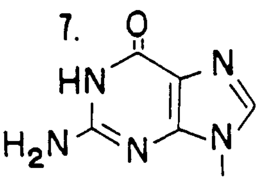

8. 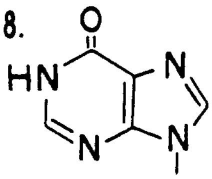

9. 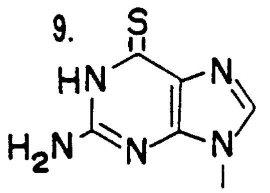

10. 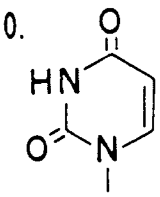

11. 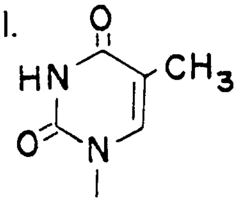

12. 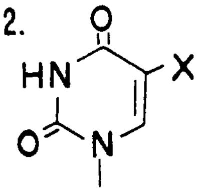

X= F, Cl, Br, I

FIG. 2

1. 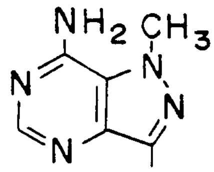

2. 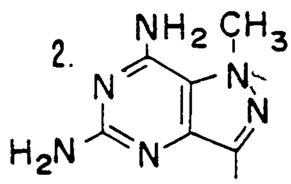

3. 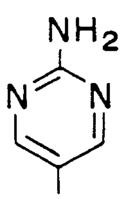

4. 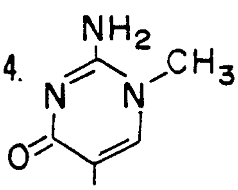

5. 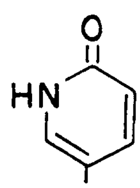

6. 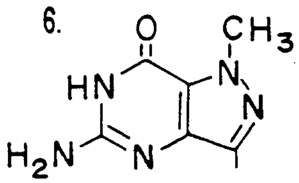

7. 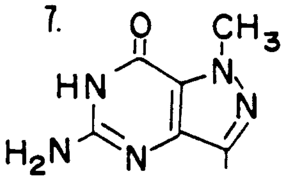

8. 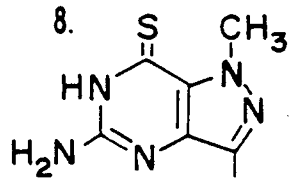

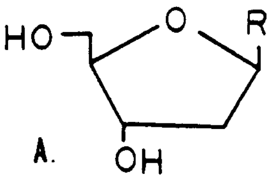
A.

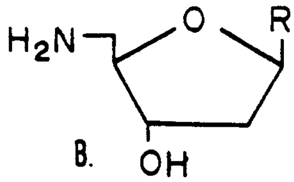
B.

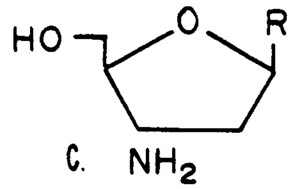
C.

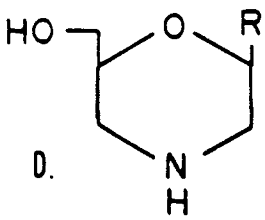
D.

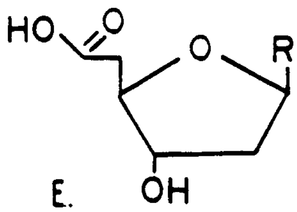
E.

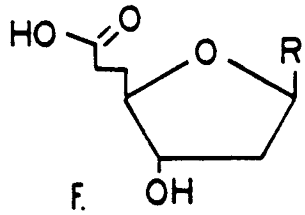
F.

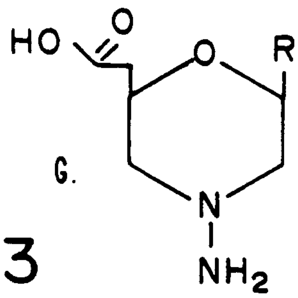
G.

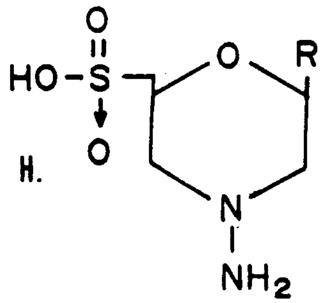
H.

FIG. 3

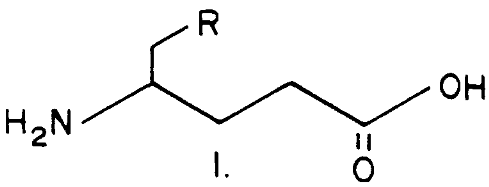
I.

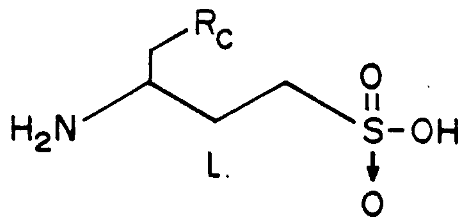
L.

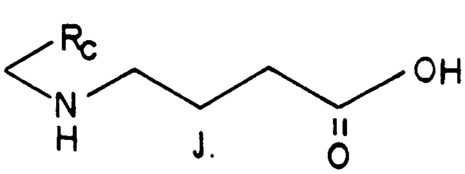
J.

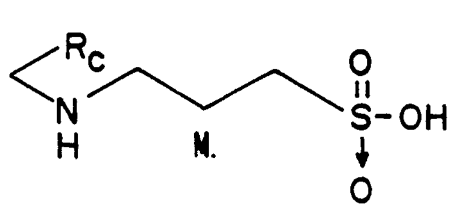
M.

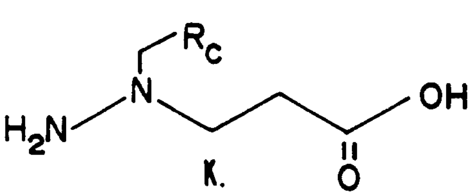
K.

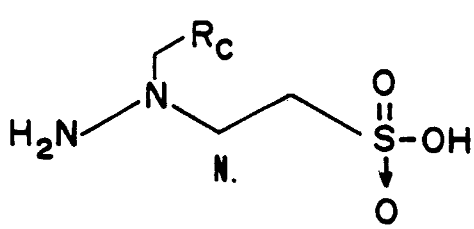
N.

FIG. 4

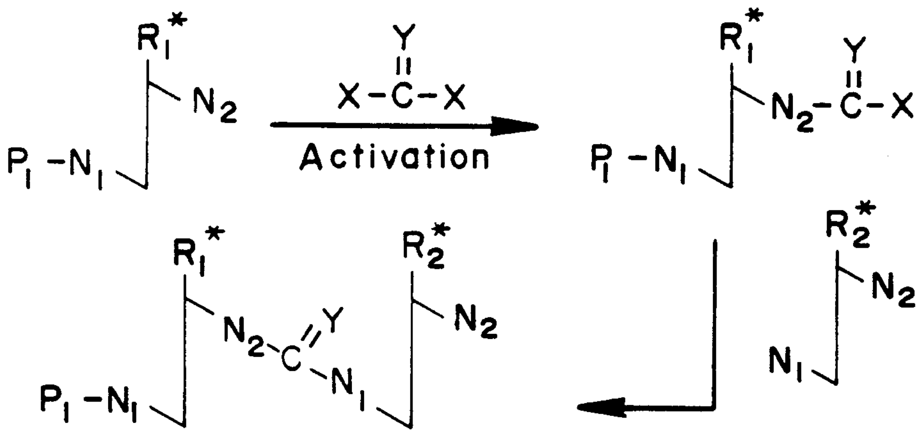


FIG. 5A

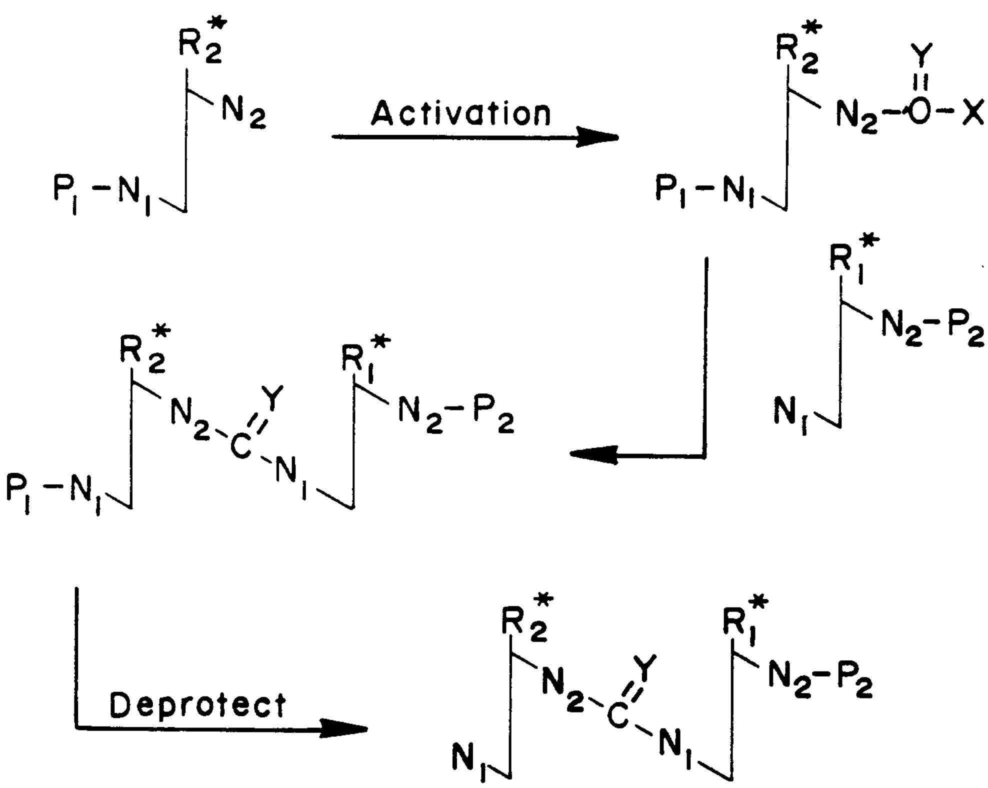


FIG. 5B

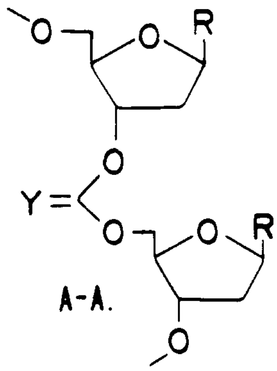
A-A.

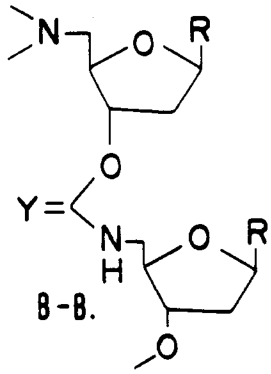
B-B.

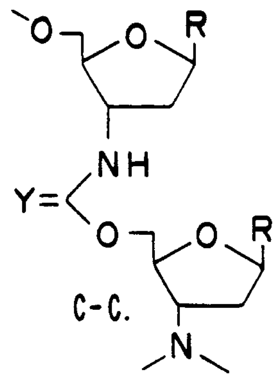
C-C.

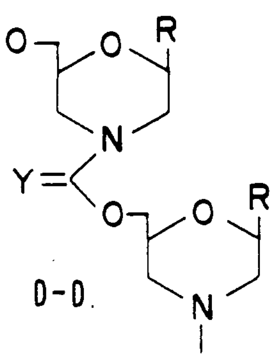
D-D.

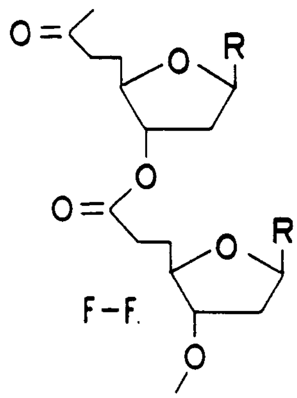
F-F.

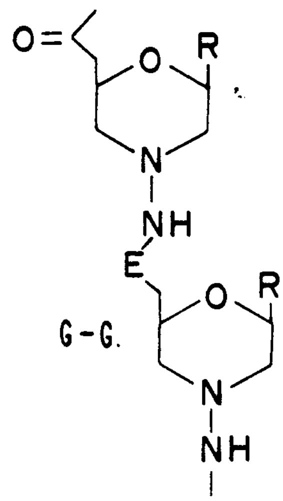
G-G.

FIG. 6

FIG. 8

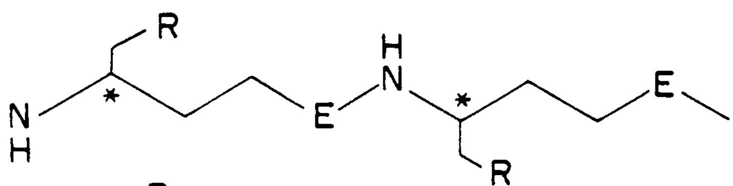
I-I, L-L

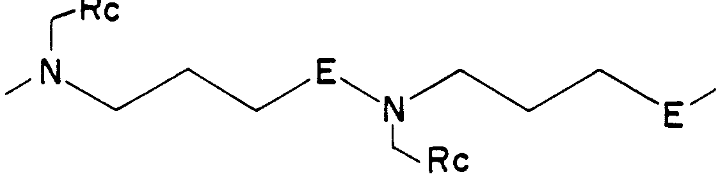
J-J, M-M

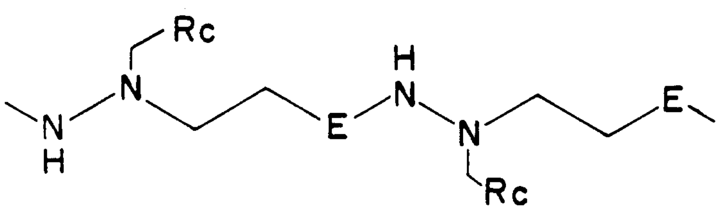
K-K, N-N

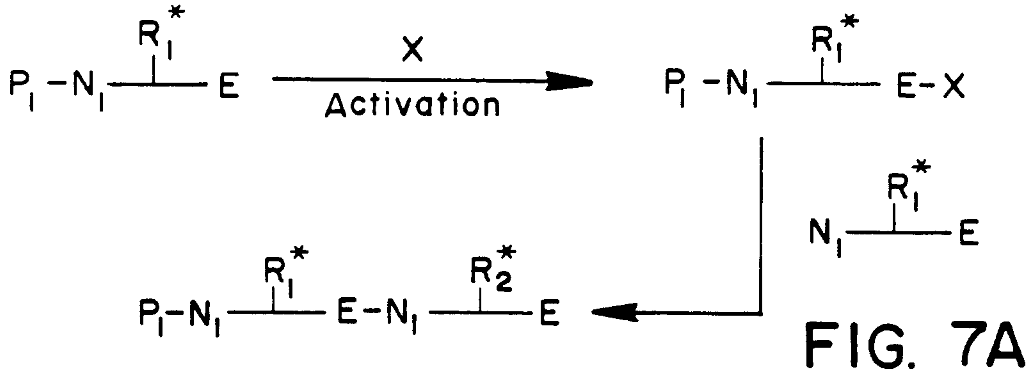


FIG. 7A

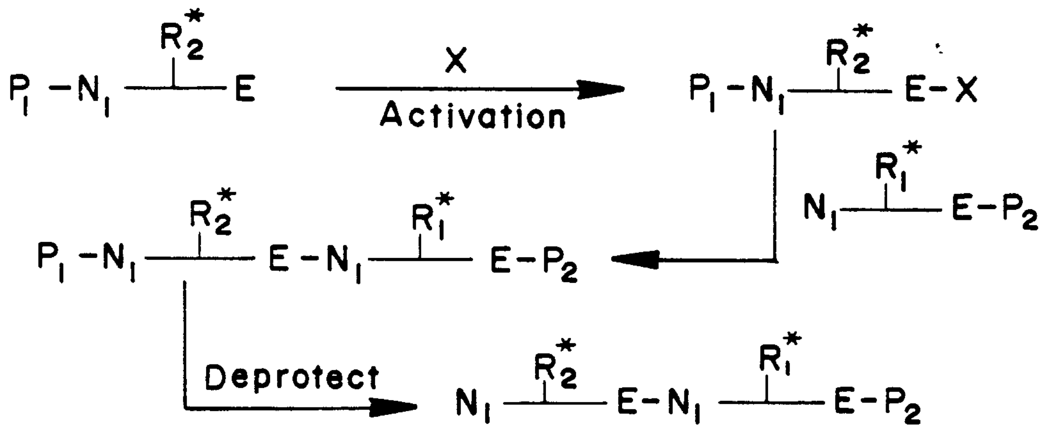


FIG. 7B

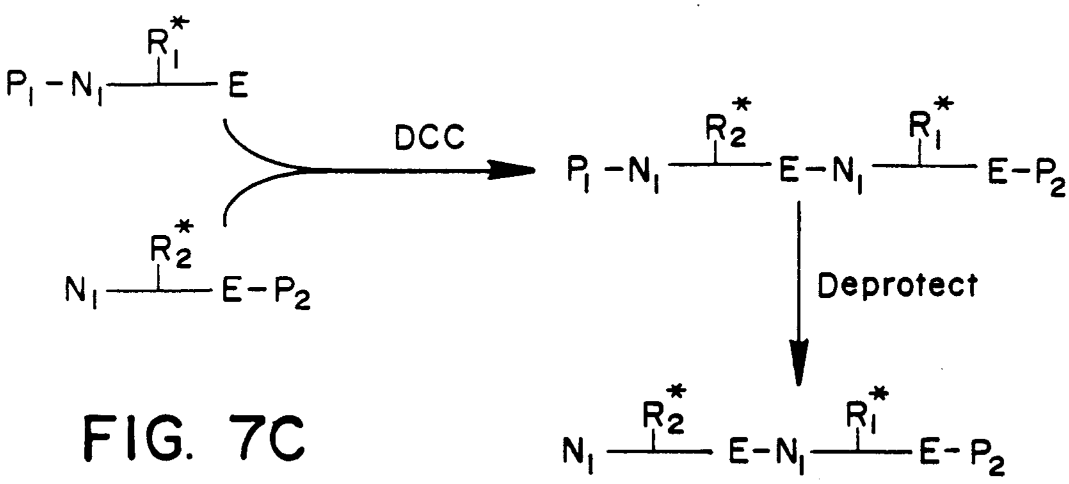


FIG. 7C